# EUROPEAN PATENT SPECIFICATION

(11) **EP 3 661 514 B1**
(45) Date of publication and mention of the grant of the patent: **17.09.2025**
(21) Application number: 18759209.2
(22) Date of filing: 03.08.2018
(51) Int. Cl.: A61K 31/513, A61P 9/04, A61P 9/10

(54) **MAVACAMTEN FOR USE IN THE TREATMENT OF HYPERTROPHIC CARDIOMYOPATHY**
MAVACAMTEN ZUR VERWENDUNG BEI DER BEHANDLUNG DER HYPERTROPHEN KARDIOMYOPATHIE
MAVACAMTEN POUR L'UTILISATION DANS LE TRAITEMENT DE LA CARDIOMYOPATHIE HYPERTROPHIQUE

(30) Priority: 04.08.2017 US 201762541591 P; 10.11.2017 US 201762584537 P; 07.03.2018 US 201862639922 P; 15.05.2018 US 201862671585 P
(43) Date of publication of application: 10.06.2020
(73) Proprietor: MyoKardia, Inc., Brisbane, CA 94005 (US)
(72) Inventor: SEMIGRAN, Marc J., Brisbane, CA 94005 (US); LEE, June H., Brisbane, CA 94005 (US); LAMBING, Joseph, Brisbane, CA 94005 (US); GREEN, Eric, Brisbane, CA 94005 (US); EVANCHIK, Marc, Brisbane, CA 94005 (US); CARLSON, Timothy, Belmont, CA 94002 (US); ZHANG, David, San Carlos, CA 94070 (US)
(74) Representative: Snaith, James Michael
(86) International application number: PCT/US2018/045180
(87) International publication number: WO 2019/028360

(56) References cited:
- US-A1- 2016 030 428
- JOSHUA A. STERN ET AL: "A Small Molecule Inhibitor of Sarcomere Contractility Acutely Relieves Left Ventricular Outflow Tract Obstruction in Feline Hypertrophic Cardiomyopathy", PLOS ONE, vol. 11, no. 12, 14 December 2016 (2016-12-14), pages e0168407, XP055514347, DOI: 10.1371/journal.pone.0168407
- DANIEL JACOBY ET AL: "Heart Failure and Cardiomyopathies REDUCTION IN LEFT VENTRICULAR OUTFLOW TRACT GRADIENT WITH MAVACAMTEN (MYK-461) IN SYMPTOMATIC OBSTRUCTIVE HYPERTROPHIC CARDIOMYOPATHY PATIENTS (PIONEER-HCM)", 11 March 2018 (2018-03-11), XP055513739, Retrieved from the Internet <URL:http://www.onlinejacc.org/content/accj/71/11_Supplement/A644.full.pdf> [retrieved on 20181009]
- ANONYMOUS: "A Phase 2 Open-label Pilot Study Evaluating MYK-461 in Subjects With Symptomatic Hypertrophic Cardiomyopathy and Left Ventricular Outflow Tract Obstruction - Full Text View - ClinicalTrials.gov", 14 December 2017 (2017-12-14), XP055514386, Retrieved from the Internet <URL:https://clinicaltrials.gov/ct2/show/NCT02842242> [retrieved on 20181011]

## Description

### BACKGROUND

Genetic (heritable) hypertrophic cardiomyopathy (HCM) comprises a group of highly penetrant, monogenic, autosomal dominant myocardial diseases. HCM is caused by one or more of over 1,000 known point mutations in any one of the proteins contributing to the functional unit of myocardium, the sarcomere. About 1 in 500 individuals in the general population are found to have left ventricular hypertrophy unexplained by other known causes (e.g., hypertension or valvular disease), and many of these can be shown to have HCM, once other heritable (e.g., lysosomal storage diseases), metabolic, or infiltrative causes have been excluded.

Medical therapy for HCM is limited to the treatment of symptoms and does not address the fundamental, underlying cause of disease--disruptions in normal sarcomere function. Currently available therapies are variably effective in alleviating symptoms but typically show decreased efficacy with increasing disease duration. Patients are thus empirically managed with beta-blockers, non-dihydropyridine calcium channel blockers, and/or disopyramide. None of these agents carry labeled indications for treating HCM, and essentially no rigorous clinical trial evidence is available to guide their use. Compounding this unfortunate situation is the fact that no new medical therapies for HCM have been identified for many years. In approximately 60% of patients with HCM, the left ventricular outflow tract becomes obstructed, impeding the flow of blood and creating a pressure gradient between the LV cavity and the aorta. For patients with hemodynamically significant outflow tract obstruction (gradient ≥50 mmHg), surgical myectomy or alcohol septal ablation can be utilized to alleviate the hemodynamic obstruction albeit with significant clinical morbidity and mortality. Provided are new therapeutic agents and methods that remedy the long-felt need for improved treatment of HCM and related cardiac disorders.

US2016030428A1 has an abstract, which states "[p]rovided are novel pyrimidine dione compounds and pharmaceutically acceptable salts thereof, that are useful for the treatment of hypertrophic cardiomyopathy (HCM) and conditions associated with left ventricular hypertrophy or diastolic dysfunction. The synthesis and characterization of the compounds and pharmaceutically acceptable salts thereof, are described, as well as methods for treating HCM and other forms of heart disease."

Joshua A Stern et al., PLOS ONE, vol. 11, no. 12, 14 December 2016 has the title "[a] Small Molecule Inhibitor of Sarcomere Contractility Acutely Relieves Left Ventricular Outflow Tract Obstruction in Feline Hypertrophic Cardiomyopathy."

### SUMMARY

In one aspect defined in claim 1, there is provided a compound, having the formula: 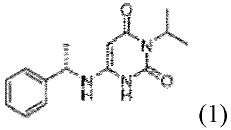 or a pharmaceutically acceptable salt thereof, for use in a method of treating hypertrophic cardiomyopathy (HCM), the method comprising administering a therapeutically effective amount of Compound 1 to said subject, wherein said therapeutically effective amount is a total daily dosage of 2 mg to 15 mg +/- 10%.

In another aspect defined in claim 9, there is provided a compound, having the formula: 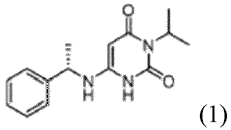 or a pharmaceutically acceptable salt thereof, for use in a method of treating myocardial diastolic dysfunction in a subject in need thereof, the method comprising administering a therapeutically effective amount of Compound 1 to said subject, wherein said therapeutically effective amount is a total daily dosage of 2 mg to 15 mg +/- 10%.

In another aspect defined in claim 10, there is provided a compound, having the formula: 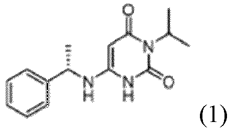
or a pharmaceutically acceptable salt thereof, for use in a method for treating hypertrophic cardiomyopathy (HCM) in an individual in need thereof comprising administering a daily dose of 5 mg of Compound 1 to the individual for at least one week, and
increasing said daily dose to 10 mg when the blood plasma concentration of Compound 1 in the individual is below 200 ng/mL; or
decreasing said daily dose to 2.5 mg when the blood plasma concentration of Compound 1 in the individual is above 750 ng/mL; or
maintaining said daily dose at 5 mg of Compound 1 when the blood plasma concentration of Compound 1 in the individual is from 200 to 750 ng/mL.

In another aspect defined in claim 11, there is provided a compound, having the formula: 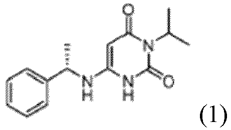 or a pharmaceutically acceptable salt thereof, for use in a method for treating hypertrophic cardiomyopathy (HCM) in an individual in need thereof by achieving and maintaining a blood plasma concentration of Compound 1 from 200 to 750 ng/mL, said method comprising
a) administering a daily dose selected from the group consisting of 2.5, 5, 10, and 15 mg of Compound 1 to an individual.

In another aspect defined in claim 12, there is provided a compound, having the formula: 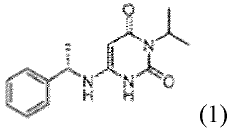 or a pharmaceutically acceptable salt thereof, for use in a method for treating hypertrophic cardiomyopathy in an individual in need thereof comprising administering to the individual a total daily dose of Compound 1 in an amount selected from the group consisting of 2.5 mg, 5 mg, 10 mg, and 15 mg.

Also described herein but not presently claimed is a method of treating hypertrophic cardiomyopathy (HCM). The method includes administering to a subject in need thereof a therapeutically effective amount of Compound 1, as described herein, where the pharmaceutically effective amount is a total daily dosage of about 2 mg to 50 mg, or about 5 mg to 30 mg.

Also described herein but not presently claimed is a method of treating myocardial diastolic dysfunction. The method includes administering to a subject in need thereof a therapeutically effective amount of Compound 1, as described herein, where the pharmaceutically effective amount is a total daily dosage of about 2 mg to 50 mg, or about 5 mg to 30 mg.

Also described herein but not presently claimed is a method of treating hypertrophic cardiomyopathy in an individual in need thereof comprising administering Compound 1 to the individual in an amount effective to achieve and maintain a blood plasma concentration of Compound 1 from 200 to 750 ng/mL. The blood plasma concentration of Compound 1 may be achieved and maintained at 350 to 700 ng/mL. The effective amount to achieve and maintain the desired blood plasma concentration of Compound 1 may be from 2 to 15 mg of Compound 1. The effective amount to achieve and maintain the desired blood plasma concentration of Compound 1 may be from 2.5 to 15 mg of Compound 1. The effective amount to achieve and maintain the desired blood plasma concentration of Compound 1 may be from 2 to 20 mg of Compound 1. The effective amount to achieve and maintain the desired blood plasma concentration of Compound 1 may be from 2.5 to 20 mg of Compound 1. The daily dose of Compound 1 may be adjusted after initial administration based on the determined blood plasma concentration. The HCM may be obstructive HCM (oHCM). The HCM may be non-obstructive HCM.

### BRIEF DESCRIPTION OF THE DRAWINGS

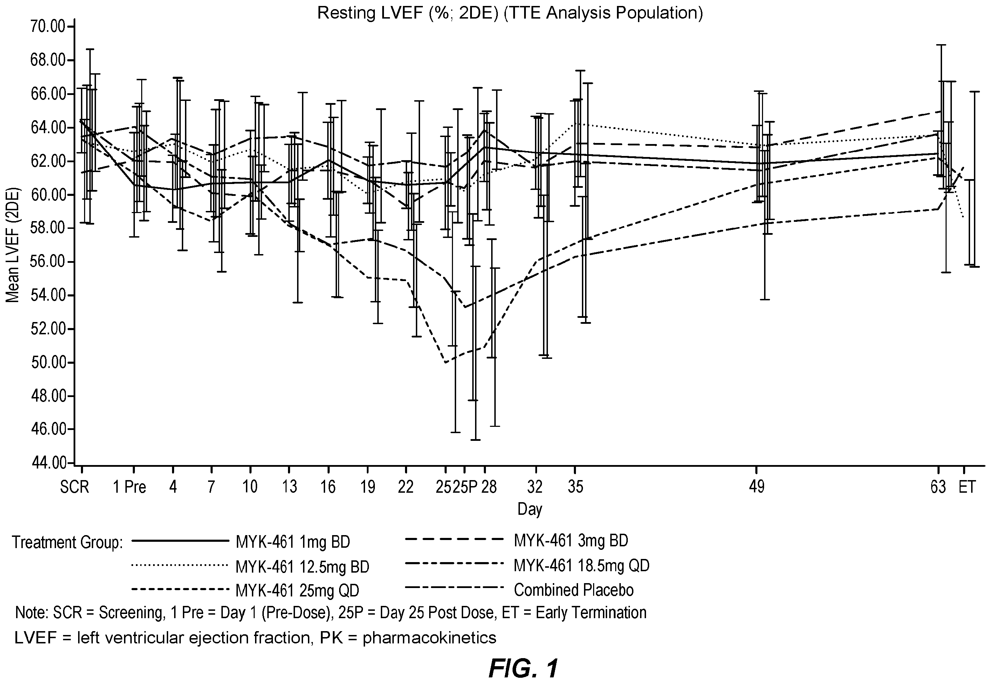

**FIG. 1** shows the resting LVEF (%; 2DE) in a TTE analysis population.

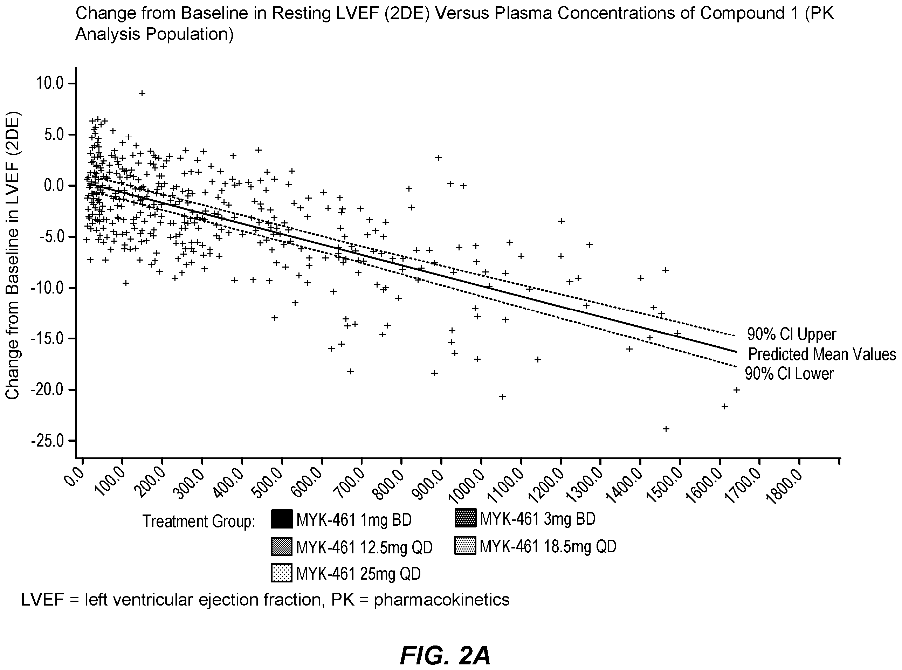

**FIG. 2A** shows a change from baseline in resting LVEF (2DE) versus plasma concentrations of Compound 1 (MYK-461).

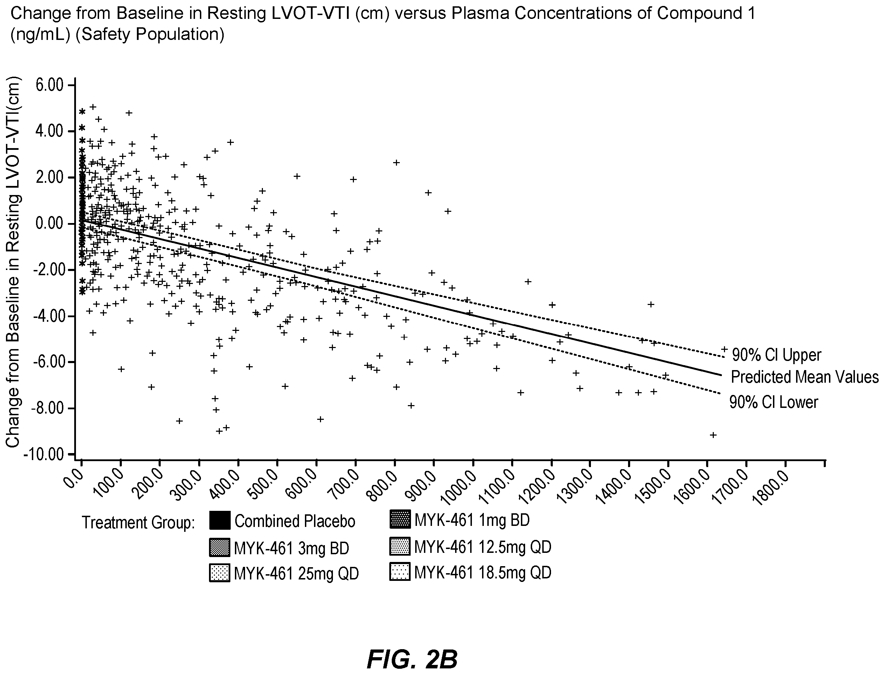

**FIG. 2B** shows a change from baseline in resting LVOT-VTI (left ventricular outflow tract-velocity time integral, cm) versus plasma concentrations of Compound 1 (MYK-46 1).
**FIG. 3** depicts the study scheme. The abbreviations used in the figures are: CPET, cardiopulmonary exercise testing; D, day; EOS, end of study; HSE, hemodynamic stress echocardiography; LVEF, left ventricular ejection fraction; PK, pharmacokinetic sample; QD, once daily; RE/V, resting echocardiography/Valsalva; W, week.
**FIG. 4** shows the change from baseline in post-exercise LVOT gradient over time.
**FIG. 5** shows the stress ejection fraction (EF) percentage at weeks 0, 4, and 12 in subjects receiving Compound 1 therapy.
**FIG. 6** shows the resting LVOT gradient in subjects receiving Compound 1 (10, 15, or 20 mg) between weeks 0 and 12.
**FIG. 7** plots the resting LVOT gradient v. Compound 1 concentration.
**FIG. 8** plots the resting left ventricle ejection fraction (LVEF) (%) v. Compound 1 concentration.
**FIG. 9** plots the average resting LVOT gradient measured each week of the study. Administration of Compound 1 is stopped after week 12.
**FIG. 10** plots the average resting left ventricle ejection fraction (LVEF) (%) measured each week of the study. Administration of Compound 1 is stopped after week 12.
**FIG. 11** illustrates a type of plot that can be obtained when measuring oxygen consumption (mL/kg/min) and arterial lactate (mM/L) during Cardiopulmonary Exercise Testing.
**FIG. 12** shows peak VO₂ (pVO₂) values measured during cardiopulmonary exercise testing (CPET) at baseline (0 weeks, solid bar) and after week 12 (hashed bar).
**FIG. 13** shows peak circulatory power measured during cardiopulmonary exercise testing (CPET) at baseline (0 weeks, solid bar) and after week 12 (hashed bar).
**FIG. 14** plots the absolute change in pVO₂ v. absolute change in left ventricle ejection fraction (LVEF).
**FIG. 15** plots the N-terminal pro B-type natriuretic peptide (NT-proBNP) concentration (pg/mL) at weeks 0, 4, 8, and 12 in subjects receiving Compound 1.
**FIG. 16** plots the N-terminal pro B-type natriuretic peptide (NT-proBNP) concentration (pg/mL) v. the concentration of Compound 1 (ng/mL).
**FIG.** 17 shows the New York Heart Association (NYHA) functional class of subjects at baseline (0 weeks), after 12 weeks of Compound 1 administration, and after week 16 (12 weeks of Compound 1 administration and 4 weeks of Compound 1 washout).
**FIG. 18** shows the mean dyspnea symptom score for each subject at the indicated times (Day 1 - Week 12). Compound 1 was administered through week 12.
**FIG. 19** plots the mean post-exercise resting LVOT gradient (mm Hg) at weeks 4, 12, and 16 of the Pioneer-HCM-B trial (12 weeks of administering Compound 1, 4 weeks of washout).
**FIG. 20** plots the change in resting LVEF (%) at weeks 1-8, 12, and 16 of the Pioneer-HCM-B trial (12 weeks of administering Compound 1, 4 weeks of washout).
**FIG. 21** illustrates the improvement in NYHA functional class in individuals after 12 weeks and the reversal of improvement after 4 weeks of washout of Compound 1 during the Pioneer-HCM-B trial.
**FIG. 22** illustrates the rapid improvement in peak in dyspnea throughout the Pioneer-HCM-B trial. It also shows that the improvement is reversed after 4 weeks of washout of Compound 1.
**FIG. 23** illustrates the improvement in peak VO₂ by week 12 during the Pioneer-HCM-B trial.
**FIG. 24** plots the resting LVOT (mmHg) as a function of Compound 1 concentration. Cohort B are open squares and cohort A are filled squares.
**FIG. 25** shows the study design for the pivotal trial to confirm safety and efficacy in Obstructive hypertrophic cardiomyopathy (oHCM) patients.

### DETAILED DESCRIPTION

Described herein are methods for treating hypertrophic cardiomyopathy (HCM) in a subject. The methods include specific dosing regimens that have great efficacy in treating the patient and that are well tolerated in patients. Any references to methods of treatment in this description are to be interpreted as references to the compounds, pharmaceutical compositions and medicaments of the present invention for use in a method for treatment of the human (or animal) body by therapy (or for diagnosis).

### Definitions

While various embodiments and aspects of the present invention are shown and described herein, it will be obvious to those skilled in the art that such embodiments and aspects are provided by way of example only. Numerous variations, changes, and substitutions will now occur to those skilled in the art without departing from the invention. It should be understood that various alternatives to the embodiments of the invention described herein may be employed in practicing the invention.

The section headings used herein are for organizational purposes only.

Unless defined otherwise, technical and scientific terms used herein have the same meaning as commonly understood by a person of ordinary skill in the art. See, e.g., Singleton et al., DICTIONARY OF MICROBIOLOGY AND MOLECULAR BIOLOGY, 2nd ed., J. Wiley & Sons (New York, NY 1994); Sambrook et al., MOLECULAR CLONING, A LABORATORY MANUAL, Cold Springs Harbor Press (Cold Springs Harbor, NY 1989). Any methods, devices and materials similar or equivalent to those described herein can be used in the practice of this invention. The following definitions are provided to facilitate understanding of certain terms used frequently herein.

The terms "a" or "an," as used in herein means one or more.

The terms "comprise," "include," and "have," are used herein interchangeably as comprehensive, open-ended terms. For example, use of "comprising," "including," or "having" means that whatever element is comprised, had, or included, is not the only element encompassed by the subject of the clause that contains the verb.

As used herein, the term "about" means a range of values including the specified value, which a person of ordinary skill in the art would consider reasonably similar to the specified value. In some embodiments, the term "about" means within a standard deviation using measurements generally acceptable in the art. In some embodiments, about means a range extending to +/- 10% of the specified value. In some embodiments, about means the specified value.

As used herein, "treatment" or "treating," or "palliating" or "ameliorating" are used interchangeably herein. These terms refer to an approach for obtaining beneficial or desired results including but not limited to a therapeutic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder. Treatment includes causing the clinical symptoms of the disease to slow in development by administration of a composition; suppressing the disease, that is, causing a reduction in the clinical symptoms of the disease; inhibiting the disease, that is, arresting the development of clinical symptoms by administration of a composition after the initial appearance of symptoms; and/or relieving the disease, that is, causing the regression of clinical symptoms by administration of a composition after their initial appearance. For example certain methods described herein treat hypertrophic cardiomyopathy (HCM) by decreasing or reducing the occurrence, or progression of HCM; or treat HCM by decreasing a symptom of HCM. Symptoms of, or test results indicating HCM would be known or may be determined by a person of ordinary skill in the art and may include, but are not limited to, shortness of breath (especially during exercise), chest pain (especially during exercise), fainting (especially during or just after exercise), sensation of rapid, fluttering or pounding heartbeats, atrial and ventricular arrhythmias, heart murmur, hypertrophied and non-dilated left ventricle, thickened heart muscle, thickened left ventricular wall, elevated pressure gradient across left ventricular outflow tract (LVOT), elevated post-exercise LVOT gradient, and high left ventricular ejection fraction (LVEF).

An "effective amount" or a "pharmaceutically effective amount" is an amount sufficient to accomplish a stated purpose (e.g. achieve the effect for which it is administered, treat a disease, reduce enzyme activity, reduce one or more symptoms of a disease or condition, reduce viral replication in a cell). An example of an "effective amount" is an amount sufficient to contribute to the treatment, or reduction of a symptom or symptoms of a disease, which could also be referred to as a "therapeutically effective amount." A "reduction" of a symptom or symptoms (and grammatical equivalents of this phrase) means decreasing of the severity or frequency of the symptom(s), or elimination of the symptom(s ). Efficacy can also be expressed as "-fold" increase or decrease. For example, a therapeutically effective amount can have at least a 1.2-fold, 1.5-fold, 2-fold, 5-fold, or more effect over a control.

"Patient" or "subject" or "subject in need thereof" refers to a living organism suffering from or prone to a disease or condition that can be treated by using the methods provided herein. The term does not necessarily indicate that the subject has been diagnosed with a particular disease, but typically refers to an individual under medical supervision. Non-limiting examples include humans, other mammals, bovines, rats, mice, dogs, cats, monkeys, goat, sheep, cows, deer, and other non-mammalian animals. In some embodiments, a patient, subject or subject in need thereof is a human.

Hypertrophic cardiomyopathy (HCM) is defined clinically as unexplained left ventricular (LV) hypertrophy in the absence of known causes such as pressure overload, systemic diseases, or infiltrative processes. The phenotypic hallmark of HCM is myocardial hypercontractility accompanied by reduced LV compliance, reflected clinically as reduced ventricular chamber size, often supranormal ejection fraction, increased wall thickness, and diastolic dysfunction. Some of the symptoms and signs that HCM patients have include, but are not limited to, shortness of breath (especially during exercise), chest pain (especially during exercise), fainting (especially during or just after exercise), sensation of rapid, fluttering or pounding heartbeats, and heart murmur.

Obstructive HCM (oHCM) is defined as at least a 30 mmHg (i.e., 30 mm Hg or higher) pressure gradient across the LVOT in an individual at rest, during or immediately after Valsalva maneuver, or post-exercise. In some embodiments, an individual with oHCM has an LVOT pressure gradient of at least 40 mm Hg, 45 mm Hg or 50 mm Hg. In some embodiments, the pressure gradient across the LVOT in the individual is measured at rest. In some embodiments, the pressure gradient across the LVOT in the individual is measured during or immediately after a Valsalva maneuver is performed. In some embodiments, the pressure gradient across the LVOT in the individual is measured post-exercise. Degree of obstruction and manifestation of clinical symptoms consisting with heart failure is primary criterion for invasive intervention. oHCM may lead to severe symptoms of heart failure, arrhythmias, and/or death. Obstruction relief improves symptoms and function.

As used herein "Valsalva gradient" refers to the pressure gradient across LVOT in an individual while this individual is performing a Valsalva maneuver.

Diastolic dysfunction is present or an important feature of a series of diseases including, but not limited to, hypertrophic cardiomyopathy (HCM), heart failure with preserved ejection fraction (HFpEF) - including both disorders of active relaxation and disorders of chamber stiffness (diabetic HFpEF); dilated cardiomyopathy (DCM), ischemic cardiomyopathy, cardiac transplant allograft vasculopathy, restrictive cardiomyopathy - including inflammatory subgroups (e.g., Loefllers and EMF), infiltrative subgroups (e.g., amyloid, sarcoid and XRT), storage subgroups (e.g., hemochromatosis, Fabry and glycogen storage disease), idiopathic/inherited subgroups such as Trop I (beta myosin HC), Trop T (alpha cardiac actin) and desmin related (usually includes skeletal muscle), congenital heart disease subgroups (including pressure-overloaded RV, Tetrology of Fallot (diastolic dysfunction pre-op and early post-op, systolic dysfunction post-op) and pulmonic stenosis), and valvular heart disease (e.g., aortic stenosis - including elderly post AVR/TAVR and congenital forms).

### Methods

In one aspect defined in claim 1, provided herein is Compound 1 or a pharmaceutically acceptable salt thereof, for use in a method of treating hypertrophic cardiomyopathy (HCM). The method includes administering to a subject in need thereof a therapeutically effective amount of Compound 1, having the formula: 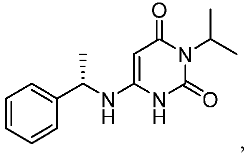 or a pharmaceutically acceptable salt thereof, where the therapeutically effective amount is a total daily dosage of 2 mg to 15 mg +/- 10%. In some embodiments, the total daily dosage of Compound 1 is 2 mg to 10 mg, or 2 mg to 5 mg, +/- 10%. In some embodiments, the total daily dosage of Compound 1 is 2.5 mg to 15 mg. In some embodiments, the total daily dosage of Compound 1 is 5 mg to 15 mg. In some embodiments, the total daily dosage of Compound 1 is 2.5 mg to 15 mg.

In one embodiment, the invention is directed to treatment of hypertrophic cardiomyopathy (HCM) in a subject in need thereof. In another embodiment, the treatment is directed to obstructive HCM in the subject. In a further embodiment, the treatment comprises administering a therapeutically effective amount of Compound 1 to the subject. In yet another embodiment, the therapeutically effective amount is dosed such that the plasma concentration of Compound 1 in said subject is between 225-600 ng/mL. In some embodiments, the therapeutically effective amount is dosed such that the plasma concentration of Compound 1 in said subject is between 350-700 ng/mL. In some embodiments, the blood plasma concentration of Compound 1 is between 150-600 ng/mL. In some embodiments, the blood plasma concentration of Compound 1 is between 200-400 ng/mL. In another embodiment, the treatment comprises administering Compound 1 to the subject whereby the LVOT within the subject decreases without a significant change in LVEF. In yet another embodiment, the treatment comprises the concomitant administration to said subject of one or more beta blockers in addition to Compound 1.

Individuals with hypertrophic cardiomyopathy can be symptomatic or asymptomatic. Generally speaking, symptomatic individuals are those that display evidence or have manifestations indicating the existence of their heart condition such as limitations to physical activity, whereas individuals who do not experience these limitations are considered asymptomatic. As a non-limiting example, individuals that are NYHA class II, III, or IV are considered symptomatic individuals and individuals are the NYHA class I are considered asymptomatic. In some embodiments, the individuals treated in the methods described herein have symptomatic hypertrophic cardiomyopathy. In some embodiments, the individuals treated in the methods described herein have asymptomatic hypertrophic cardiomyopathy.

In some embodiments, the subject has not received a β-blocker therapy for a period of at least one to eight weeks prior to initiating treatment with Compound 1. In some embodiments, the subject has not received a β-blocker therapy for a period of at least one, two, three, or four weeks prior to initiating treatment with Compound 1. In some embodiments, the subject has not received a β-blocker therapy for a period of at least two weeks prior to initiating treatment with Compound 1. In some embodiments, the subject has not received a β-blocker therapy for a period of at least three weeks prior to initiating treatment with Compound 1. In some embodiments, the subject has not received a β-blocker therapy for a period of at least four weeks prior to initiating treatment with Compound 1.

In some embodiments, Compound 1 is administered in combination with a β-blocker therapy. In such combination therapy, the β-blocker therapy and Compound 1 are generally administered according to label instructions.

In some embodiments, the subject has not received a Ca channel blocker therapy for a period of at least one to eight weeks prior to initiating treatment with Compound 1. In some embodiments, the subject has not received a Ca channel blocker therapy for a period of at least one, two, three, or four weeks prior to initiating treatment with Compound 1. In some embodiments, the subject has not received a Ca channel blocker therapy for a period of at least two weeks prior to initiating treatment with Compound 1. In some embodiments, the subject has not received a Ca channel blocker therapy for a period of at least three weeks prior to initiating treatment with Compound 1. In some embodiments, the subject has not received a Ca channel blocker therapy for a period of at least four weeks prior to initiating treatment with Compound 1.

In some embodiments, Compound 1 is administered in combination with a Ca channel blocker therapy. In such combination therapy, the Ca channel blocker therapy and Compound 1 are generally administered according to label instructions.

In the methods described herein, co-administration of Compounds 1 with an additional medicament can be desirable. In some embodiments, the additional medicament is selected from the group consisting of β-blocker therapy and Ca channel blocker therapy.

In some embodiments, HCM treated by the methods provided herein is an obstructive HCM (oHCM).

In some embodiments, oHCM is characterized as having at least 30 mmHg (e.g., about 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 mmHg or higher) pressure gradient across the left ventricular outflow tract (LVOT) in said subject at rest.

In some embodiments, oHCM is characterized as having at least 35 mmHg (e.g., about 35, 36, 37, 38, 39, 40, 41, 42, 43, 44, 45, 46, 47, 48, 49, 50, 51, 52, 53, 54, 55, 56, 57, 58, 59, 60 mmHg or higher) pressure gradient across left ventricular outflow tract (LVOT) in said subject at rest.

In some embodiments, oHCM is characterized as having at least 40 mmHg (e.g., about 40, 41, 42, 43, 44, 45, 46, 47, 48, 49, 50, 51, 52, 53, 54, 55, 56, 57, 58, 59, 60 mmHg or higher) pressure gradient across left ventricular outflow tract (LVOT) in said subject at rest.

In some embodiments, oHCM is characterized as having at least 45 mmHg (e.g., about 45, 46, 47, 48, 49, 50, 51, 52, 53, 54, 55, 56, 57, 58, 59, 60 mmHg or higher) pressure gradient across left ventricular outflow tract (LVOT) in said subject at rest.

In some embodiments, oHCM is characterized as having at least 50 mmHg (e.g., about 50, 51, 52, 53, 54, 55, 56, 57, 58, 59, 60 mmHg or higher) pressure gradient across left ventricular outflow tract (LVOT) in said subject at rest.

In some embodiments, oHCM is characterized as having at least 30 mmHg (e.g., about 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 mmHg or higher) pressure gradient across the left ventricular outflow tract (LVOT) in said subject during or immediately after a Valsalva maneuver is performed.

In some embodiments, oHCM is characterized as having at least 35 mmHg (e.g., about 35, 36, 37, 38, 39, 40, 41, 42, 43, 44, 45, 46, 47, 48, 49, 50, 51, 52, 53, 54, 55, 56, 57, 58, 59, 60 mmHg or higher) pressure gradient across left ventricular outflow tract (LVOT) in said subject during or immediately after a Valsalva maneuver is performed.

In some embodiments, oHCM is characterized as having at least 40 mmHg (e.g., about 40, 41, 42, 43, 44, 45, 46, 47, 48, 49, 50, 51, 52, 53, 54, 55, 56, 57, 58, 59, 60 mmHg or higher) pressure gradient across left ventricular outflow tract (LVOT) in said subject during or immediately after a Valsalva maneuver is performed.

In some embodiments, oHCM is characterized as having at least 45 mmHg (e.g., about 45, 46, 47, 48, 49, 50, 51, 52, 53, 54, 55, 56, 57, 58, 59, 60 mmHg or higher) pressure gradient across left ventricular outflow tract (LVOT) in said subject during or immediately after a Valsalva maneuver is performed.

In some embodiments, oHCM is characterized as having at least 50 mmHg (e.g., about 50, 51, 52, 53, 54, 55, 56, 57, 58, 59, 60 mmHg or higher) pressure gradient across left ventricular outflow tract (LVOT) in said subject during or immediately after a Valsalva maneuver is performed.

In some embodiments, oHCM is characterized as having at least 30 mmHg (e.g., about 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 mmHg or higher) pressure gradient across the left ventricular outflow tract (LVOT) in said subject post-exercise.

In some embodiments, oHCM is characterized as having at least 35 mmHg (e.g., about 35, 36, 37, 38, 39, 40, 41, 42, 43, 44, 45, 46, 47, 48, 49, 50, 51, 52, 53, 54, 55, 56, 57, 58, 59, 60 mmHg or higher) pressure gradient across left ventricular outflow tract (LVOT) in said subject post-exercise.

In some embodiments, oHCM is characterized as having at least 40 mmHg (e.g., about 40, 41, 42, 43, 44, 45, 46, 47, 48, 49, 50, 51, 52, 53, 54, 55, 56, 57, 58, 59, 60 mmHg or higher) pressure gradient across left ventricular outflow tract (LVOT) in said subject post-exercise.

In some embodiments, oHCM is characterized as having at least 45 mmHg (e.g., about 45, 46, 47, 48, 49, 50, 51, 52, 53, 54, 55, 56, 57, 58, 59, 60 mmHg or higher) pressure gradient across left ventricular outflow tract (LVOT) in said subject post-exercise.

In some embodiments, oHCM is characterized as having at least 50 mmHg (e.g., about 50, 51, 52, 53, 54, 55, 56, 57, 58, 59, 60 mmHg or higher) pressure gradient across left ventricular outflow tract (LVOT) in said subject post-exercise.

In some embodiments, Compound 1 is administered orally. In some embodiments, Compound 1 is administered in a unit dosage form including 2 mg, 2.5 mg, 5 mg, 7.5 mg, 10 mg, or 15 mg of Compound 1. In some embodiments, Compound 1 is administered daily in single, divided, or continuous doses.

In some embodiments, Compound 1 is administered once daily, at a daily dosage amount of 2.5, 5, 10, or 15 mg. In some embodiments, Compound 1 is administered twice daily. In some embodiments, Compound 1 is administered three times daily.

In some embodiments, Compound 1 is administered daily for at least twelve weeks. In some embodiments, Compound 1 is administered daily for at least ten weeks. In some embodiments, Compound 1 is administered daily for at least eight weeks. In some embodiments, Compound 1 is administered daily for at least six weeks. In some embodiments, Compound 1 is administered daily for at least four weeks. In some embodiments, Compound 1 is administered daily for at least two weeks.

In some embodiments, Compound 1 is administered daily for at least 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83 or 84 days.

In some embodiments, the therapeutically effective amount is sufficient to lower a post-exercise or resting LVOT gradient to less than 30 mmHg (e.g., about 29, 28, 27, 26, 25, 24, 23, 22, 21, 20, 19, 18, 17, 16, 15, 14, 13, 12, 11, 10, 9, 8, 7, 6, 5 mmHg) in the subject upon the administration of the compound. In some embodiments, the therapeutically effective amount is sufficient to lower a post-exercise gradient LVOT to less than 10 mmHg (e.g., about 9, 8, 7, 6, 5 mmHg) in the subject upon the administration of the compound. Post-exercise (stress) gradient LVOT can be measured by any methods known in the art.

In some embodiments, the therapeutically effective amount of Compound 1 is sufficient to improve, stabilize or delay worsening in New York Heart Association (NYHA) functional classification of subjects. The NYHA functional classification grades the severity of heart failure symptoms as one of four functional classes. The NYHA functional classification is widely used in clinical practice and in research because it provides a standard description of severity that can be used to assess response to treatment and to guide management. The NYHA functional classification based on severity of symptoms and physical activity:
Class I: No limitation of physical activity. Ordinary physical activity does not cause undue breathlessness, fatigue, or palpitations.
Class II: Slight limitation of physical activity. Comfortable at rest, but ordinary physical activity results in undue breathlessness, fatigue, or palpitations.
Class III: Marked limitation of physical activity. Comfortable at rest, but less than ordinary physical activity results in undue breathlessness, fatigue, or palpitations.
Class IV: Unable to carry on any physical activity without discomfort. Symptoms at rest can be present. If any physical activity is undertaken, discomfort is increased.

Administration of Compound 1 may be used for the treatment of all NYHA functional classes of heart failure but particularly the classes II-IV of the NYHA functional classification system.

In some embodiments, administration of a therapeutically effective amount of Compound 1 reduces the New York Heart Association (NYHA) functional classification of the subject. In some embodiments, the NYHA functional classification is reduced from class IV to class III, from class IV to class II, or from class IV to class I. In some embodiments, the NYHA functional classification is reduced from class III to class II. In some embodiments, the NYHA functional classification is reduced from class III to class I. In some embodiments, the NYHA functional classification is reduced from class II to class I.

In some embodiments, the therapeutically effective amount is a total daily dosage of 2 mg to 10 mg +/- 10% (e.g., 2, 2.5, 3, 4, 5, 6, 7, 7.5, 8, 9, 10 mg/day, +/- 10%). In some embodiments, the therapeutically effective amount is a total daily dosage of 5, 6, 7, 7.5, 8, 9, 10, 11, 12, 12.5, 13, 14, or 15 mg/day +/- 10%). In some embodiments, the therapeutically effective amount is a total daily dosage of 2 mg +/- 10%. In some embodiments, the therapeutically effective amount is a total daily dosage of 2.5 mg +/- 10%. In some embodiments, the therapeutically effective amount is a total daily dosage of 5 mg +/- 10%. In some embodiments, the therapeutically effective amount is a total daily dosage of 7.5 mg +/- 10%. In some embodiments, the therapeutically effective amount is a total daily dosage of 10 mg +/- 10%. In some embodiments, the therapeutically effective amount is a total daily dosage of 12.5 mg +/- 10%. In some embodiments, the therapeutically effective amount is a total daily dosage of 15 mg +/- 10%.

In some embodiments, the total daily dose is adjusted according to individual patient requirements. For example, the total daily dose may be adjusted after 1 to 8 weeks (e.g. after 1, 2, 3, 4, 5, 6, 7, 8 weeks, or any number of days in between) of initiating Compound 1 therapy depending on the response profile of the subject. In some embodiments, the total daily dose is decreased when the subject's New York Heart Association (NYHA) functional classification is reduced. As a non-limiting example of this reduction, in some embodiments, the total daily dose is decreased from 5 mg to 2.5 mg when the subject's NYHA functional classification is reduced from class III to class I, from class III to class II, or from class II to class I. In some embodiments, the total daily dose is increased when the subject's New York Heart Association (NYHA) functional classification is not reduced or worsens. As a non-limiting example of this increase, in some embodiments, the total daily dose is increased from 5 mg to 7.5 mg or 10 mg or 15 mg.

In some embodiments, the individual subjects requirements used to adjust the total daily dose are the subject's resting left ventricular ejection fraction and resting left ventricular outflow tract (LVOT) peak gradient. For example, in some embodiments, the total daily dose is 5 mg, and said dose is increased when the subject's resting left ventricular ejection fraction (LVEF) is ≥55% and resting left ventricular outflow tract (LVOT) peak gradient is ≥30 mm Hg. In some embodiments, the total daily dose is increased to 7.5 mg when the subject's resting left ventricular ejection fraction (LVEF) is ≥55% and resting left ventricular outflow tract (LVOT) peak gradient is from ≥30 mm Hg to <50 mm Hg. In some embodiments, the total daily dose is increased to 10 mg when the subject's resting left ventricular ejection fraction (LVEF) is ≥55% and resting left ventricular outflow tract (LVOT) peak gradient is ≥50 mm Hg. In an additional non-limiting example, in some embodiments, the total daily dose is 5 mg, and said dose is increased to 7.5 mg when the subject's resting left ventricular ejection fraction (LVEF) is 54-46% and resting left ventricular outflow tract (LVOT) peak gradient is ≥50 mm Hg.

In some embodiments, the therapeutically effective amount can be adjusted according to the left ventricular ejection fraction (LVEF) level of the subject.

In some embodiments, the method provided herein also includes measuring the left ventricular ejection fraction (LVEF) in the subject prior to the administration of the compound, thereby providing a first LVEF value (baseline). In some embodiments, the method provided herein also include measuring the LVEF in the subject sometime (e.g., 1, 2, 3, 4, 5, 6, 7, 8, 9, 10, 11, 12, 13, 14, 15, 16, 17, 18, 19, 20, 21, 22, 23, 24, 25, 26, 27, or 28 days) after the imitation of of the compound, thereby providing a second LVEF value, and calculating a percentage of change of the second LVEF value compared to the first LVEF value. Accordingly, in some embodiments, total daily dosage is adjusted according to the percentage of change of LVEF.

In some embodiments, the second LVEF is measured 4 weeks after the administration of the compound. In some embodiments, the total daily dosage is increased by 10 mg when said percentage of change is less than 10% (e.g., about 9%, 8%, 7%, 6%, 5%, 4%, 3%, 2%, 1% or less). In some embodiments, the total daily dosage is increased by 5 mg when said percentage of change is 10% or higher but below 15% (e.g., about 10%, 11%, 12%, 13%, or 14%). In some embodiments, the total daily dosage remains the same when said percentage of change is 15% or higher but below 20% (e.g., about 15%, 16%, 17%, 18%, or 19%). In some embodiments, the total daily dosage is reduced by 5 mg when said percentage of change is 20% or higher (e.g., about 20%, 21%, 22%, 23%, 24%, 25%, 26%, 27%, 28%, 29%, 30% or higher).

In some embodiments, the therapeutically effective amount can be adjusted according to the weight of the subject. In some embodiments, the method provided herein also includes measuring the weight of the subject prior to the administration of the Compound 1. In some embodiments, the initial daily dosage is 10 mg +/- 10% when said subject has a weight of 60 kg or less. In some embodiments, the initial daily dosage is 15 mg +/- 10% when said subject has a weight of more than 60 kg. In some embodiments, the compound is administered in a therapeutically effective amount of this initial daily dosage for at least 1, 2, 3, 4, 5, 6, 7, 8, 9, 10, 11, 12, 13, 14, 15, 16, 17, 18, 19, 20, 21, 22, 23, 24, 25, 26, 27, or 28 days.

In some embodiments, the daily dosage is 10 mg +/- 10% for the first four weeks of the treatment when said subject has a weight of 60 kg or less. In some embodiments, the daily dosage is 15 mg +/- 10% for the first four weeks of the treatment when said subject has a weight of more than 60 kg.

In each of the methods described herein, in some embodiments, the individual weighs at least 45 kg.

In some embodiments, the therapeutically effective amount can be adjusted according to the plasma concentration of the Compound 1. In some embodiments, the method also includes measuring the plasma concentration of the Compound 1 at least 1, 2, 3, 4, 5, 6, 7, 8, 9, 10, 11, 12, 13, 14, 15, 16, 17, 18, 19, 20, 21, 22, 23, 24, 25, 26, 27, or 28 days after administration of the Compound 1. In some embodiments, the therapeutically effective amount of the daily dose of Compound 1 can be adjusted as described herein.

In some embodiments, the therapeutically effective amount can be adjusted based on 'trough' measurements. 'Trough' measurements (either concentration or any pharmacodynamic measurement) refers to measurements taken just prior to the next dose. For example, for once daily (QD) dosing these occur every ~24 hours just prior to the patient taking their next dosage (typically a tablet or capsule). For pharmacokinetic reasons, these measurements are used as a way to standardize assessments and minimize variability. When an individual "achieves and maintains" a certain blood plasma concentration of Compound 1, the individual's trough measurement does not go below the referenced minimum level or above the referenced maximum level.

Adjustments to dosage can also be made based on an individual's ability to metabolize Compound 1. Poor metabolizers of Compound 1 can include individuals with mutant forms of CYP 2C19 or CYP 3A4 enzymes. Poor metabolizers of Compound 1 can be administered a lower starting dose and/or the dose can be adjusted to a lower amount such as 1 mg. For example, in some embodiments, a poor metabolizer of Compound 1 is administered an initial dose of 2.5 mg and the dose may be adjusted down to 1 mg if the trough measurement of Compound 1 in the individuals blood plasma is above a desired maximum level. In some embodiments, a poor metabolizer of Compound 1 is administered an initial dose of 1 mg. In some embodiments, poor metabolizers of Compound 1 are of Japanese descent.

In some embodiments, the methods herein further comprise measuring a plasma concentration of Compound 1 two weeks after the administration.

In some embodiments, the subject, prior to treatment, has a left ventricular wall thickness of 15 mm or thicker (e.g., about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 mm or thicker).

In some embodiments, the subject, prior to treatment, has a body mass index (BMI) of about 18 to about 37 kg/m². In some embodiments, the subject, prior to treatment, has a body mass index (BMI) of about 18 to about 50 kg/m² or greater (e.g., about 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 kg/m²).

In some embodiments, the subject, prior to treatment, has LVEF of 55% or higher (e.g., 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, or 80% or higher).

In some embodiments, the subject, prior to treatment, has a LVOT gradient of 30 mmHg or higher (e.g., about 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 mmHg or higher).

In some embodiments, the subject, prior to treatment, has a post-exercise LVOT gradient of 50 mmHg or higher (e.g., about 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 mmHg or higher).

In another aspect defined in claim 9, provided herein is Compound 1 or a pharmaceutically acceptable salt thereof, for use in a method of treating myocardial diastolic dysfunction in a subject in need thereof, the method comprising administering a therapeutically effective amount of Compound 1, having the formula: 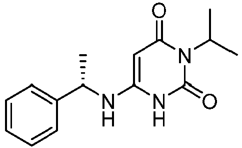 or a pharmaceutically acceptable salt thereof, to said subject, wherein said therapeutically effective amount is a total daily dosage of 2 mg to 15 mg +/- 10%.

In some embodiments, the therapeutically effective amount is a total daily dosage of 2 mg +/- 10%. In some embodiments, the therapeutically effective amount is a total daily dosage of 10 mg to 15 mg +/- 10%.

Subjects in need of treatment for diastolic dysfunction include subjects from a patient population characterized by non-obstructive hypertrophic cardiomyopathy (non-o HCM), or subjects characterized by heart failure with preserved ejection fraction (HFpEF). Subjects in need of treatment for diastolic dysfunction include subjects who exhibit left ventricle stiffness as measured by echocardiography or left ventricle stiffness as measured by cardiac magnetic resonance.

In some embodiments, the subject in need thereof is from a patient population characterized by HFpEF. In some embodiments, the subject having HFpEF has an ejection fraction of ≥50% and has evidence of abnormal diastolic function. Abnormal diastolic function includes impaired left ventricle relaxation, filling, diastolic distensibility, or stiffness. These traits can be measured using echocardiography. In some embodiments, subjects are considered to have abnormal diastolic function when at least one of the following echocardiography values are met septal e' < 7 cm/sec; lateral e' < 10 cm/sec, average E/e' ratio > 14; LA volume index > 34mL/m²; peak TR velocity > 2.8 m / sec. In some embodiments, subjects are considered to have abnormal diastolic function when at least three of the above listed values are met.

In some embodiments, the subject in need thereof exhibits left ventricle stiffness as measured by echocardiography. A subject is considered to have left ventricle stiffness as measured by echocardiography when at least one of the following characteristics are met: mitral E/A ratio > 0.8; septal e' < 7 cm/sec; lateral e' < 10 cm/sec, average E/e'≥ 14; LA volume index > 34 mL/m²; peak TR velocity > 2.8 m / sec. In some embodiments, subjects are considered to have left ventricle stiffness when at least three of the above listed values are met.

Further determining factors for diagnosing diastolic dysfunction using echocardiography are described in J Am Soc Echocardiogr. 29(4):277-314 (2016).

In some embodiments, the subject in need thereof exhibits left ventricle stiffness as measured by cardiac magnetic resonance. Cardiac magnetic resonance is used to determine peak filling rate, time to peak filling, and peak diastolic strain rate. Accordingly, in some embodiments, a subject has left ventricle stiffness as measured by cardiac magnetic resonance when at least one of the following characteristics are met: abnormal peak filing rate, time to peak filling, or peak diastolic strain rate.

Subjects with diastolic dysfunction may also display increased levels of biomarkers in the blood. For example, brain natriuretic peptide (BNP) or N-terminal-pro-brain natriuretic peptide (NT-pro BNP) are present at elevated levels in the blood of individuals with diastolic dysfunction. Accordingly, in some embodiments, the subject in need thereof exhibits elevated serum blood levels of brain natriuretic peptide (BNP) or N-terminal-pro-brain natriuretic peptide (NT-pro BNP). In some embodiments, a subject's serum blood levels of BNP is elevated when the concentration is at least 35, 45, 55, 65, 75, 85, 95, 100, 105, 115 pg/mL. In some embodiments, a subject's serum blood levels of BNP is elevated when the concentration is at least 35 pg/mL. In some embodiments, a subject's serum blood levels of BNP is elevated when the concentration is at least 85 pg/mL. In some embodiments, a subject's serum blood level of pro-NT BNP is elevated when the concentration of pro-NT BNP is at least 95, 105, 115, 125, 135, 145, 155, 165, or 175 pg/mL. In some embodiments, a subject's serum blood levels of pro-NT BNP is elevated when the concentration is at least 125 pg/mL. In some embodiments, a subject's serum blood levels of pro-NT BNP is elevated when the concentration is at least 155 pg/mL.

A person of skill in the art will recognize that blood samples can be taken from a number of different locations in the body, the arm and finger being two of the most common locations. In some embodiments, blood is drawn from the arm of the subject.

In some aspects, methods of treating hypertrophic cardiomyopathy include achieving and maintaining a particular blood plasma concentration of Compound 1 during the course of treatment. For example, in some embodiments, methods of treating HCM include achieving and maintaining a blood plasma concentration of Compound 1 from 200 to 750 ng/mL. In some embodiments, the blood plasma concentration of Compound 1 is from 350 to 700 ng/mL.

The amount of Compound 1 that achieves and maintains the desired blood plasma concentration will vary depending on the individual. In some embodiments, the amount administered to achieve and maintain the desired blood plasma concentration of Compound 1 is 2 to 15 mg. In some embodiments, the amount administered to achieve and maintain the desired blood plasma concentration of Compound 1 is 2.5 to 15 mg. In some embodiments, the amount administered to achieve and maintain the desired blood plasma concentration of Compound 1 is 5 mg. In some embodiments, the amount administered to achieve and maintain the desired blood plasma concentration of Compound 1 is 2 mg. In some embodiments, the amount administered to achieve and maintain the desired blood plasma concentration of Compound 1 is 2.5 mg. In some embodiments, the amount administered to achieve and maintain the desired blood plasma concentration of Compound 1 is 10 mg. In some embodiments, the amount administered to achieve and maintain the desired blood plasma concentration of Compound 1 is 15 mg. In some embodiments, the amount administered to an individual to achieve and maintain the desired blood plasma concentration is adjusted during the treatment based on measured levels. When the blood plasma concentration of Compound 1 exceeds the upper threshold, the amount of Compound 1 administered is lowered, and when the blood plasma concentration of Compound 1 is below the lower threshold, the amount of Compound 1 administered is increased. As a non-limiting example, the amount of Compound 1 administered can be lowered from 5 mg to 2 mg, from 5 to 2.5 mg, from 10 mg to 7.5 mg, from 7.5 mg to 5 mg, from 15 to 12.5 mg, or from 12.5 mg to 10 mg. As additional non-limiting examples, the amount of Compound 1 administered can be increased from 5 mg to 7.5 mg, from 5 mg to 10 mg, from 5 mg to 7.5 mg, from 10 mg to 12.5 mg or from 10 mg to 15 mg.

Also described herein are methods of treating hypertrophic cardiomyopathy in an individual in need thereof comprising administering Compound 1 to the individual in an amount effective to achieve and maintain a blood plasma concentration of Compound 1 from 200 to 750 ng/mL. The methods of treating hypertrophic cardiomyopathy in an individual in need thereof may comprise administering Compound 1 to the individual in an amount effective to achieve and maintain a blood plasma concentration of Compound 1 from 350 to 700 ng/mL.

Also described herein are methods of treating hypertrophic cardiomyopathy in an individual in need thereof comprising administering Compound 1 to the individual in an amount effective to achieve and maintain a blood plasma concentration of Compound 1 from 150 to 1000 ng/mL. The methods of treating hypertrophic cardiomyopathy in an individual in need thereof may comprise administering Compound 1 to the individual in an amount effective to achieve and maintain a blood plasma concentration of Compound 1 from 200 to 1000 ng/mL.

In some embodiments, 2 to 15 mg of Compound 1 achieves and maintains a blood plasma concentration of Compound 1 from 200 to 750 ng/mL. In some embodiments, 5 to 15 mg of Compound 1 achieves and maintains a blood plasma concentration of Compound 1 from 200 to 750 ng/mL. In some embodiments, 2.5 mg +/- 10% of Compound 1 achieves and maintains a blood plasma concentration of Compound 1 from 200 to 750 ng/mL. In some embodiments, 5 mg +/- 10% of Compound 1 achieves and maintains a blood plasma concentration of Compound 1 from 200 to 750 ng/mL. In some embodiments, 10 mg +/-10% of Compound 1 achieves and maintains a blood plasma concentration of Compound 1 from 200 to 750 ng/mL. In some embodiments, 15 mg +/- 10% of Compound 1 achieves and maintains a blood plasma concentration of Compound 1 from 200 to 750 ng/mL.

In some embodiments, 2 to 15 mg of Compound 1 achieves and maintains a blood plasma concentration of Compound 1 from 350 to 700 ng/mL. In some embodiments, 5 to 15 mg of Compound 1 achieves and maintains a blood plasma concentration of Compound 1 from 350 to 700 ng/mL. In some embodiments, 2.5 mg +/- 10% of Compound 1 achieves and maintains a blood plasma concentration of Compound 1 from 350 to 700 ng/mL. In some embodiments, 5 mg +/- 10% of Compound 1 achieves and maintains a blood plasma concentration of Compound 1 from 350 to 700 ng/mL. In some embodiments, 10 mg +/-10% of Compound 1 achieves and maintains a blood plasma concentration of Compound 1 from 350 to 700 ng/mL. In some embodiments, 15 mg +/- 10% of Compound 1 achieves and maintains a blood plasma concentration of Compound 1 from 350 to 700 ng/mL.

In some embodiments, 2 to 15 mg of Compound 1 achieves and maintains a blood plasma concentration of Compound 1 from 200 to 400 ng/mL. In some embodiments, 5 to 15 mg of Compound 1 achieves and maintains a blood plasma concentration of Compound 1 from 200 to 400 ng/mL. In some embodiments, 2.5 mg +/- 10% of Compound 1 achieves and maintains a blood plasma concentration of Compound 1 from 200 to 400 ng/mL. In some embodiments, 5 mg +/- 10% of Compound 1 achieves and maintains a blood plasma concentration of Compound 1 from 200 to 400 ng/mL. In some embodiments, 10 mg +/-10% of Compound 1 achieves and maintains a blood plasma concentration of Compound 1 from 200 to 400 ng/mL. In some embodiments, 15 mg +/- 10% of Compound 1 achieves and maintains a blood plasma concentration of Compound 1 from 200 to 400 ng/mL.

In another aspect defined in claim 10, provided herein is Compound 1 or a pharmaceutically acceptable salt thereof, for use in methods of treating hypertrophic cardiomyopathy (HCM) in an individual in need thereof comprising
administering a daily dose of 5 mg of Compound 1 to the individual for at least one week, and
increasing said daily dose to 10 mg when the blood plasma concentration of Compound 1 in the individual is below 200 ng/mL; or
decreasing said daily dose to 2.5 mg when the blood plasma concentration of Compound 1 in the individual is above 750 ng/mL; or
maintaining said daily dose at 5 mg of Compound 1 when the blood plasma concentration of Compound 1 in the individual is from 200 to 750 ng/mL.

In some embodiments, after administration of 5 mg of Compound 1 for at least one week,
said daily dose is increased to 10 mg when the blood plasma concentration of Compound 1 in the individual is below 350 ng/mL;
said daily dose in decreased to 2.5 mg when the blood plasma concentration of Compound 1 in the individual is above 700 ng/mL;
said daily dose is maintained at 5 mg when the blood plasma concentration of Compound 1 in the individual is from 350 ng/mL to 700 ng/mL.

In some embodiments, the increase in daily dose is only performed when in addition to the above noted blood plasma concentration of Compound 1, the individuals Valsalva gradient is greater than or equal to 30 mm Hg. If the individuals Valsalva gradient is less than 30 mm Hg and the blood plasma concentration of Compound 1 in the individual is below the noted concentration, the individual's daily dose is maintained.

In some embodiments, the individual is administered 5 mg of Compound 1 for at least two weeks. In some embodiments, the individual is administered 5 mg of Compound 1 for at least four weeks. In some embodiments, the individual is administered 5 mg of Compound 1 for at least six weeks. In some embodiments, the individual is administered 5 mg of Compound 1 for at least eight weeks.

In some embodiments, after administration of 5 mg of Compound 1 for at least one week, said daily dose is increased to 10 mg, and a daily dose of 10 mg of Compound 1 is administered for at least one week, said method further comprising
increasing said daily dose to 15 mg when the blood plasma concentration of Compound 1 in the individual is below 200 ng/mL; or
decreasing said daily dose to 5 mg when the blood plasma concentration of Compound 1 in the individual is above 750 ng/mL; or
maintaining said daily dose at 10 mg of Compound 1 when the blood plasma concentration of Compound 1 in the individual is from 200 to 750 ng/mL.

In some embodiments, after administration of 5 mg of Compound 1 for at least one week, said daily dose is increased to 10 mg, and a daily dose of 10 mg of Compound 1 is administered for at least one week, said method further comprising
increasing said daily dose to 15 mg when the blood plasma concentration of Compound 1 in the individual is below 350 ng/mL; or
decreasing said daily dose to 5 mg when the blood plasma concentration of Compound 1 in the individual is above 700 ng/mL; or
maintaining said daily dose at 10 mg of Compound 1 when the blood plasma concentration of Compound 1 in the individual is from 350 to 700 ng/mL.

In some embodiments, the increase in daily dose is only performed when in addition to the above noted blood plasma concentration of Compound 1, the individuals Valsalva gradient is greater than or equal to 30 mm Hg. If the individuals Valsalva gradient is less than 30 mm Hg and the blood plasma concentration of Compound 1 in the individual is below the noted concentration, the individual's daily dose is maintained.

In some embodiments, the individual is administered 10 mg of Compound 1 for least two weeks. In some embodiments, the individual is administered 10 mg of Compound 1 for least four weeks. In some embodiments, the individual is administered 10 mg of Compound 1 for least six weeks. In some embodiments, the individual is administered 10 mg of Compound 1 for least eight weeks.

In some embodiments, after administration of 5 mg of Compound 1 for at least a week, said daily dose is decreased to 2 mg, and 2 mg of Compound 1 is administered for at least one additional week, said method further comprising
increasing said daily dose to 5 mg when the blood plasma concentration of Compound 1 in the individual is below 200 ng/mL; or
maintaining said daily dose at 2 mg of Compound 1 when the blood plasma concentration of Compound 1 in the individual is from 200 to 750 ng/mL.

In some embodiments, after administration of 5 mg of Compound 1 for at least a week, said daily dose is decreased to 2.5 mg, and 2 mg of Compound 1 is administered for at least one additional week, said method further comprising
increasing said daily dose to 5 mg when the blood plasma concentration of Compound 1 is below 350 ng/mL; or
maintaining said daily dose at 2 mg of Compound 1 when the blood plasma concentration of Compound 1 is from 350 to 700 ng/mL.

In some embodiments, the increase in daily dose is only performed when in addition to the above noted blood plasma concentration of Compound 1, the individuals Valsalva gradient is greater than or equal to 30 mm Hg. If the individuals Valsalva gradient is less than 30 mm Hg and the blood plasma concentration of Compound 1 in the individual is below the noted concentration, the individual's daily dose is maintained.

In some embodiments, the individual is administered 2 mg of Compound 1 for least two weeks. In some embodiments, the individual is administered 2 mg of Compound 1 for least four weeks. Alternatively, in some embodiments, 2.5 mg of Compound 1 may be administered instead of 2 mg.

In some embodiments, the HCM is obstructive HCM (oHCM). In some embodiments, the HCM is non-obstructive HCM.

In another aspect defined in claim 11, provided herein is Compound 1 or a pharmaceutically acceptable salt thereof, for use in methods of treating hypertrophic cardiomyopathy (HCM) in an individual in need thereof by achieving and maintaining a blood plasma concentration of Compound 1 from 200 to 750 ng/mL, said method comprising
a) administering a daily dose selected from the group consisting of 2.5, 5, 10, and 15 mg of Compound 1 to an individual.

In some embodiments, the blood plasma concentration of Compound 1 is 350 to 700 ng/mL.

In some embodiments, the daily dose of step a) above, is selected from the group consisting of 2, 2.5, 5, 10, 12.5, and 15 mg of Compound 1.

In some embodiments, the methods further comprise
b-i) increasing said daily dose to the next highest dosage amount when the blood plasma concentration of Compound 1 in the individual is below 200 ng/mL; or
b-ii) decreasing said daily dose to the next lowest dosage amount when the blood plasma concentration of Compound 1 in the individual is above 750 ng/mL; or
b-iii) maintaining said daily dose of Compound 1 when the blood plasma concentration of Compound 1 in the individual is from 200 to 750 ng/mL.

In some embodiments, the methods further comprise
b-i) increasing said daily dose to the next highest dosage amount when the blood plasma concentration of Compound 1 in the individual is below 350 ng/mL; or
b-ii) decreasing said daily dose to the next lowest dosage amount when the blood plasma concentration of Compound 1 in the individual is above 700 ng/mL; or
b-iii) maintaining said daily dose of Compound 1 when the blood plasma concentration of Compound 1 in the individual is from 350 to 700 ng/mL.

In some embodiments, the increase in daily dose (step b-i)) is only performed when in addition to the above noted blood plasma concentration of Compound 1, the individuals Valsalva gradient is greater than or equal to 30 mm Hg. If the individuals Valsalva gradient is less than 30 mm Hg and the blood plasma concentration of Compound 1 in the individual is below the noted concentration, the individual's daily dose is maintained.

In some embodiments, the methods further comprise
c) repeating step b) every two to four weeks.

In some embodiments, the methods further comprise
c) repeating step b) every two to six weeks.

In some embodiments, the methods further comprise
c) repeating step b) every two to eight weeks.

In some embodiments, the methods further comprise
c) repeating step b) every two to twelve weeks.

In some embodiments, steps b) is repeated every 2 weeks. In some embodiments, steps b) is repeated every 4 weeks. In some embodiments, steps b) is repeated every 6 weeks. In some embodiments, steps b) is repeated every 8 weeks. In some embodiments, steps b) is repeated every 10 weeks. In some embodiments, steps b) is repeated every 12 weeks.

In some embodiments, the HCM is obstructive HCM (oHCM). In some embodiments, the HCM is non-obstructive HCM.

In some embodiments, provided herein are methods of treating hypertrophic cardiomyopathy (HCM) in an individual in need thereof by achieving and maintaining a blood plasma concentration of Compound 1 in the individual from between 200 to 750 ng/mL, said method comprising administering a daily dose 2.5 to 15 mg Compound 1 and increasing said daily dose when the blood plasma concentration of Compound 1 in the individual is less than 200 ng/mL; or decreasing said daily does when the blood plasma concentration of Compound 1 in the individual is greater than 750 ng/mL.

In some embodiments, provided herein are methods of treating hypertrophic cardiomyopathy (HCM) in an individual in need thereof by achieving and maintaining a blood plasma concentration of Compound 1 in the individual from between 350 to 700 ng/mL, said method comprising administering a daily dose 2.5 to 15 mg Compound 1 and increasing said daily dose when the blood plasma concentration of Compound 1 in the individual is less than 350 ng/mL; or decreasing said daily does when the blood plasma concentration of Compound 1 in the individual is greater than 700 ng/mL.

In some embodiments, provided herein are methods of treating hypertrophic cardiomyopathy (HCM) in an individual in need thereof by achieving and maintaining a blood plasma concentration of Compound 1 in the individual from between 200 to 400 ng/mL, said method comprising administering a daily dose 2.5 to 15 mg Compound 1 and increasing said daily dose when the blood plasma concentration of Compound 1 in the individual is less than 200 ng/mL; or decreasing said daily does when the blood plasma concentration of Compound 1 in the individual is greater than 400 ng/mL.

In some embodiments, the increase in daily dose of Compound 1 is only performed when in addition to the above noted blood plasma concentration of Compound 1, the individuals Valsalva gradient is greater than or equal to 30 mm Hg. If the individuals Valsalva gradient is less than 30 mm Hg and the blood plasma concentration of Compound 1 in the individual is below the noted concentration, the individual's daily dose is maintained.

In some embodiments, the HCM is obstructive HCM (oHCM). In some embodiments, the HCM is non-obstructive HCM.

In some embodiments, provided herein are methods of treating hypertrophic cardiomyopathy (HCM) in an individual in need thereof by achieving and maintaining a blood plasma concentration of Compound 1 from between 200 to 750 ng/mL, said method comprising
a) administering a daily dose 2.5 to 15 mg of Compound 1 for at least 1 week;
b) measuring the blood plasma concentration of Compound 1 in the individual; and
c) increasing the daily dose of Compound 1 when said blood plasma concentration of Compound 1 in the individual is less than 200 ng/mL; or
   decreasing the daily dose of Compound 1 when said blood plasma concentration of Compound 1 in the individual is greater than 750 ng/mL; or
   continuing the same daily dose of Compound 1 when said blood plasma concentration of Compound 1 in the individual is from 200 to 750 ng/mL.

In some embodiments, the increase in daily dose in step c) is only performed when in addition to the above noted blood plasma concentration of Compound 1, the individuals Valsalva gradient is greater than or equal to 30 mm Hg. If the individuals Valsalva gradient is less than 30 mm Hg and the blood plasma concentration of Compound 1 in the individual is below the noted concentration, the individual's daily dose is maintained.

In some embodiments, the method further comprises
d) repeating steps a) to c) every 1 to 12 weeks.

In some embodiments, provided herein are methods of treating hypertrophic cardiomyopathy (HCM) in an individual in need thereof by achieving and maintaining a blood plasma concentration of Compound 1 in the individual from between 350 to 700 ng/mL, said method comprising
a) administering a daily dose 2 to 15 mg of Compound 1 for at least 1 week;
b) measuring the blood plasma concentration of Compound 1 in the individual; and
c) increasing the daily dose of Compound 1 when said blood plasma concentration of Compound 1 in the individual is less than 350 ng/mL; or
   decreasing the daily dose of Compound 1 when said blood plasma concentration of Compound 1 in the individual is greater than 700 ng/mL; or
   continuing the same daily dose of Compound 1 when said blood plasma concentration of Compound 1 in the individual is from 350 to 700 ng/mL.

In some embodiments, the increase in daily dose in step c) is only performed when in addition to the above noted blood plasma concentration of Compound 1, the individuals Valsalva gradient is greater than or equal to 30 mm Hg. If the individuals Valsalva gradient is less than 30 mm Hg and the blood plasma concentration of Compound 1 in the individual is below the noted concentration, the individual's daily dose is maintained.

In some embodiments, the method further comprises
d) repeating steps a) to c) every 1 to 12 weeks.

In some embodiments, steps a) to c) are repeated every 2 weeks. In some embodiments, steps a) to c) are repeated every 4 weeks. In some embodiments, steps a) to c) are repeated every 6 weeks. In some embodiments, steps a) to c) are repeated every 8 weeks. In some embodiments, steps a) to c) are repeated every 10 weeks. In some embodiments, steps a) to c) are repeated every 12 weeks.

In some embodiments, the daily dose is selected from the group consisting of 2, 2.5, 5, 7.5, 10, 12.5, and 15 mg of Compound 1, and the daily dose increase or decrease of Compound 1 in step c) is to the next highest or lowest amount of Compound 1. In some embodiments, the daily dose is selected from the group consisting of 2.5, 5, 10, and 15 mg of Compound 1, and the daily dose increase or decrease of Compound 1 in step c) is to the next highest or lowest amount of Compound 1. In some embodiments, the initial daily dose of Compound 1 in step a) is 5 mg.

In some embodiments, the HCM is obstructive HCM (oHCM). In some embodiments, the HCM is non-obstructive HCM.

In another aspect defined in claim 12, provided herein is Compound 1 or a pharmaceutically acceptable salt thereof, for use in methods of treating hypertrophic cardiomyopathy (HCM) in an individual in need thereof comprising administering to the individual a total daily dose of Compound 1 in an amount selected from the group consisting of 2 mg, 5 mg, 10 mg, and 15 mg. In some embodiments, the total daily dose of Compound 1 is selected from the group consisting of 2.5 mg, 5 mg, 7.5 mg, 10 mg, 12.5 mg, and 15 mg.

In some embodiments, said total daily dose of Compound 1 is 2 mg. In some embodiments, said total daily dose of Compound 1 is 2.5 mg. In some embodiments, said total daily dose of Compound 1 is 5 mg. In some embodiments, said total daily dose of Compound 1 is 7.5 mg. In some embodiments, said total daily dose of Compound 1 is 10 mg. In some embodiments, said total daily dose of Compound 1 is 12.5 mg. In some embodiments, said total daily dose of Compound 1 is 15 mg.

In some embodiments, said HCM is obstructive HCM (oHCM).In some embodiments, said HCM is non-obstructive HCM.

In some embodiments, provided herein are methods of treating hypertrophic cardiomyopathy (HCM) in an individual in need thereof, said method comprising
a) administering a daily dose of 2 to 15 mg of Compound 1 to said individual for at least 1 week;
b) monitoring said individual; and
c) adjusting the daily dose of Compound 1 based on pharmacokinetic and/or pharmacodynamics measures.

In some embodiments, monitoring in step b) uses a noninvasive monitoring technique. Noninvasive monitoring techniques include, but are not limited to, measuring peak oxygen update (VO₂) levels, assessing NYHA functional classification, measuring Valsalva gradient, measuring heart activity with an an echocardiogram, and combinations thereof. In some embodiments, the noninvasive monitoring technique employed is measuring peak VO₂.

In some embodiments, monitoring in step b) includes measuring the blood plasma concentration of Compound 1 in the individual or the blood plasma concentration of N-terminal-pro-brain natriuretic peptide (NT-pro BNP) in the individual.

Results from the monitoring step are used to adjust the daily dose of Compound 1 to best treat the individual. For example, in some embodiments the adjusting step includes:
increasing the daily dose of Compound 1 when said individual demonstrates no clinical improvement in one or more of the noninvasive monitoring techniques; or
decreasing the daily dose of Compound 1 when said individual demonstrates clinical regression in one or more of the noninvasive monitoring techniques; or
continuing the same daily dose of Compound 1 when said individual demonstrates a clinical improvement in one or more of the noninvasive monitoring techniques.

Assessing clinical improvement, stasis, or regression will depend on the monitoring techniques employed. For example, when assessing peak VO₂ levels, an increase in peak VO₂ of 2.0 mL/kg/min or more from baseline is considered a clinical improvement, a decrease in peak VO₂ of 2.0 mL/kg/min or more from baseline is considered a clinical regression, and a less than 2.0 mL/kg/min variance in peak VO₂ is considered no clinical change. Echocardiography is particularly useful in assessing left ventricle stiffness. A subject is considered to have abnormal left ventricle stiffness when at least one of the following characteristics are met: mitral E/A ratio > 0.8; E/e'≥ 14; septal e' < 7 cm/sec; lateral e' < 10 cm/sec. A clinical improvement or regression in left ventricle stiffness is when there's at least a 5% change one of the listed characteristics, whereas a less than 5% change is considered no clinical improvement.

In some embodiments, blood plasma concentration of Compound 1 is monitored and said adjusting step comprises
increasing the daily dose of Compound 1 when said blood plasma concentration of Compound 1 in the individual is less than 200 ng/mL; or
decreasing the daily dose of Compound 1 when said blood plasma concentration of Compound 1 in the individual is greater than 750 ng/mL; or
continuing the same daily dose of Compound 1 when said blood plasma concentration of Compound 1 in the individual is from 200 to 750 ng/mL.

In some embodiments, blood plasma concentration of Compound 1 is monitored and said adjusting step comprises
increasing the daily dose of Compound 1 when said blood plasma concentration of Compound 1 in the individual is less than 350 ng/mL; or
decreasing the daily dose of Compound 1 when said blood plasma concentration of Compound 1 in the individual is greater than 700 ng/mL; or
continuing the same daily dose of Compound 1 when said blood plasma concentration of Compound 1 in the individual is from 350 to 700 ng/mL.

In some embodiments, the increase in daily dose is only performed when in addition to the above noted blood plasma concentration of Compound 1, the individuals Valsalva gradient is greater than or equal to 30 mm Hg. If the individuals Valsalva gradient is less than 30 mm Hg and the blood plasma concentration of Compound 1 in the individual is below the noted concentration, the individual's daily dose is maintained.

In some embodiments, the method further comprises
d) repeating steps a) to c) every 1 to 12 weeks.

In some embodiments, steps a) to c) are repeated every 2 weeks. In some embodiments, steps a) to c) are repeated every 4 weeks. In some embodiments, steps a) to c) are repeated every 6 weeks. In some embodiments, steps a) to c) are repeated every 8 weeks. In some embodiments, steps a) to c) are repeated every 10 weeks. In some embodiments, steps a) to c) are repeated every 12 weeks.

In some embodiments, the method further comprises
d) repeating steps a) to c) every 1 to 12 weeks.

In some embodiments, steps a) to c) are repeated every 2 weeks. In some embodiments, steps a) to c) are repeated every 4 weeks. In some embodiments, steps a) to c) are repeated every 6 weeks. In some embodiments, steps a) to c) are repeated every 8 weeks. In some embodiments, steps a) to c) are repeated every 10 weeks. In some embodiments, steps a) to c) are repeated every 12 weeks.

In some embodiments, the daily dose is selected from the group consisting of 2, 2.5, 5, 7.5, 10, 12.5, and 15 mg of Compound 1, and the daily dose increase or decrease of Compound 1 in step c) is to the next highest or lowest amount of Compound 1. In some embodiments, the daily dose is selected from the group consisting of 2, 2.5, 5, 10, and 15 mg of Compound 1, and the daily dose increase or decrease of Compound 1 in step c) is to the next highest or lowest amount of Compound 1. In some embodiments, the initial daily dose of Compound 1 in step a) is 5 mg.

In some embodiments, the HCM is obstructive HCM (oHCM). In some embodiments, the HCM is non-obstructive HCM.

Also contemplated herein are methods of treating hypertrophic cardiomyopathy (HCM) in an individual in need thereof by administering a loading dose to an individual. A "loading dose" establishes a baseline amount of Compound 1 in the blood plasma of an individual such that they quickly achieve and maintain a desired blood plasma concentration of Compound 1. "Loading dose" time frames include 3 days, 5 day, 7 days, 10 days, or 14 days. "Loading dose" amounts includes a total daily dose of 15 mg once per day, 12.5 mg once per day, 10 mg once per day, 7.5 mg once per day. After completing the "loading dose," the dose adjustments described herein can be used to readjust the dosing level to achieve and maintain the desired blood plasma concentration.

As such, provided herein are methods of treating hypertrophic cardiomyopathy (HCM) in an individual in need thereof, the method comprising
a) administering a loading dose of Compound 1 to the individual for 3 to 14 days;
b) measuring the blood plasma concentration of Compound 1 in the individual; and
c) adjusting the daily dose of Compound 1 based on the blood plasma concentrations described in this application to achieve and maintain the desired blood plasma concentration of Compound 1.

Also described herein are methods of eliminating the symptoms associated with, amelioration of, or reducing the incidence of hypertrophic cardiomyopathy (HCM) in an individual in need thereof. The method includes administering to a subject in need thereof a therapeutically effective amount of Compound 1 or a pharmaceutically acceptable salt thereof, where the therapeutically effective amount is a total daily dosage of about 2 mg to 50 mg. The total daily dosage of Compound 1 may be about 2 mg to 30 mg, 10 mg to 20 mg, 2 mg to 10 mg, or 2 mg to 5 mg. The total daily dosage of Compound 1 may be 2.5 mg to 15 mg. The total daily dosage of Compound 1 may be 5 mg to 15 mg. The total daily dosage of Compound 1 may be 2 mg to 15 mg. The total daily dosage of Compound 1 may be 2.5 mg to 15 mg. The methods of eliminating the symptoms associated with the onset, amelioration of, or reducing the incidence of HCM may further include the dose adjustment steps described herein to achieve and maintain a desired blood plasma concentration.

Also described herein are methods of preventing or delaying the onset of hypertrophic cardiomyopathy (HCM) in an individual in need thereof. The method includes administering to a subject in need thereof a therapeutically effective amount of Compound 1 or a pharmaceutically acceptable salt thereof, where the therapeutically effective amount is a total daily dosage of about 2 mg to 50 mg. The total daily dosage of Compound 1 may be about 2 mg to 30 mg, 10 mg to 20 mg, 2 mg to 10 mg, or 2 mg to 5 mg. The total daily dosage of Compound 1 may be 2.5 mg to 15 mg. The total daily dosage of Compound 1 may be 5 mg to 15 mg. The total daily dosage of Compound 1 may be 2 mg to 15 mg. The total daily dosage of Compound 1 may be 2.5 mg to 15 mg. The methods of preventing or delaying the onset of HCM may further include the dose adjustment steps described herein to achieve and maintain a desired blood plasma concentration.

### Pharmaceutical Compositions

Compound 1 can be prepared in various compositions suitable for delivery to a subject. A composition suitable for administration to a subject typically comprises Compound 1, or a pharmaceutically acceptable salt thereof and a pharmaceutically acceptable excipient.

The pharmaceutical compositions for the administration of Compound 1 or pharmaceutically acceptable salts described herein may conveniently be presented in unit dosage form and may be prepared by any of the methods known in the art of pharmacy and drug delivery. All methods include the step of bringing the active ingredient into association with a carrier containing one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition, the active agent is generally included in an amount sufficient to produce the desired effect upon myocardial contractility (i.e. to decrease the often supranormal systolic contractility in HCM) and to improve left ventricular relaxation in diastole. Such improved relaxation can alleviate symptoms in hypertrophic cardiomyopathy and other etiologies of diastolic dysfunction. It can also ameliorate the effects of diastolic dysfunction causing impairment of coronary blood flow, improving the latter as an adjunctive agent in angina pectoris and ischemic heart disease. It can also confer benefits on adverse left ventricular remodeling in HCM and other causes of left ventricular hypertrophy due to chronic volume or pressure overload from, e.g., valvular heart disease or systemic hypertension.

The pharmaceutical compositions containing Compound 1, or a pharmaceutically acceptable salt thereof, may be in a form suitable for oral use, for example, as tablets, troches, lozenges, aqueous or oily suspensions, dispersible powders or granules, emulsions, hard or soft capsules, syrups, elixirs, solutions, buccal patch, oral gel, chewing gum, chewable tablets, effervescent powder and effervescent tablets. Compositions intended for oral use may be prepared according to any method known to the art for the manufacture of pharmaceutical compositions and such compositions may contain one or more agents selected from the group consisting of sweetening agents, flavoring agents, coloring agents, antioxidants and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as cellulose, silicon dioxide, aluminum oxide, calcium carbonate, sodium carbonate, glucose, mannitol, sorbitol, lactose, calcium phosphate or sodium phosphate; granulating and disintegrating agents, for example, corn starch, or alginic acid; binding agents, for example PVP, cellulose, PEG, starch, gelatin or acacia, and lubricating agents, for example magnesium stearate, stearic acid or talc. The tablets may be uncoated or they may be coated, enterically or otherwise, by known techniques to delay disintegration and absorption in the gastrointestinal tract and thereby provide a sustained action over a longer period. For example, a time delay material such as glyceryl monostearate or glyceryl distearate may be employed. They may also be coated to form osmotic therapeutic tablets for controlled release.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil. Additionally, emulsions can be prepared with a non-water miscible ingredient such as oils and stabilized with surfactants such as mono-diglycerides, PEG esters and the like.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions described herein may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. Oral solutions can be prepared in combination with, for example, cyclodextrin, PEG and surfactants.

### Pharmaceutical Dosage Forms

The present disclosure includes novel pharmaceutical dosage forms of Compound 1 and pharmaceutically acceptable salts thereof. The dosage forms described herein are suitable for oral administration to a subject. The dosage form may be in any form suitable for oral administration, including, but not limited to, a capsule or a tablet.

Described herein is a single unit dosage capsule or tablet form containing 2-10 mg or 2-15 mg of Compound 1, having the formula:

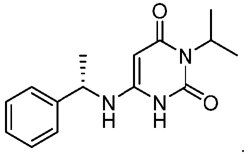

The amount of Compound 1 may be from about 2 to 5 mg. The amount of Compound 1 may be from about 5 to 10 mg. The amount of Compound 1 may be about 2.5 mg. The amount of Compound 1 may be about 5 mg. The amount of Compound 1 may be about 7.5 mg. The amount of Compound 1 may be about 10 mg.

The single unit dosage form of Compound 1 may be a capsule. The single unit dosage form of Compound 1 may be a tablet.

The single unit dosage form may be in a capsule of size #0, #1, #2, #3, #4, or #5. The single unit dosage form may be in a capsule of size #4. The single unit dosage form may be in a capsule of size #5.

### Kits

The disclosure also encompasses kits comprising pharmaceutical compositions and dosage forms of the invention.

Also described herein is a kit that includes Compound 1. Some of the kits described herein include a label describing a method of administering Compound 1. Some of the kits described herein include a label describing a method of treating hypertrophic cardiomyopathy (HCM). The kits described herein may include a label describing a method of treating obstructive HCM (oHCM). The kits described herein may include a label describing a method of reducing a subject's left verntricular outflow tract (LVOT) gradient. The kits described herein may include a label describing a method of reducing a subject's resting left verntricular outflow tract (LVOT) pressure gradient. The kits described herein may include a label describing a method of reducing a subject's stress left verntricular outflow tract (LVOT) gradient.

The compositions described herein, including but not limited to, compositions comprising Compound 1 in a bottle, jar, vial, ampoule, tube, or other container-closure system approved by the Food and Drug Administration (FDA) or other regulatory body, which may provide one or more dosages containing the compounds. The package or dispenser may also be accompanied by a notice associated with the container in a form prescribed by a governmental agency regulating the manufacture, use, or sale of pharmaceuticals, the notice indicating approval by the agency. The kit may include a formulation or composition as described herein, a container closure system including the formulation or a dosage unit form including the formulation, and a notice or instructions describing a method of use as described herein.

### EXAMPLES

### Example 1. Preparation of (S)-3-Isopropyl-6-((1-phenylethyl) amino) pyrimidine-2, 4(1H,3H)-dione.

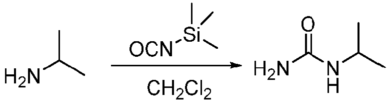

**Compound 1.1. Isopropylurea.** To a stirred solution of isopropylamine (15.3 g, 0.258 mol, 1.0 equiv) in CH₂Cl₂ (200 mL) under argon at 0 °C was added dropwise trimethylsilyl isocyanate (30 g, 0.26 mol, 1.0 equiv). The resulting mixture was allowed to reach ambient temperature and stirred overnight. After cooling to 0 °C, CH₃OH (100 mL) was added dropwise. The resulting solution was stirred for 2 hours (h) at room temperature and then concentrated under reduced pressure. The crude residue was recrystallized from CH₃OH:Et₂O (1:20) to yield 15.4 g (58%) the title compound as a white solid. LC/MS: m/z (ES+) 103 (M+H)⁺.

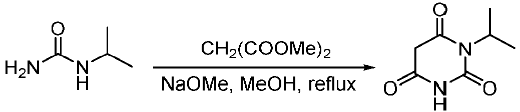

**Compound 1.2. 1-Isopropyl barbituric acid.** To a stirred solution of 1.1 (14.4 g, 0.14 mol, 1.00 equiv) in CH₃OH (500 mL) were added dimethyl malonate (19.55 g, 0.148 mol, 1.05 equiv) and sodium methoxide (18.9 g, 0.35 mol, 2.50 equiv). The resulting mixture was stirred overnight at 65 °C. After cooling to ambient temperature and then to 0 °C, the pH was carefully adjusted to 3 using aqueous concentrated HCl. The resulting mixture was concentrated under reduced pressure. The residue was taken up in EtOH (200 mL) and filtered. The filtrate was concentrated under reduced pressure and the residue was purified by silica gel column chromatography using CH₂Cl₂/CH₃OH (20:1) as eluent to yield 16.8 g (50%) of the title compound as a white solid.. LC/MS: m/z (ES+) 171 (M+H)⁺.^{1 1}H-NMR (300 MHz, d₆-DMSO): δ 11.19 (s, 1H), 4.83 (m, 1H), 3.58 (s, 2H), 1.32 (d, J = 6.0 Hz, 6H).

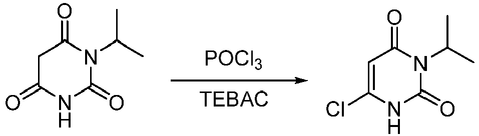

**Compound 1.3. 6-chloro-3-isopropylpyrimidine-2,4(1H,3H)-dione.** To a 100-mL round-bottom flask containing compound **1.2** (11.4 g, 66.99 mmol, 1.00 equiv) under argon were added triethylbenzylammonium chloride (21.3 g, 93.51 mmol, 1.40 equiv) and POCl₃ (30 mL). The resulting mixture was stirred overnight at 50 °C. After cooling to room temperature, the mixture was concentrated under reduced pressure. The residue was dissolved in CH₂Cl₂ (150 mL) followed by slow addition of H₂O (100 mL). The phases were separated and the organic layer was washed with H₂O (100 mL), dried with anhydrous Na₂SO₄, and concentrated under reduced pressure. The crude residue was purified by silica gel column chromatography using EtOAc/petroleum ether (1:1) as eluent to yield 5.12 g (40%) of the title compound as a light yellow solid. ¹H-NMR (300 MHz, d₆-DMSO): δ 12.22 (s, 1H), 5.88 (s, 1H), 4.95 (m, 1H), 1.34 (d, J = 6.0 Hz, 6H).

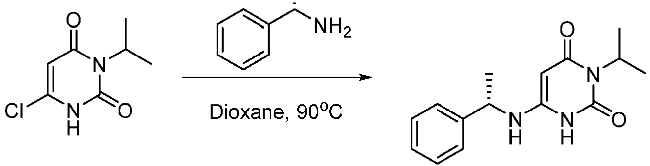

**Compound 1. (5)-3-Isopropyl-6-((1-phenylethyl) amino) pyrimidine-2, 4(1H,3H)-dione.** To a solution of 6-chloro-3-isopropylpyrimidine-2,4(1H,3H)-dione (1.3, 1.0 g, 5.31 mmol) in 1,4-dioxane (20 mL) was added (*S*)-α-methylbenzylamine (Sigma-Aldrich, 1.43 g, 11.7 mmol, 2.2 equiv). The reaction mixture was stirred at 80 °C for 24 h. After cooling to ambient temperature, the mixture was concentrated under reduced pressure. The residual was taken up in EtOAc (70 mL) and washed with aqueous 1N HCl (2 x 50 mL) and brine (40 mL). The organic layer was dried with anhydrous Na₂SO₄ and then concentrated under reduced pressure to half the original volume to yield a precipitate. Hexane (20 mL) was added and the mixture was stirred at room temperature. The resulting solid was collected by filtration, washed with hexane (20 mL), and dried to yield 1.0 g (69%) of the title compound as a white solid. LC/MS: m/z (ES+) 274 (M+H)⁺. ¹H-NMR (400 MHz, d₆-DMSO): δ 9.77 (s, 1H), 7.32 (m, 4H), 7.24 (m, 1H), 6.50 (d, *J =* 6.8 Hz, 1H), 4.87 (m, 1H), 4.52 (m, 1H), 4.31 (d, *J*=6.8 Hz, 1H), 1.37 (m, 3H ), 1.24 (m, 6H). ¹H NMR (400 MHz, CD₃OD) δ ppm 7.39-7.20 (m, 5H), 5.01 (m, 1H), 4.48 (m, 1H), 1.49 (d, J = 6.7 Hz, 3H), 1.36 (m, 6H).

### Reference Example 2. Phase I Clinical Studies

Three clinical studies have investigated the safety and tolerability of Compound 1 to date; conduct of 2 studies has completed and 1 study is ongoing. A double-blind, placebo-controlled, single ascending-dose study (SAD) (Compound 1-002) was conducted in healthy men. Subjects received single doses ranging from 1 mg to 48 mg. Conduct of an open-label sequential-group SAD study in subjects with clinically stable HCM (Compound 1-001) is also complete; single doses of up to 144 mg were administered. In addition, a double-blind, placebo-controlled, multiple ascending-dose (MAD) study (Compound 1-003) is being conducted in which healthy adult subjects have received multiple doses of Compound 1 up to 25 mg once daily (QD) for up to 28 days.

| **Table 1.** Three Phase 1 Trials of Compound 1 in HCM Patients and Healthy Volunteers. | | | |
|---|---|---|---|
| | Compound 1-001 | Compound 1-002 | Compound 1-003 |
| | Single Ascending Dose (SAD) Trial in HCM Patients | SAD Trial in Healthy Volunteers | Multiple Ascending Dose (MAD) Trial in Healthy Volunteers |
| Trial Design | Open-label, sequential-group, SAD study | Randomized, double-blind, placebo-controlled SAD study | Randomized, double-blind, placebo-controlled MAD study |
| Study Size | 15 HCM patients (2 with LVOT obstruction) | 48 healthy adults | 60 healthy adults |
| Treatment | Three cohorts: single dose of 48, 96 and 144 mg⁽¹⁾ | Six cohorts of eight subjects each: single dose of 1, 2, 6, 12, 24 and 48 mg⁽²⁾ | Five cohorts of 12 subjects each⁽³⁾: 28-day treatment of 2, 6, 12.5, 18.5 and 25 mg⁽⁴⁾ |
| Primary Endpoints | Establish safety and tolerability of single (001 and 002) and multiple (003) oral doses of Compound 1 | | |
| Secondary Endpoints | Document pharmacokinetic (PK) profile and generate evidence of pharmacodynamic (PD) activity | | |

| | | | |
|---|---|---|---|
| (1) 48mg cohort (n=4 patients); 96mg cohort (n=6), 144mg cohort (n=5). (2) Each cohort randomized 6:2 Compound 1 or matching placebo. (3) Each cohort randomized 10:2 Compound 1 or matching placebo. (4) 25mg cohort treated for 25 days. | | | |

### 2.1 Compound 1-002 Trial

In Study Compound 1-002, single doses from 1 mg to 48 mg were administered as an oral suspension to healthy men. All dose levels were well tolerated, with no serious adverse events (SAEs), deaths, or withdrawals from study due to adverse events (AEs) reported. The incidence of treatment-emergent AEs did not appear to increase with dose. The pharmacokinetic (PK) profile of Compound 1 displayed rapid absorption and subsequent distribution followed by a long elimination phase, with a mean elimination half-life of approximately 8 days. Single doses of ≥ 12 mg of Compound 1 were associated with detectable reductions in LV contractility from baseline as assessed by 3 independent echocardiographic measures (left ventricular ejection fraction [LVEF], LV fractional shortening (LVFS), and LVOT velocity time integral [VTI]). At the highest dose studied in healthy subjects (48 mg), mean reduction in LVEF at any time point was -6% relative to baseline. No changes in systolic blood pressure (BP) or heart rate (HR) were detected at any of the dose levels tested in this study.

### 2.2 Compound 1-001 Trial

In Study MYK-461-001, single doses of up to 144 mg were administered to 15 subjects with HCM. All dose levels were well tolerated without deaths, withdrawals due to AEs, or dose-related treatment-emergent AEs, except for 1 of 5 subjects who received the 144 mg dose. This subject experienced an SAE described as a vasovagal reaction characterized by a period of asystole and hypotension that spontaneously resolved without sequelae. In terms of causality assessment, there are no nonclinical or other clinical data to implicate Compound 1 in cardiac ion channel current modulation, or cardiac impulse generation or conduction. Compound 1 has no documented vasoactive properties and is biochemically inactive against smooth muscle myosin. Other potential contributions to the event include chronic β-blockade with metoprolol succinate 150 mg QD, and the observation that the subject inadvertently performed the Valsalva maneuver during the handgrip strength assessment immediately prior to the event.

LVOT obstruction was resolved in patients following a single dose of Compound 1, as shown in the results in **Table 2.** Two subjects enrolled in 001 study displayed an LVOT pressure gradient (upon provocation). Each patient was administered single 96 mg dose of Compound 1. Both patients' gradients were resolved following dosing.

| **Table 2.** LVOT Gradient (mmHg) Value In Patients | | | |
|---|---|---|---|
| | Pre-dose | Post-dose | % Decrease |
| Patient 1 | 42 | 9 | (79%) |
| Patient 2 | 28 | 5 | (82%) |

Preliminary PK analysis in Study Compound 1-001 revealed a PK profile in subjects with HCM similar to that of healthy subjects. All subjects with HCM demonstrated proof of mechanism in terms of documenting reduced LV contractility by at least 1 of 3 independent echocardiographic measures. In 2 subjects, measurable LVOT gradients following Valsalva were reduced to < 10 mm Hg following a single 96 mg dose of Compound 1.

### 2.3 Compound 1-003 Trial

In Study Compound 1-003, 4 cohorts of healthy adult subjects have been dosed, each scheduled to dose for 28 days. Compound 1 was well tolerated at multiple doses up to 25 mg QD. All AEs were mild or moderate in severity, and there were no SAEs. In cohort 3, 3 of 12 subjects who received blinded treatment (either Compound 1 12.5 mg or placebo QD) had diarrhea and/or abdominal cramps. One of these subjects was discontinued due to recurrence of diarrhea upon rechallenge, and the other 2 subjects were successfully restarted on study medication. The investigator considered these gastrointestinal AEs either mild or moderate in severity and likely related to study medication. No gastrointestinal AEs were reported in cohort 4 (Compound 1 25 mg or placebo).

Dosing in cohort 4 was stopped on Day 25 by the investigator, because 5 of 12 subjects who received blinded treatment (either Compound 1 25 mg or placebo QD) achieved the predefined stopping criterion for dose ascension of ≥ 20% reduction in LVEF. All subjects remained asymptomatic.

PD evidence of reduced contractility was apparent in the higher dose-level groups (12.5 mg QD and 25 mg QD). These data, along with the PK and PD data from all subjects in all studies to date, have been incorporated into a robust PK-PD model to help inform a safe dosing strategy for the current study that is also likely to be efficacious.

Overall, favorable safety profile has been demonstrated in HCM patients and healthy volunteers, as shown in **Table 3** following.

| **Table 3.** Summary of Adverse Events. | | |
|---|---|---|
| HCM Patients | • 35 adverse events (AEs) in 12 patients; all mild to moderate | |
| | • One severe adverse event (SAE) | |
| | | - Reported in highest dose cohort (144 mg) |
| | | - Vasovagal reaction; spontaneously resolved without lasting consequences |
| Healthy Volunteers | • 54 AEs reported in 33 subjects; all mild to moderate | |
| | • No SAEs reported | |

Clinically relevant and dose dependent response were observed across all patient groups in the Phase 1 trials.

| **Table 4.** | | | | | | | | |
|---|---|---|---|---|---|---|---|---|
| Compound 1-001 | | | Compound 1-002 | | | Compound 1-003 | | |
| SAD Trial in HCM Patients | | | SAD Trial in Healthy Volunteers | | | MAD Trial in Healthy Volunteers | | |
| | | | | | | | | |
| Dose Cohort | Responders^{( 1)} | | Dose Cohort | Responders⁽²⁾ | | Dose Cohort | Responders⁽³⁾ | |
| | n | % | | n | % | | n | % |
| 48 mg | 2/4 | 50% | 12 mg | 3/6 | 50% | 12.5 mg | 3/10 | 30% |
| 96 mg | 5/6 | 83% | 24 mg | 5/6 | 83% | 18.5 mg | 10 / 10 | 100% |
| 144 mg | 4/5 | 80% | 48 mg | 6/6 | 100% | 25 mg⁽⁴⁾ | 10 / 10 | 100% |

| | | | | | | | | |
|---|---|---|---|---|---|---|---|---|
| (1) 48mg cohort (n=4 patients); 96mg cohort (n=6); 144mg cohort (n=5). (2) Each cohort randomized 6:2 Compound 1 or matching placebo. (3) Each cohort randomized 10:2 Compound 1 or matching placebo. (4) 25mg cohort treated for 25 days. | | | | | | | | |

**Table 5 below summarizes the three Phase 1 programs.**

| **Table 5.** Summary and Next Steps. | |
|---|---|
| Summary Phase 1 Program | • Three Phase 1 studies of Compound 1 in 108 healthy volunteers and 15 HCM patients |
| Safety | • Favorable safety profile demonstrated across a meaningful dose range, including multiple doses to 28 days |
| Pharmacokinetics (PK) | • PK profile well documented for single and multiple doses |
| | • Experience to inform PIONEER-HCM Phase 2 trial design |
| Proof of Mechanism | • Clinical proof of mechanism observed; dose-dependent reduction in cardiac contractility |
| | • Modest yet clinically relevant reduction in LVEF shows high responder rate across three Phase 1 trials |
| Next Steps | • Initiate Phase 2 trial in oHCM patients in H2 2016 |
| | • Endpoints to include obstruction relief and improvement in symptom/function |
| | • Additional guidance on late clinical development program at R&D Day in Fall 2016 |

### Example 3. A Phase 2 Open-label Pilot Study to Evaluate Efficacy, Pharmacokinetics, Pharmacodynamics, Safety, and Tolerability of Compound 1 in Subjects With Symptomatic Hypertrophic Cardiomyopathy and Left Ventricular Outflow Tract Obstruction (Pioneer-HCM-A)

### Study Objectives:

### Primary Objective:

To characterize the effect of 12 weeks of Compound 1 treatment on reducing post-exercise peak left ventricular outflow tract (LVOT) gradient in subjects with symptomatic hypertrophic cardiomyopathy (HCM) and LVOT obstruction

### Secondary Objectives:

To assess the proportion of subjects achieving an LVOT gradient response of post-exercise peak gradient < 30 mm Hg in subjects with symptomatic HCM and LVOT obstruction

To assess the effects of 12 weeks of Compound 1 treatment on dyspnea symptom score, peak oxygen consumption (pVO₂), and volume expired (VE)/carbon dioxide production (VCO₂) slope in subjects with symptomatic HCM and LVOT obstruction

To evaluate the pharmacokinetics (PK) of Compound 1 in subjects with symptomatic HCM and LVOT obstruction

To evaluate the pharmacodynamics (PD) of Compound 1 in subjects with symptomatic HCM and LVOT obstruction as assessed by a variety of echocardiographic imaging parameters in subjects with symptomatic HCM and LVOT obstruction

To evaluate the safety and tolerability of Compound 1 in subjects with symptomatic HCM and LVOT obstruction

To evaluate the post treatment reversibility of the effects of Compound 1 after 4 weeks of washout in subjects with symptomatic HCM and LVOT obstruction

### Exploratory Objectives:

To assess the proportion of subjects achieving an LVOT gradient response of post-exercise peak gradient < 10 mm Hg in subjects with symptomatic HCM and LVOT obstruction

To assess the effects of 12 weeks of Compound 1 treatment on New York Heart Association (NYHA) functional class, N-terminal pro B-type natriuretic peptide (NT-proBNP), levels and the Kansas City Cardiomyopathy Questionnaire (KCCQ) Overall Summary Score (OSS) in subjects with symptomatic HCM and LVOT obstruction

To assess the effects of Compound 1 treatment on arterial pulse wave morphology assessed using an optical biosensor in subjects with symptomatic HCM and LVOT obstruction

To assess the effect of Compound 1 treatment on heart rate and heart rhythm as assessed using a cardiac monitoring skin patch in subjects with symptomatic HCM and LVOT obstruction

### Inclusion Criteria:

Each subject must meet the following criteria to be included in this study.
1. Able to understand and comply with the study procedures, understand the risks involved in the study, and provide written informed consent according to federal, local, and institutional guidelines before the first study-specific procedure.
2. Men or women 18 to 70 years of age at the Screening visit.
3. Diagnosed with HCM (hypertrophied and non-dilated left ventricle in absence of systemic or other known cause), with left ventricular [LV] wall thickness ≥ 15 mm at time of initial diagnosis or ≥ 13 mm with a positive family history of HCM.
4. Body mass index (BMI) 18 to 37 kg/m², inclusive, at the Screening visit, calculated using the institution's standard formula.
5. All safety laboratory parameters (chemistry, hematology, and urinalysis) within normal limits (laboratory reference range) at the Screening visit as assessed by the central laboratory, or if outside of the limits must meet both of the following criteria:
   - considered by the investigator to be clinically unimportant
   - if a liver function test result, must be < 1.5 × the upper limit of the laboratory reference range.
6. Has documented left ventricular ejection fraction (LVEF) ≥ 55% at the Screening visit as determined by the investigator and the investigational site's echocardiography laboratory.
7. Resting LVOT gradient ≥ 30 mg Hg and post-exercise peak LVOT gradient ≥ 50 mm Hg at the Screening visit as determined by the investigator and the investigational site's echocardiography laboratory.
8. NYHA functional class II or higher, judged by the investigator to be due to LVOT obstruction.
9. Has adequate acoustic windows, in the judgment of the investigator and the investigational site's echocardiography laboratory, to enable accurate transthoracic echocardiograms (TTEs).
10. Female subjects must not be pregnant or lactating and, if sexually active, must be using one of the following acceptable birth control methods from the Screening visit through 3 months after the last dose of investigational medicinal product (IMP).
   - Double-barrier method (e.g., male using a condom and female using a diaphragm or cervical cap)
   - Barrier plus hormonal contraception (e.g., male using a condom, and female using hormonal contraception)
   - Female is surgically sterile for 6 months or postmenopausal for 2 years. Permanent sterilization includes hysterectomy, bilateral oophorectomy, bilateral salpingectomy, and/or documented bilateral tubal occlusion at least 6 months prior to Screening. Females are considered postmenopausal if they have had amenorrhea for at least 2 years or more following cessation of all exogenous hormonal treatments and follicle-stimulating hormone (FSH) levels are in the postmenopausal range.
11. Male subjects with female partners (including postmenopausal partners) must agree to use highly effective contraceptive measures from the Screening visit through 3 months after the last dose of study medication. Highly effective contraception includes documented vasectomy, abstaining from sexual intercourse, double-barrier method (e.g., male using a condom and female using a diaphragm or cervical cap), or barrier plus hormonal contraception (e.g., male using a condom and female using hormonal contraception). As there may be a risk of drug being secreted in the ejaculate, male subjects (including men who have had vasectomies) whose partners are currently pregnant should use barrier methods for the duration of the study and for 3 months after the last dose of study medication. In addition, male subjects with sexual partners should use condoms for the duration of the study and for 3 months after the last dose of study medication, even if the partner is not pregnant or capable of becoming pregnant, in order to prevent passing Compound 1 to the partner in the ejaculate.
12. Must be able to complete the Dyspnea Numeric Rating Scale (NRS) and the KCCQ per established guidelines.

### Exclusion Criteria:

Subjects who meet any of the following criteria will be excluded from the study.
1. Hypersensitivity to Compound 1 or any of the components of the Compound 1 formulation.
2. Presence of any medical condition that precludes exercise stress testing.
3. History of sustained ventricular tachyarrhythmia.
4. History of syncope with exercise within past 6 months.
5. Active infection.
6. Persistent atrial fibrillation or atrial fibrillation at Screening.
7. Has QTc Fridericia (QTcF) > 500 ms, or any other electrocardiogram (ECG) abnormality considered by the investigator to pose a risk to subject safety (e.g., second degree atrioventricular block type II).
8. Aortic stenosis or fixed subaortic obstruction.
9. History of LV systolic dysfunction (LVEF < 45%) at any time during their clinical course.
10. History of coronary artery disease.
11. History of malignancy of any type, with the following exceptions: in situ cervical cancer more than 5 years prior to Screening or surgically excised non-melanomatous skin cancers more than 2 years prior to Screening.
12. Positive serologic test at Screening for infection with human immunodeficiency virus (HIV), hepatitis C virus (HCV), or hepatitis B virus (HBV).
13. Positive test for alcohol or drugs of abuse at Screening.
14. History or evidence of any other clinically significant disorder, condition, or disease (with the exception of those outlined above) that, in the opinion of the investigator or MyoKardia physician, would pose a risk to subject safety or interfere with the study evaluation, procedures, or completion.
15. Participated in a clinical trial where the subject received any investigational drug (or is currently using an investigational device) within 30 days prior to Screening, or at least 5 times the respective elimination half-life (whichever is longer). Previous participation in a clinical trial with Compound 1 is acceptable as long as these criteria are met.
16. Current use of tobacco- or nicotine-containing products exceeding 10 cigarettes per day or equivalent.
17. Subjects who in the opinion of the investigator require ongoing therapy with β-blockers, calcium channel blockers, or disopyramide. Subjects on any of these medications who, in the opinion of the investigator, can safely be withdrawn are eligible as long as medication is discontinued at least 14 days prior to the Screening visit.
18. Prior treatment with cardiotoxic agents such as doxorubicin or similar, or current treatment with antiarrhythmic drugs that have negative inotropic activity, e.g., flecainide or propafenone.
19. Unable to comply with the study restrictions/requirements, including the number of required visits to the clinical site.
20. Is employed by, or is a relative of someone employed by MyoKardia, the investigator, or his/her staff or family.

### Overall Study Design and Plan

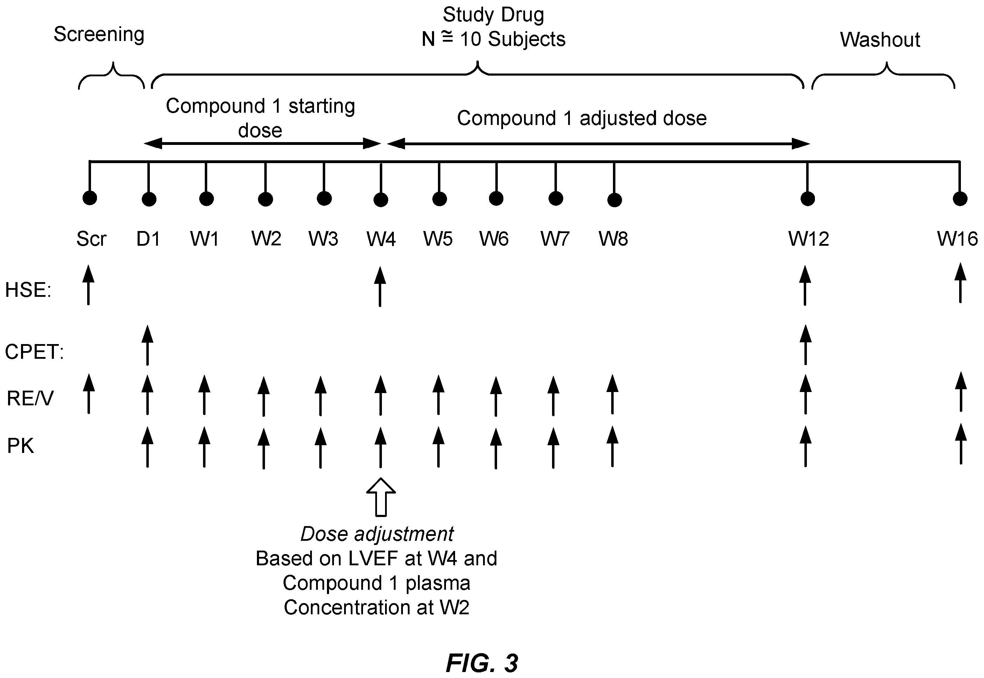

This is a phase 2 open-label pilot study to evaluate the efficacy, PK, PD, safety, and tolerability of Compound 1 in subjects with symptomatic HCM and LVOT obstruction. Approximately 10 adult subjects with symptomatic HCM, a resting LVOT gradient ≥ 30 mm Hg, and post-exercise peak LVOT gradient ≥ 50 mm Hg will be enrolled. The study will have a 12-week treatment phase and a 4-week washout phase (**FIG. 3**).

At Screening, subjects will undergo transthoracic echocardiography (TTE) in the supine or left lateral decubitus position. In addition to a standardized complete TTE, both resting instantaneous peak LVOT gradient (baseline) and provoked peak LVOT gradient will be collected. Subjects will then undergo a standard symptom-limited exercise test (hemodynamic stress echocardiography). Subjects will be assessed systematically for symptoms of dyspnea and/or angina. Instantaneous peak LVOT gradient will be assessed immediately post-exercise by TTE.

On Day 1, resting TTE will be performed, blood samples will be drawn for PK, and subjects will have an assessment of pVO₂, VE/VCO₂, and other variables via cardiopulmonary exercise testing (CPET). After CPET, subjects will be dosed with open-label oral Compound 1 at the investigational site and will be observed for at least 1 hour after dosing, with assessment of vital signs and ECGs at 1 hour postdose. Subjects will then be supplied with Compound 1 sufficient for QD dosing at home.

Subjects will be evaluated at the investigational site at Weeks 1, 2, 3, 4, 5, 6, 7, 8, 12, and 16. At each of these visits, resting TTE will be performed and blood samples will be drawn for PK. On study visit days, study medication will be administered at the investigational site in order to collect blood samples for PK about 2 hours prior to dosing. At Weeks 4, 12, and 16, hemodynamic stress echocardiography will be performed and peak LVOT gradient will be assessed. CPET will be conducted between Weeks 10 and 12 (inclusive).

The Compound 1 starting dose will be 10 mg for subjects who weigh about 60 kg and 15 mg for subjects who weigh > 60 kg. At the end of Week 4 (Day 28), each subject's LVEF will be evaluated, and the subject's dose may be increased, decreased, or remain unchanged based on the predetermined criteria in **Table 6,** in consultation between the investigator and the medical monitor. If Compound 1 plasma concentration exceeds 750 ng/mL at Week 2, no dose increase is allowed, regardless of LVEF data at Week 4; however, dose decrease per LVEF data is allowed in this circumstance. The subject will subsequently receive that dose from Week 5 through Week 12.

| **Table 6 Week 4 Dose Adjustment Criteria** | |
|---|---|
| **Percent Relative Decrease From Baseline in LVEF at the End of Week 4 (Day 28)** | **Action for Compound 1 Dose Week 5-Week 12^{a}** |
| < 10 | increase dose by 10 mg |
| ≥ 10 and < 15 | increase dose by 5 mg |
| ≥15 and < 20 | no change in dose |
| ≥ 20 | decrease dose by 5 mg |

| | |
|---|---|
| LVEF, left ventricular ejection fraction; QD, once daily. ^{a} No dose increase allowed if plasma concentration of Compound 1 > 750 ng/mL at Week 2. | |

For all subjects who provide consent, blood will be drawn on Day 1 for assessment of HCM genotype and potentially additional deoxyribonucleic acid (DNA) sequencing.

In addition, subjects may consent separately to collection of pharmacogenetic samples that will be stored for potential future analysis of genetic biomarkers of efficacy, safety-related, PD, or PK parameters as determined by future studies using clinically meaningful endpoints, through DNA sequencing or other genetic testing.

Blood samples will be collected from all subjects on Day 1 and at Week 12 and stored for exploratory circulating biomarker analysis, e.g., proteomic analysis related to disease activity, metabolic pathways, efficacy measures, or safety measures.

The expected study duration is approximately 8 months (4 months for recruitment and 4 months for study conduct). Each subject is expected to be in the study no more than 140 days: up to 28 days for screening and up to 112 days for study conduct.

### Compound 1, Administration, and Schedule

Study medication will consist of Compound 1 immediate release oral tablets, supplied in 4 strengths: 2 mg, 5 mg, 10 mg, and 20 mg. Compound 1 tablets are uncoated and plain and white in appearance. The 2 mg and 5 mg tablets are round-convex in shape and of different sizes, while the 10 mg and 20 mg tablets are oval shaped of different sizes.

Compound 1 tablets are manufactured according to current Good Manufacturing Practice (cGMP) regulations. They will be supplied in high-density polyethylene bottles with induction seals and child resistant caps at 30 count per bottle. All study medication will be labeled according to applicable local regulatory guidelines.

The Compound 1 tablets must be stored at controlled room temperature at 20°C to 25°C (68°F to 77°F) in the packaging supplied by MyoKardia. Study medication at the investigational site will be stored in a secure area with access limited to authorized study personnel.

IMP will be supplied to subjects at least every 4 weeks in 30-count high-density polyethylene bottles that are appropriately labelled, with written instructions for daily dosing. The subjects will be instructed to store the Compound 1 tablets/bottles in a cool dry place. Drug should be taken with approximately 8 ounces of water.

### Example 4. Phase 2 Results Evaluating Efficacy, Pharmacokinetics, Pharmacodynamics, Safety, and Tolerability of Compound 1 in Subjects With Symptomatic Hypertrophic Cardiomyopathy and Left Ventricular Outflow Tract Obstruction (Pioneer-HCM- A)

The following results were obtained generally following the procedures described in **Example 3.** Each subject in the following trials discontinued β-blocker, calcium-channel blocker, and/or disopyramide therapy at least 14 days prior to the screening visit.

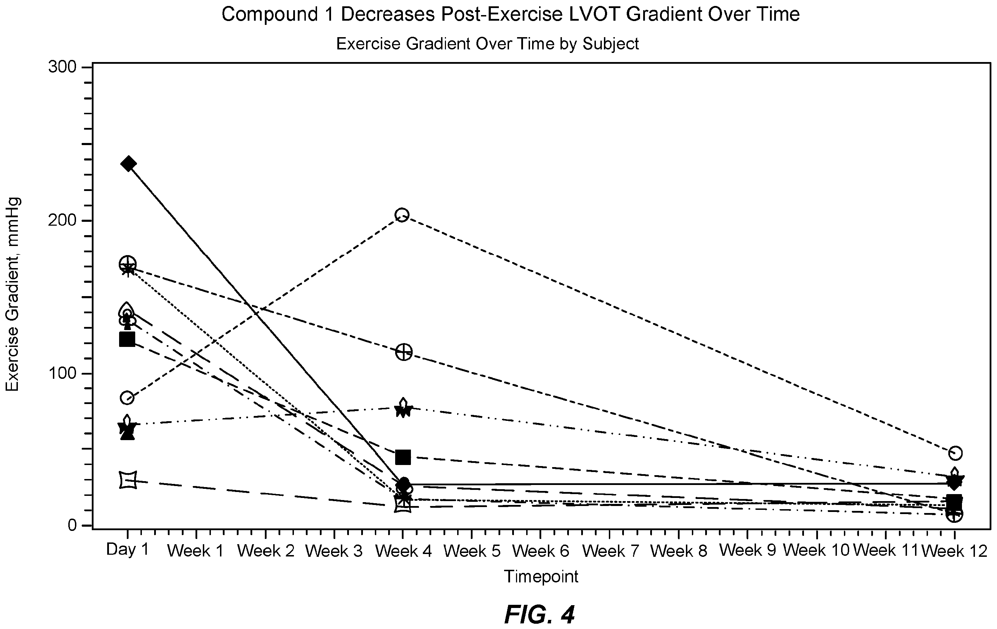

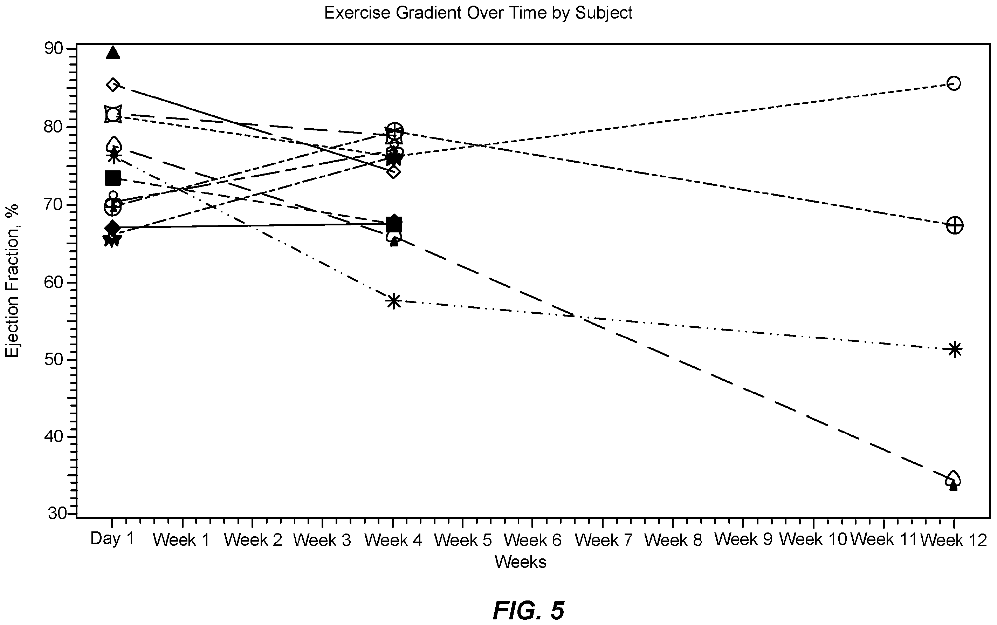

At weeks 0, 4, and 12 the stress LVOT gradient and stress ejection fraction percentage were measured in subjects receiving Compound 1 therapy (10 or 15 mg). In both patient populations, stress LVOT gradient was abolished with minimal change in LVEF. *See,* **FIG. 4** and **FIG. 5****.**

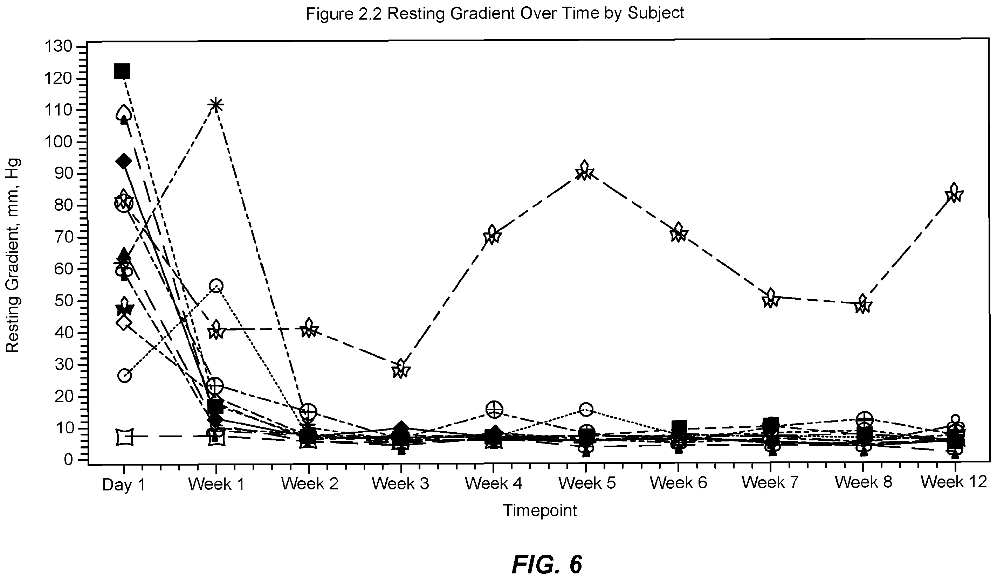

The resting LVOT gradient was also measured in subjects receiving Compound 1 (10, 15, or 20 mg). Resting LVOT gradient was abolished as early as week 1 in most subjects and by week 2 in all subjects at low exposures to Compound 1. *See,* **FIG. 6****.**

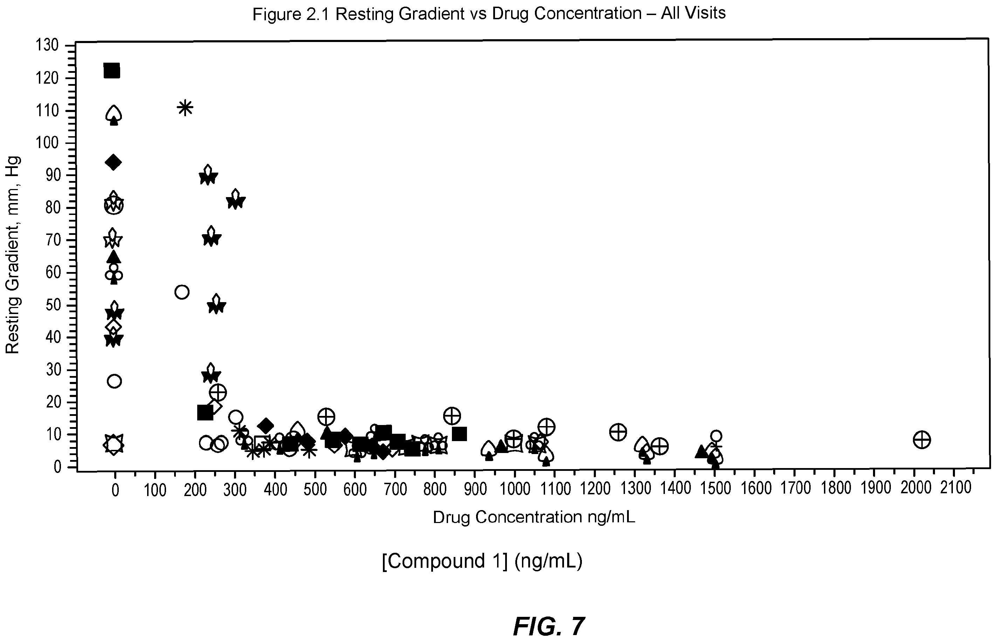

**FIG. 7** plots the resting LVOT gradient v. Compound 1 concentration.

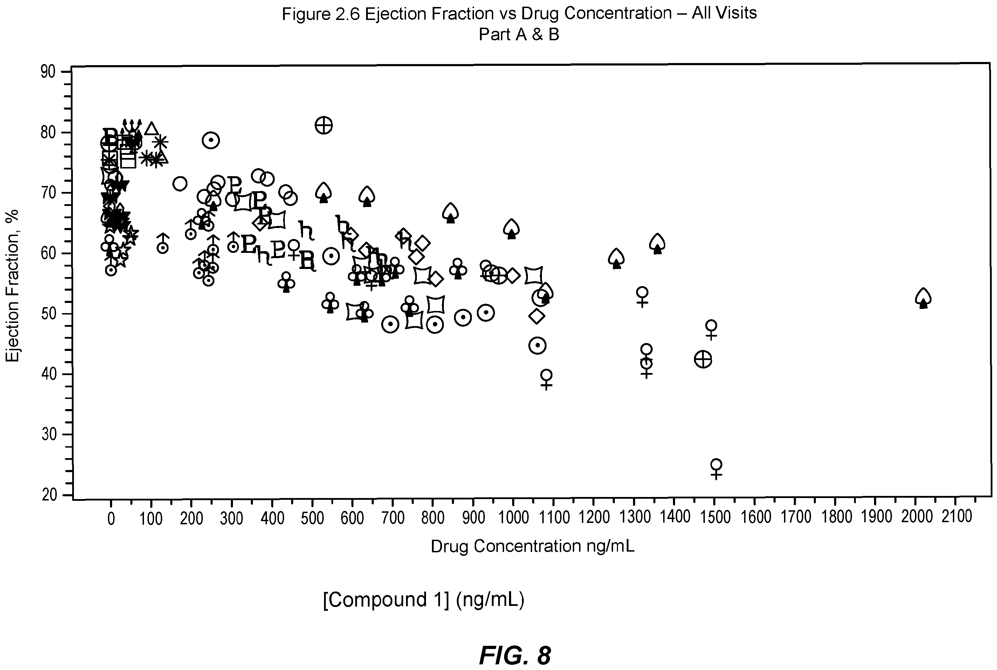

**FIG. 8** plots the resting left ventricle ejection fraction (LVEF) (%) v. Compound 1 concentration. The graph shows an exposure-dependent decrease in resting LVEF.

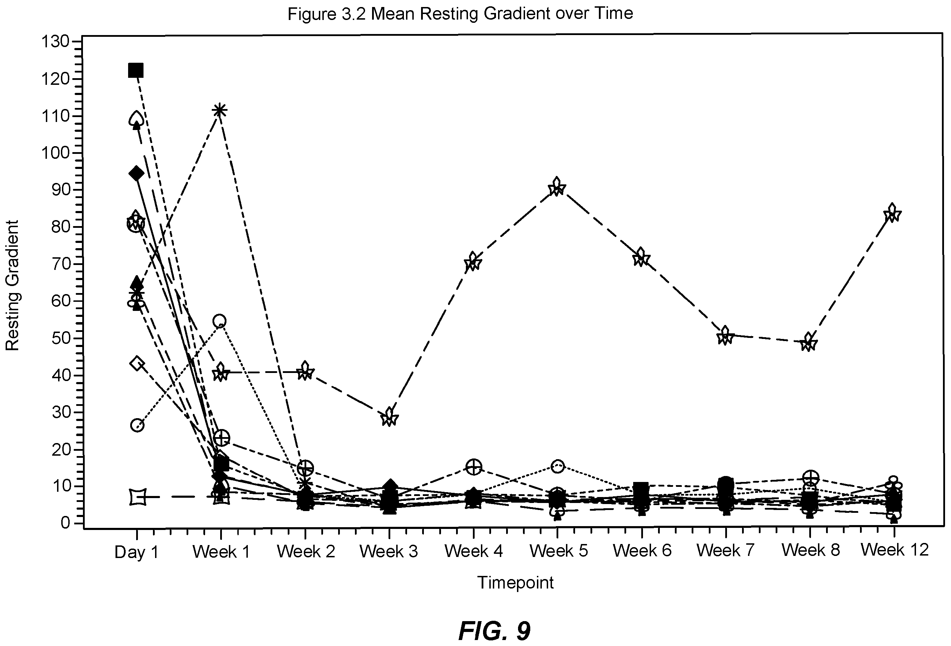

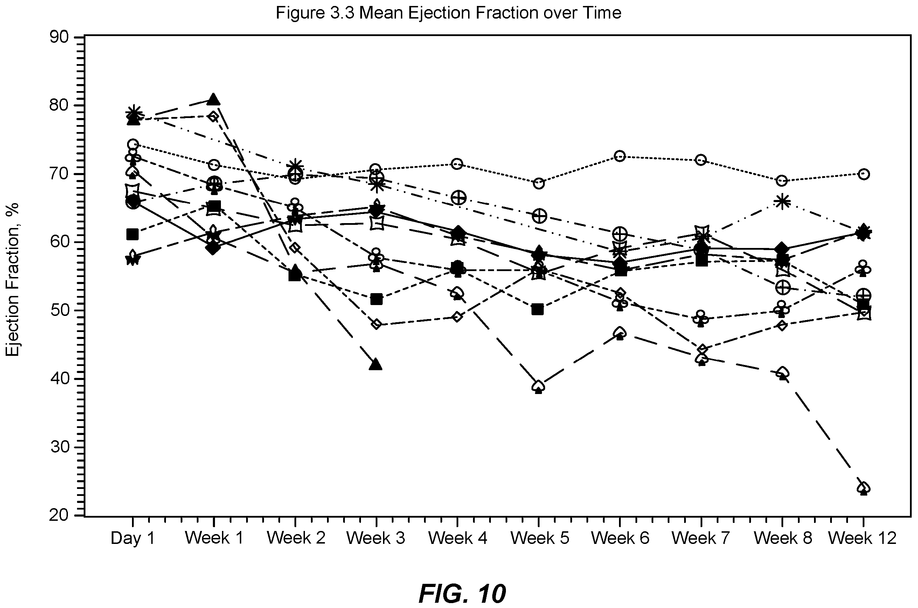

**FIG. 9** plots the average resting LVOT gradient measured each week of the study. **FIG. 10** plots the average resting left ventricle ejection fraction (LVEF) (%) measured each week of the study. Administration of Compound 1 is stopped after week 12. The obstructive HCM (oHCM) phenotype returns after cessation of Compound 1.

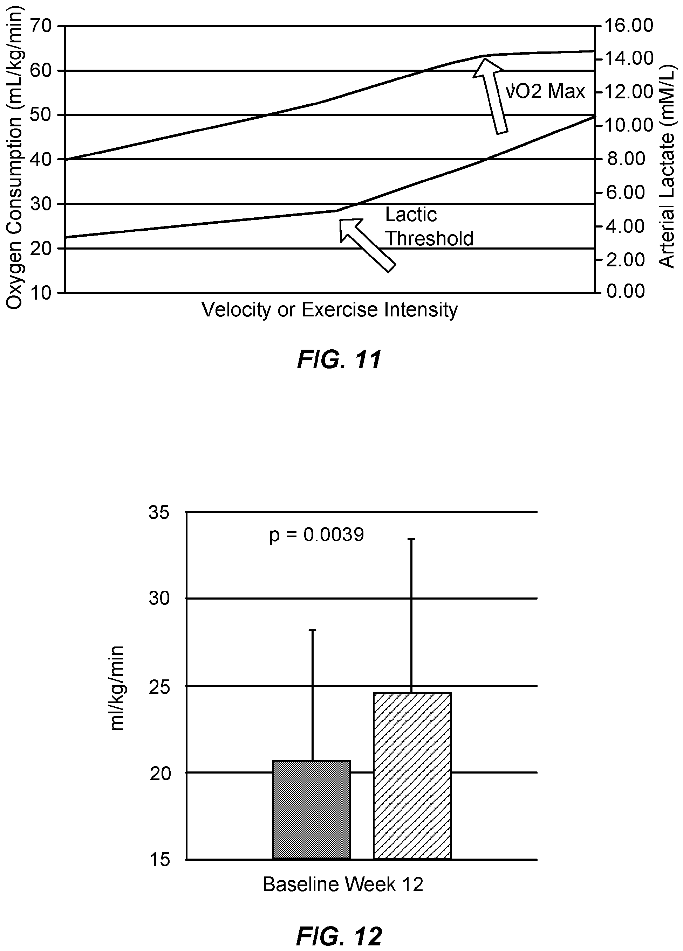

**FIG. 11** illustrates a type of plot that can be obtained when measuring oxygen consumption (mL/kg/min) and arterial lactate (mM/L) during Cardiopulmonary Exercise Testing.

**FIG. 12** shows peak VO₂ values measured during CPET at baseline (0 weeks, solid) and after week 12 (hashed bar). Compound 1 improves CPET metric.

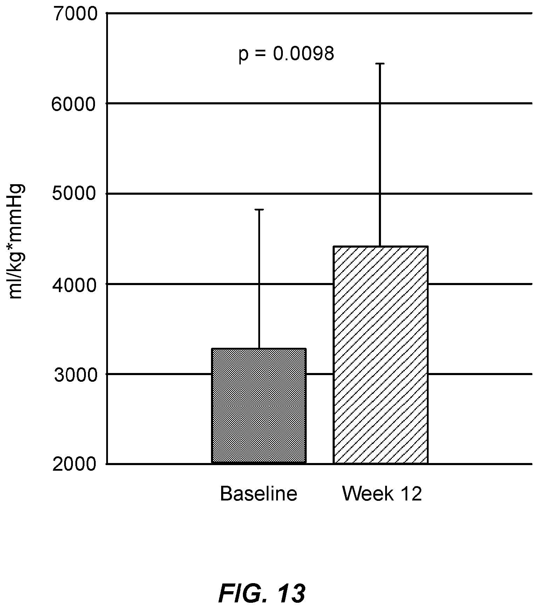

**FIG. 13** shows peak circulatory power measured during CPET at baseline (0 weeks, solid) and after week 12 (hashed bar). Compound 1 improves CPET metric.

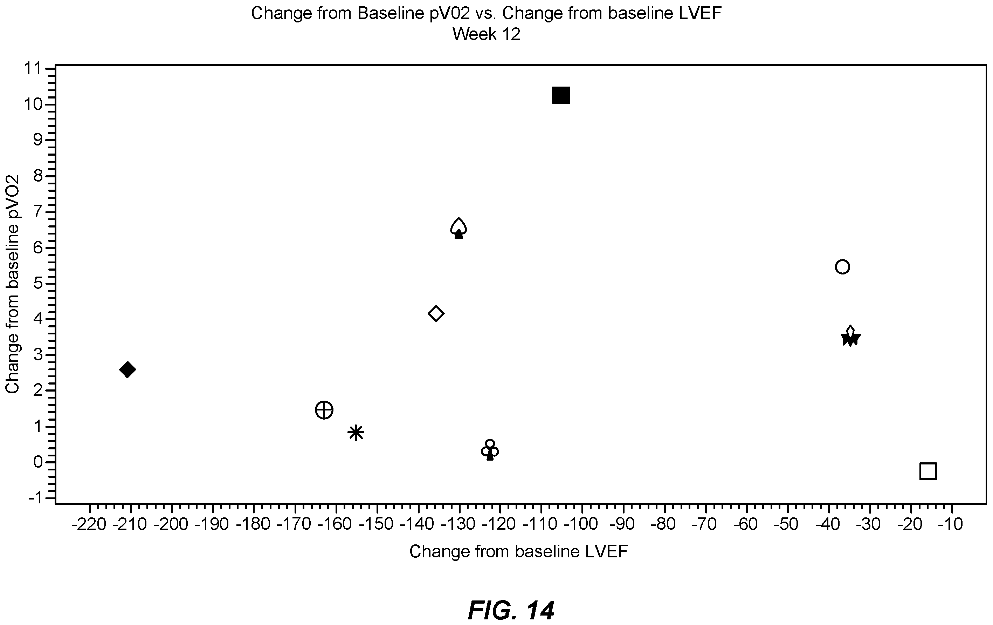

**FIG. 14** plots the absolute change in pVO₂ v. absolute change in left ventricle ejection fraction (LVEF). This plot shows that the increase in exercise capacity with Compound 1 therapy is greatest in patients with the least decrease in LVEF.

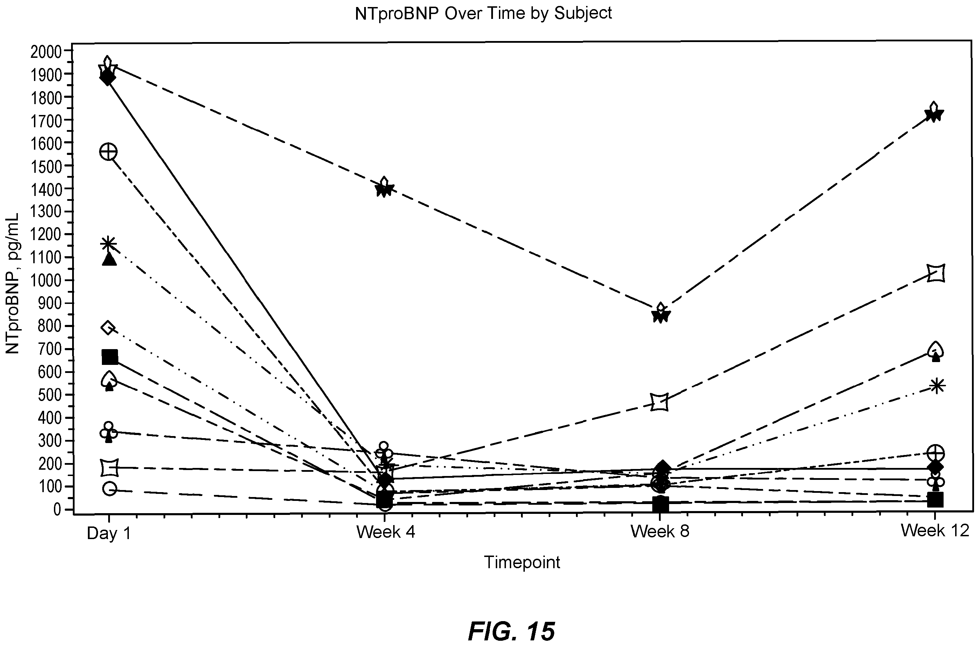

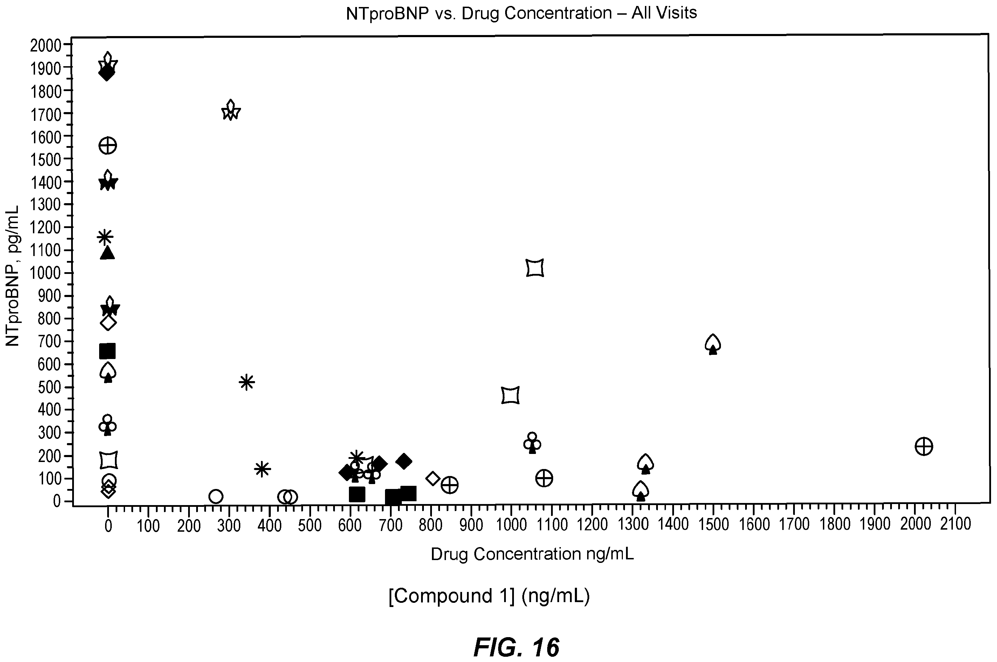

**FIG. 15** plots the N-terminal pro B-type natriuretic peptide (NT-proBNP) concentration (pg/mL) at weeks 0, 4, 8, and 12 in subjects receiving Compound 1. **FIG. 16** plots the N-terminal pro B-type natriuretic peptide (NT-proBNP) concentration (pg/mL) v. the concentration of Compound 1. These figures show that NT-proBNP levels are reduced after 4 weeks of Compound 1 but can rebound with higher exposures.

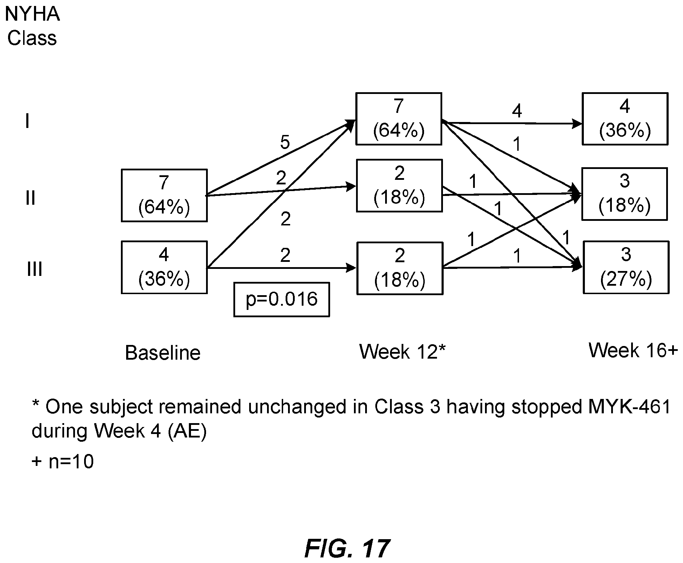

**FIG. 17** shows the New York Heart Association (NYHA) functional class of subjects at baseline (0 weeks), after 12 weeks of Compound 1 administration, and after week 16 (12 weeks of Compound 1 administration and 4 weeks of Compound 1 washout).

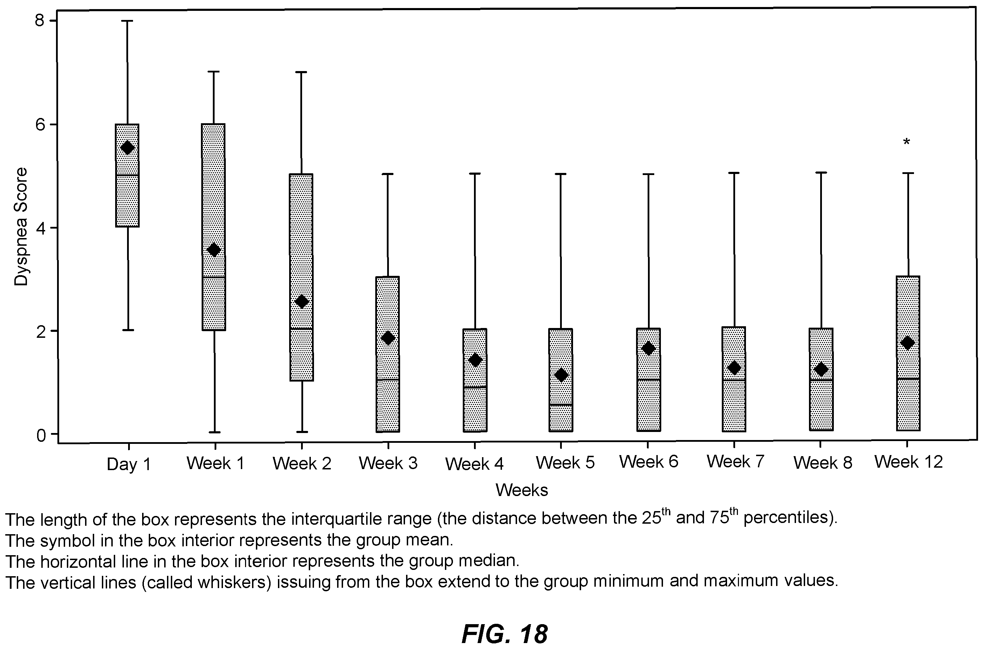

**FIG. 18** shows the mean dyspnea symptom score for each subject at the indicated times (Day 1 - Week 16). Compound 1 was administered through week 12. The week 16 data point is after 4 weeks of Compound 1 washout. Overall, the data show that dyspnea improves with Compound 1 administration, returning after cessation of therapy.

The phase 2 trial indicates that:
- Compound 1 is well tolerated at doses up to 20mg QD
- Compound 1 at a concentration of >225 ng/mL is associated with reduction of resting gradient below hemodynamic significant
- Two prognostic CPET metrics, peak VO₂ (pVO₂) and circulatory power (CP), improve in oHCM patients treated with Compound 1
- Most robust improvements in pVO₂ (> 3mL/min/kg) were associated with minimal change in resting LVEF
- Compound 1 plasma concentrations of 225-600 ng/mL provides a positive inotropic effect on myocardial contractility and gradient reduction
- NYHA Class and Dyspnea score improve with Compound 1 therapy.

### Example 5. A Phase 2 Open-label Pilot Study to Evaluate, Pharmacokinetics, Pharmacodynamics, Safety, and Tolerability of Compound 1 in Subjects With Symptomatic Hypertrophic Cardiomyopathy and Left Ventricular Outflow Tract Obstruction (Pioneer-HCM-B)

This trial was similar to the trial described in Example 3, but will have several important differences. For example, subjects in the Phase 2-B trial will not be required to discontinue their β-blocker therapy, and the dose of Compound 1 will be reduced to 2 to 5 mg. **Table 7,** below, describes some trial details as compared to the Phase 2-A trial described above.

| **Table 7** | Phase 2-A | Phase 2-B |
|---|---|---|
| 1⁰ Endpoint | Reduction in Gradient | Reduction in Gradient |
| Sample Size | ~10 | up to 10 |
| Dose (capsules) | 10 to 20 mg | 2 to 5 mg |
| Dose adjustment criteria | LVEF reduction and Week 2 PK | Gradient reduction and Week 2 PK |
| Key Inclusion | No β Blocker | Eliminate β Blocker washout |
| Criteria | Rest V ≥ 30, Ex V ≥ 50 mmHg | |
| | | Same level of gradient |
| # Trial Sites | 7 | same 7 |
| Treatment Duration | 12 weeks | 12 weeks |

### Example 6. Phase 2 Results Evaluating Efficacy, Pharmacokinetics, Pharmacodynamics, Safety, and Tolerability of Compound 1 in Subjects With Symptomatic Hypertrophic Cardiomyopathy and Left Ventricular Outflow Tract Obstruction (Pioneer-HCM-B)

The following results were obtained generally following the procedures described in **Example 3** and **Example 5.**

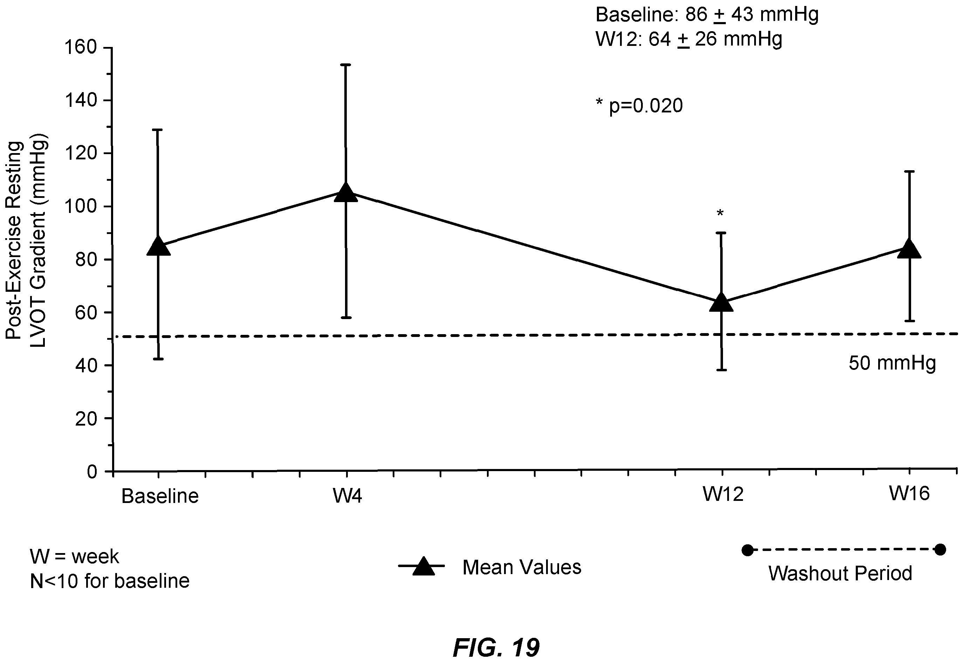

**FIG. 19** plots the mean post-exercise resting LVOT gradient (mm Hg) at weeks 4, 12, and 16 of the trial (12 weeks of administering Compound 1, 4 weeks of washout).

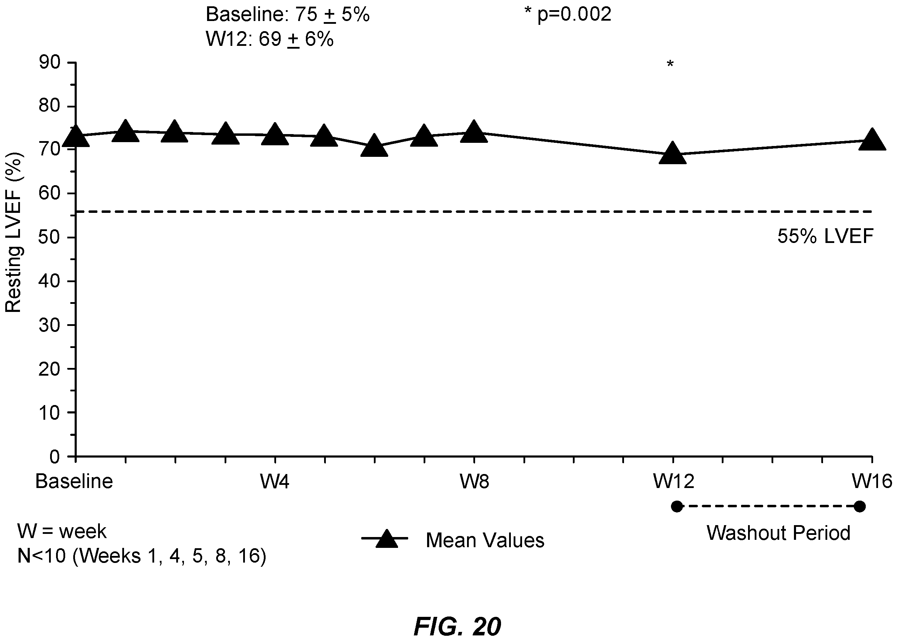

**FIG. 20** plots the change in resting LVEF (%) at weeks 1-8, 12, and 16 of the trial (12 weeks of administering Compound 1, 4 weeks of washout).

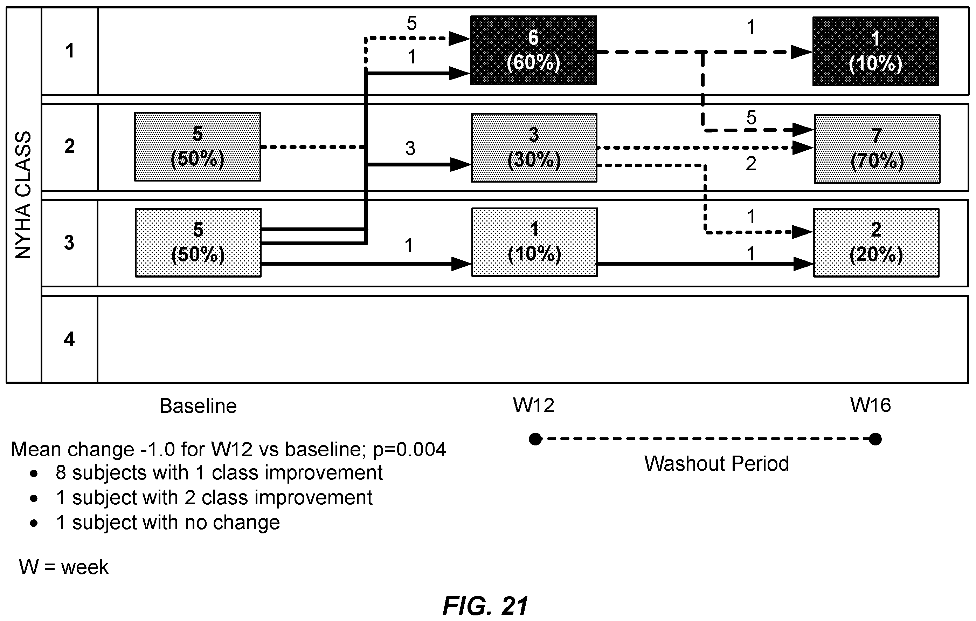

**FIG. 21** illustrates the improvement in NYHA functional class in individuals after 12 weeks and the reversal of improvement after 4 weeks of washout of Compound 1.

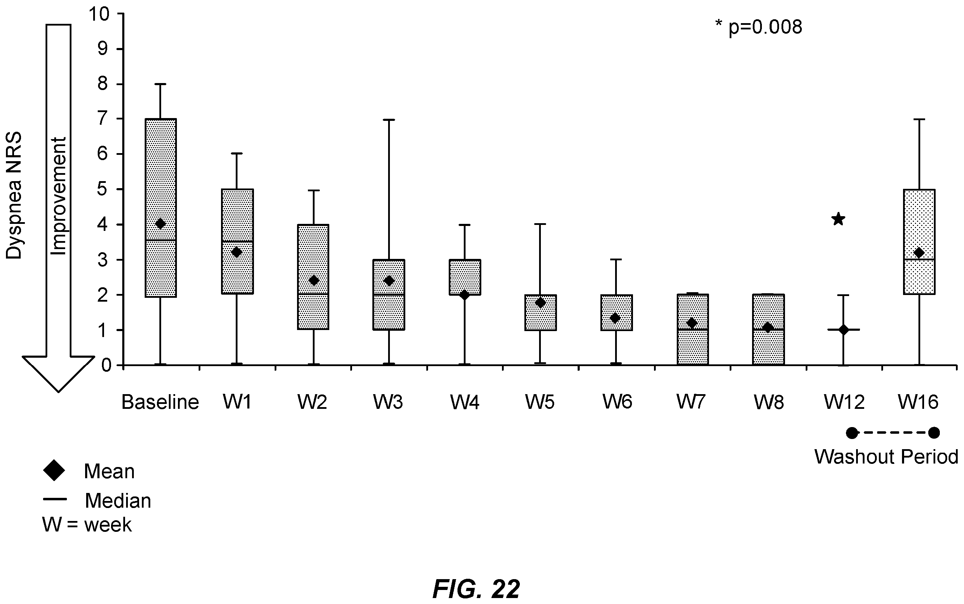

**FIG. 22** illustrates the rapid improvement in peak in dyspnea throughout the trial. It also shows that the improvement is reversed after 4 weeks of washout of Compound 1.

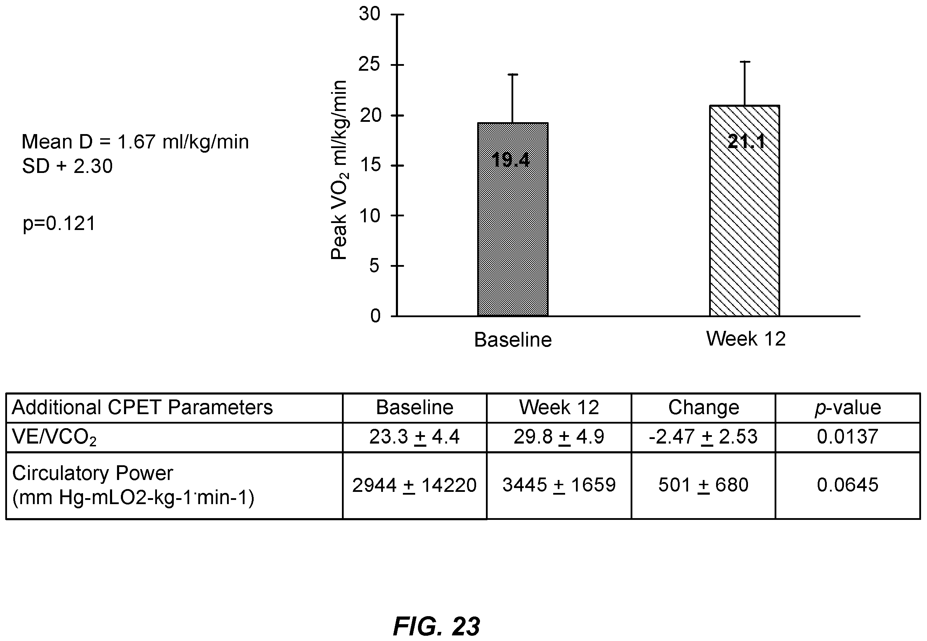

**FIG. 23** illustrates the change in peak VO₂ by week 12 during the trial.

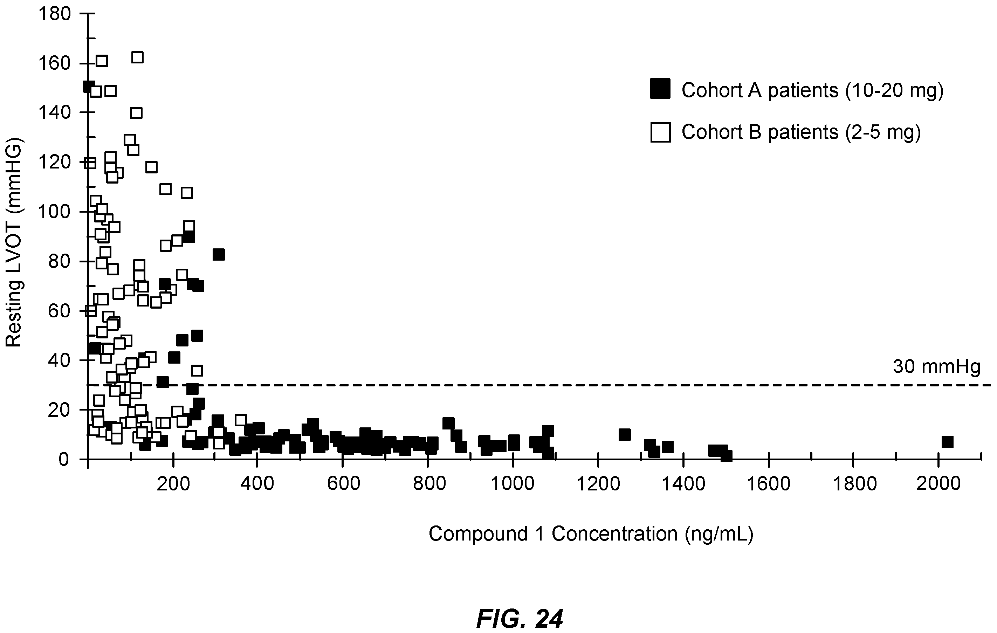

**FIG. 24** plots the resting LVOT (mmHg) as a function of Compound 1 Concentration. Cohort B are open squares and cohort A are filled squares.

The results of Cohort B (2-5 mg daily doses) are shown below in **Table** 8. Notably, 4 subjects had an improvement in peak VO₂ of ≥ 2.9 mL/kg/min

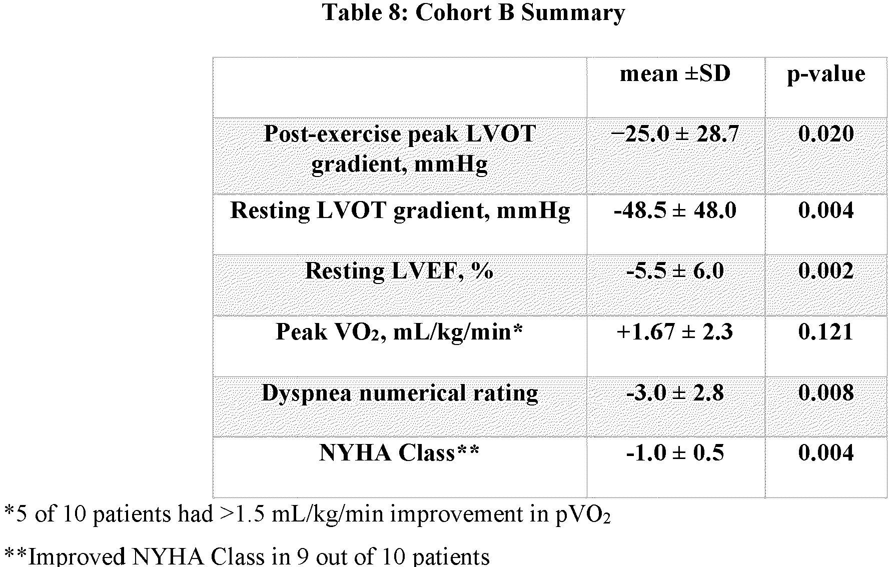

Of note, 5 of 10 patients had >1.5 mL/kg/min improvement in pVO₂, and 8 of 10 patients had improved NYHA class.

Parts A and B of the phase 2 trial indicates that:
- Compound 1 plasma concentrations of 350 to 700 ng/mL were associated with LVOT gradient reductions to generally below the 30 mm Hg level
- The gradient reduction was accompanied by improvement in symptoms, pVO₂ during exercise, a decrease in NT pro-BNP and improvement in echocardiographic measures of diastolic function
- Compound 1 plasma concentrations of 350 to 700 ng/mL resulted in clinical improvement in oHCM (symptom improvement and peak VO₂) while maintaining LVEF ≥ 55%
- The therapeutic concertation rage was well tolerate and was associated with limited decrease in LVEF, often representing a normalization of the hypercontractile state present at baseline
- Concentrations exceeding 1000 ng/mL were associated with excessive pharmacological effect

Parts A and B of the phase 2 trial also indicates diastolic improvement with Compound 1 (*see,* **Table 9** and **Table 10**)

**Table 9: Cohort A: e' and E/e' Baseline & Week 12**

| | **Baseline** | **Week 12** | **Delta** | **n** | **p-value** |
|---|---|---|---|---|---|
| **Lateral e' (cm/s)** | 6.9 ± 1.9 | 8.7 ± 1.6* | 1.6 ± 1.9 | 9 | p=0.027 |
| **E/e'** | 12.9 ± 3.3 | 9.1 ± 2.2** | -3.6 ± 3.7 | 8 | p=0.031 |
| **LVEDV** | 82 ± 24 | 97 ± 24 | 16 ± 21 | 11 | p = 0.027 |
| **LA Vol (index) (mL/m²)** | 30 ± 10 | 31 ± 8 | 1 ± 7 | 10 | p = 0.476 |
| **NT-proBNP** | 930 ± 647 | 454 ± 551 | -459 ± 722 | 10 | p = 0.084 |
| **Dyspnea score** | 4.9 ± 1.6 | 1.7 ± 1.8 | -3.1 ± 1.4 | 10 | p = 0.002 |

**Table 10: Cohort B: e' and E/e' Baseline & Week 12**

| | **Baseline** | **Week 12** | **Delta** | **n** | **p-value** |
|---|---|---|---|---|---|
| **Lateral e' (cm/s)** | 5.9 ± 1.0 | 6.1 ± 2.1 | 0.3 ± 2.2 | 10 | p=0.938 |
| **E/e'** | 13.9 ± 3.5 | 12.6 ± 4.1* | -1.3 ± 4.6 | 10 | p=0.652 |
| **LVEDV** | 95 ± 15 | 101 ± 25 | 6 ± 18 | 10 | 0.492 |
| **LA Vol (index) (mL/m²)** | 41 ± 20 | 35 ± 12 | -6 ± 12 | 10 | 0.322 |
| **NT-proBNP** | 1834 ± 3209 | 826 ± 1063 | -1195 ± 2770 | 9 | 0.074 |
| **Dyspnea score** | 4.0 ± 2.6 | 1.0 ± 0.7 | -3.0 ± 2.8 | 10 | 0.008 |

### Example 7. A Pivotal Trial to confirm safety and efficacy in oHCM patients (Explorer-HCM)

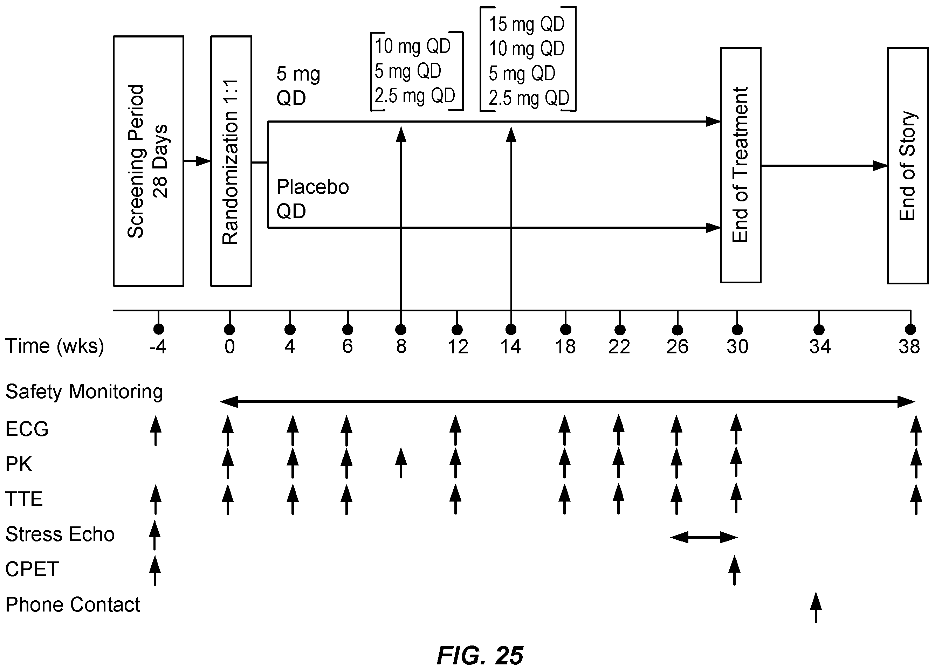

Trial will be for 24 week, and will include about 220 people. A key metric measured in this trial will be peak VO₂. **FIG. 25** shows the study design.

This is a Phase 3, double-blind, randomized, placebo-controlled, multicenter, international, parallel-group study to evaluate the safety, tolerability, and efficacy of mavacamten compared with placebo (1:1) in participants with symptomatic oHCM. Approximately 220 participants will be enrolled. This includes ~80 participants (~40 per treatment group) who consent to participate in a CMR substudy at selected sites. Randomization will be stratified according to NYHA functional classification (II or III), current treatment with β-blocker (yes or no), planned type of ergometer used during the study (treadmill or exercise bicycle), and consent for the CMR substudy (yes or no).

### The study will comprise 3 periods, summarized in FIG. 25.

**Screening period (Day -28 to Day -1):** Participants will undergo a variety of general, cardiopulmonary, laboratory, symptom, and PRO assessments over 1 to 2 days in order to assess eligibility criteria, including presence of HCM and presence of obstruction (at rest or provoked). The investigator should also confirm that participant can adequately perform a Valsalva maneuver. Key Screening tests include ECG; TTE conducted at rest, with Valsalva maneuver, and post-exercise; and CPET. Screening test results as reported by core laboratories (electrocardiogram core laboratory, echocardiography core laboratory, and CPET core laboratory) will be used to confirm eligibility for randomization. A minimum of 14 days will be needed to conduct Screening procedures.

CPET and post-exercise TTE may be conducted on the same day or separate days during the Screening period. If CPET and post-exercise TTE are conducted on the same day, participants must be exercised only once for both procedures, and participants will undergo CPET followed by post-exercise TTE. If the participants undergo CPET and post-exercise TTE on different days, then participants will first undergo resting, Valsalva, and post-exercise TTE (and send the echocardiogram to the core laboratory to determine LVOT gradients for each) and undergo CPET on a later day.

Participants who are receiving treatment for their oHCM condition should be on optimal medical therapy for their condition as determined by the investigator and informed by HCM treatment guidelines (eg, β-blocker, verapamil, or diltiazem). This treatment should be stable and well-tolerated for at least 2 weeks prior to Screening. Concomitant medications at Screening should be maintained at a stable dose throughout the study, unless safety or tolerability concerns arise.

A participant may also be considered for enrollment if the investigator has determined that receiving no treatment for their underlying oHCM condition is a valid option (eg, in case of prior intolerance or contra-indication to β-blockers). In such cases, there should be no plan to initiate treatment (with β-blocker, verapamil, or diltiazem) after randomization in the study.

### Double-blind treatment period (Day 1 [randomization] to Week 30/EOT):

Participants who meet all eligibility criteria will first be randomized via an interactive response system (IXRS) in a 1:1 ratio to receive treatment with mavacamten 5 mg starting dose or matching placebo QD.

Overall, the 30-week, double-blind treatment period includes a total of 10 scheduled clinic visits, allowing the maintenance of participant contact every 2 to 4 weeks. All clinic visits will include but are not limited to: clinical evaluation (symptoms, PRO assessments, AE/SAE assessments, concomitant medications). At all visits except the Week 8 and Week 14 visits, ECGs, TTEs, and laboratory assessments will also be conducted. Blood for PK (trough plasma concentrations) will be drawn at all visits except Week 14. Results of TTEs performed at each scheduled visit following randomization should be kept blinded to the investigator and study site personnel. An exception may occur if LVEF ≤30% is measured at the site, then the investigator will be notified and study drug will be permanently discontinued.

After randomization, participants will first be seen at Week 4 for an initial evaluation of clinical tolerability and safety. PK sample collection and blinded TTE will be performed at this visit. In the unlikely event that the results from Week 4 exceed PK/PD criteria (700 ng/mL < trough PK < 1000 ng/mL), the dose will be decreased at Week 6 to 2.5 mg via the IXRS.

Participants will subsequently be seen at Week 6 and Week 12 for repeat evaluation. Blinded assessments including trough PK, and TTE measures of LVEF and LVOT gradient with Valsalva will be performed to guide dose adjustment via the IXRS. At Week 8 and Week 14, the dose will be adjusted (dose increase, dose decrease, dose remain unchanged) based upon results of Week 6 and Week 12 assessments.

For added safety, a Week 8 blood sample will be drawn to determine trough plasma PK. If PK is greater than 700 and less than 1000 ng/mL, then an unscheduled visit will be arranged 2 weeks later to reduce dose. At any time if PK plasma concentration ≥ 1000 ng/mL, then study drug will be temporarily discontinued.

After Week 14, there are no additional scheduled dose titrations. Blinded assessments at Weeks 18, 22, and 26 can inform dose reduction or temporary discontinuation of study drug. Whenever a mavacamten dose is decreased, the participant will continue on the reduced dose to the EOT (Week 30) unless further safety concerns or intolerability arise.

At Week 30/EOT, participants will complete post-exercise TTE (between Week 26 and Week 30) and CPET (at Week 30). For any participants permanently discontinuing treatment prior to Week 30, an early termination (ET) visit should be conducted as soon as possible, including CPET and post-exercise TTE. Participants with ET will also be encouraged to complete all remaining study visits and assessments, including Week 30.

Posttreatment follow-up period (Week 30/EOT to Week 38/end of study [EOS]): After the end of the double-blind treatment period (Week 30), participants will be contacted by phone at Week 34 and return at Week 38 for an EOS visit. At the EOS visit, baseline resting assessments will be repeated. This posttreatment follow-up period applies only to participants who are receiving study drug after Week 22.

### Inclusion Criteria

Each participant must meet the following criteria to be enrolled in this study.
1. Able to understand and comply with the study procedures, understand the risks involved in the study, and provide written informed consent according to federal, local, and institutional guidelines before the first study-specific procedure
2. Is at least 18 years old at Screening
3. Body weight is greater than 45 kg at Screening
4. Has adequate acoustic windows to enable accurate TTEs
5. Diagnosed with oHCM consistent with current American College of Cardiology Foundation/American Heart Association and European Society of Cardiology guidelines, ie, satisfy both criteria below (criteria to be documented by the echocardiography core laboratory):
   a. Has unexplained LV hypertrophy with nondilated ventricular chambers in the absence of other cardiac (eg, hypertension, aortic stenosis) or systemic disease and with maximal LV wall thickness ≥15 mm (or ≥13 mm with positive family history of HCM), and
   b. Has LVOT peak gradient ≥50 mmHg during Screening as assessed by echocardiography at rest, after Valsalva maneuver, or post-exercise (confirmed by echocardiography core laboratory interpretation)
6. Has documented LVEF ≥55% by echocardiography core laboratory read of Screening TTE
7. Has NYHA Class II or III symptoms at Screening
8. Has documented oxygen saturation at rest ≥90% at Screening
9. Is able to perform an upright CPET and has a respiratory exchange ratio (RER) ≥1.0 at Screening per central reading; if the RER is between 0.91 and 1.0, the participant may be enrolled only if it is determined by the central CPET laboratory that peak exercise has been achieved in the subject (the only permitted reasons for subpeak performance are [1] a decrease in systolic blood pressure (SBP) or [2] severe angina as described in the CPET Laboratory Manual)
10. Female participants must not be pregnant or lactating and, if sexually active, must be using one of the following acceptable birth control methods from the Screening visit through 3 months after the last dose of investigational medicinal product (IMP). Hormonal contraceptives are not considered highly effective contraception for this study because mavacamten could reduce the effectiveness of hormonal contraceptives.
   - Double-barrier method (eg, vasectomy or male using a condom and female using a diaphragm or cervical cap)
   - Barrier (eg, male using a condom) plus female uses non-hormonal intrauterine device (IUD) or intrauterine system (IUS)
   - Female is surgically sterile for 6 months or postmenopausal for 2 years. Permanent sterilization includes hysterectomy, bilateral oophorectomy, bilateral salpingectomy, and/or documented bilateral tubal occlusion at least 6 months prior to Screening. Females are considered postmenopausal if they have had amenorrhea for at least 2 years or more following cessation of all exogenous hormonal treatments and follicle-stimulating hormone levels (FSH) are in the postmenopausal range
11. Male participants with sexual partners must agree to use condoms for the duration of the study and for 3 months after the last dose of IMP in order to prevent passing mavacamten to the partner in the ejaculate

### Exclusion Criteria

A participant who meets any of the following criteria will be excluded from the study.
1. Previously participated in a clinical study with mavacamten
2. Hypersensitivity to any of the components of the mavacamten formulation
3. Participated in a clinical trial in which the participant received any investigational drug (or is currently using an investigational device) within 30 days prior to Screening, or at least 5 times the respective elimination half-life (whichever is longer)
4. Known infiltrative or storage disorder causing cardiac hypertrophy that mimics oHCM, such as Fabry disease, amyloidosis, or Noonan syndrome with LV hypertrophy
5. Has any medical condition that precludes upright exercise stress testing
6. Has a history of syncope or sustained ventricular tachyarrhythmia with exercise within 6 months prior to Screening
7. Has a history of resuscitated sudden cardiac arrest (at any time) or known history of appropriate implantable cardioverter-defibrillator (ICD) discharge for life-threatening ventricular arrhythmia within 6 months prior to Screening
8. Has paroxysmal, intermittent atrial fibrillation with atrial fibrillation present per the investigator's evaluation of the participant's ECG at the time of Screening
9. Has persistent or permanent atrial fibrillation not on anticoagulation for at least 4 weeks prior to Screening and/or not adequately rate controlled within 6 months of Screening (Note - patients with persistent or permanent atrial fibrillation who are anticoagulated and adequately rate-controlled are allowed)
10. Current treatment (within 14 days prior to Screening) or planned treatment during the study with disopyramide or ranolazine
11. Current treatment (within 14 days prior to Screening) or planned treatment during the study with a combination of β-blockers and verapamil or a combination of β-blockers and diltiazem
12. For individuals on β-blockers, verapamil, or diltiazem, any dose adjustment of that medication <14 days prior to Screening or an anticipated change in treatment regimen using these medications during the study
13. Has LVOT gradient with Valsalva maneuver (Valsalva gradient) <30 mmHg at Screening TTE
14. Has been successfully treated with invasive septal reduction (surgical myectomy or percutaneous ASA) within 6 months prior to Screening or plans to have either of these treatments during the study (note: individuals with an unsuccessful myectomy or percutaneous ASA performed >6 months prior to Screening may be enrolled if study eligibility criteria for LVOT gradient criteria are met)
15. ICD placement within 6 months prior to Screening or planned ICD placement during the study
16. Has QT interval with Fridericia correction (QTcF) >480 ms or any other ECG abnormality considered by the investigator to pose a risk to participant safety (eg, second-degree atrioventricular block type II)
17. Has documented obstructive coronary artery disease (>70% stenosis in one or more epicardial coronary arteries) or history of myocardial infarction
18. Has known moderate or severe (as per investigator's judgment) aortic valve stenosis at Screening
19. Has any acute or serious comorbid condition (eg, major infection or hematologic, renal, metabolic, gastrointestinal, or endocrine dysfunction) that, in the judgment of the investigator, could lead to premature termination of study participation or interfere with the measurement or interpretation of the efficacy and safety assessments in the study
20. Has pulmonary disease that limits exercise capacity or systemic arterial oxygen saturation
21. History of clinically significant malignant disease within 10 years of Screening:
   - Participants who have been successfully treated for nonmetastatic cutaneous squamous cell or basal cell carcinoma or have been adequately treated for cervical carcinoma in situ can be included in the study
   - Participants with other malignancies who are cancer-free for more than 10 years before Screening can be included in the study
22. Has safety laboratory parameters (chemistry, hematology, coagulation, and urinalysis) outside normal limits (according to the central laboratory reference range) at Screening as assessed by the central laboratory; however, a participant with safety laboratory parameters outside normal limits may be included if he or she meets all of the following criteria:
   - The safety laboratory parameter outside normal limits is considered by the investigator to be clinically not significant
   - If there is an alanine aminotransferase or aspartate aminotransferase result, the value must be <3 × the upper limit of the laboratory reference range
   - The body size-adjusted estimated glomerular filtration rate is ≥30 mL/min/1.73 m2
23. Has a positive serologic test at Screening for infection with human immunodeficiency virus, hepatitis C virus, or hepatitis B virus
24. Has a history or evidence of any other clinically significant disorder, condition, or disease that, in the opinion of the investigator, would pose a risk to participant safety or interfere with the study evaluation, procedures, or completion
25. Is currently taking, or has taken within 14 days prior to Screening, a prohibited medication, such as a CYP 2C19 inhibitor (eg, omeprazole), a strong CYP 3A4 inhibitor, or St. John's Wort
26. Prior treatment with cardiotoxic agents such as doxorubicin or similar
27. Unable to comply with the study requirements, including the number of required visits to the clinical site
28. Is a first degree relative of personnel directly affiliated with the study at the clinical study site, any study vendor, or the study Sponsor

### Mavacamten and Matching Placebo, Administration, and Schedule

Mavacamten capsules and matching placebo have the same appearance. The study drug presentation is size 2, blue opaque capsules printed with a yellow band on the body and black band on the cap. Each capsule contains white to off-white powder. The active capsules are supplied in 4 strengths: 2.5 mg, 5 mg, 10 mg, and 15 mg.

Mavacamten active and placebo capsules have been manufactured according to current Good Manufacturing Practice (cGMP) regulations. They will be supplied in high-density polyethylene bottles with induction seals and child-resistant caps at 30 count per bottle. All study drug will be labeled according to applicable local regulatory guidelines.

The mavacamten active and placebo capsules must be stored at 2°C to 25°C (36°F to 77°F) in the packaging supplied by MyoKardia. Study medication at the investigational site will be stored in a secure area with access limited to authorized study personnel.

Study drug will be supplied to participants in 30-count high-density polyethylene bottles that are appropriately labeled. Participants will take study drug as directed by the study investigator. The participants will be instructed to store the study drug capsules and bottles in a cool, dry place. Study drug should be taken with approximately 8 ounces (48 tablespoons or ~235 mL) of water.

### Dose Adjustments

### Dose Titration Period (Day 1 to Week 14)

The double-blind treatment period will include a two-step dose titration scheme at Week 8 and Week 14 designed to achieve safe and effective dosing for each participant based on their own PK/PD response parameters **(****FIG. 25** **and Table 11).**

In this study, the starting dose of study drug is mavacamten 5 mg or matching placebo QD, and each participant will receive this dose of study drug from Day 1 through Week 8 unless a PK/PD criterion is met at Week 4, in which case the dose will be reduced at Week 6 **(Table 11).**

At Weeks 8 and 14, participants will undergo dose adjustment (dose increase, dose decrease, or dose unchanged) based on their results from PK/PD assessments at Week 6 and Week 12, respectively **(Table 11).** Note that **Table 11** is provided for IXRS programming. Sites and investigators will not be actively adjusting doses. All dose adjustments will occur in a double-blind manner via IXRS, and all participants, whether receiving mavacamten or matching placebo, will undergo assessments that could lead to a blinded dose adjustment. However, note that participants who are on placebo will remain on placebo (in blinded fashion) unless the participant has a temporary discontinuation or a permanent treatment discontinuation.

For added safety, a Week 8 blood sample will be drawn to determine trough plasma PK. If PK is greater than 700 and less than 1000 ng/mL, then an unscheduled visit will be arranged 2 weeks later to reduce dose (see **Table 11**). Dose reduction will occur via IXRS. To avoid potential bias the IXRS will randomly select a participant from the placebo arm to undergo an unscheduled follow-up visit.

If the mavacamten dose is decreased at any time during the study, then the participant will continue on the reduced dose to the EOT (Week 30) unless safety concerns or intolerability arise requiring further dose reduction or dose discontinuation.

Based on PK modeling informed by prior mavacamten clinical studies, and depending on the demographics of enrolled patients, it is estimated that the following percentages of participants will reach the target steady-state concentrations for the following doses: 2.5 mg (< 1%), 5 mg (5-20%), 10 mg (40-50%), and 15 mg (30-55%).

If the PK/PD criteria for down-titration are met, then dose reduction will be implemented as follows:

**Table 11: Blinded Dose Adjustment Schedule (Day 1 to Week 14) PK/PD Criteria for Down-titration (requires LVEF ≥50%)^{a}**

| **Time of Assessment** | **Trough PK (ng/mL)^{a}** | **Time and Dose Reduction^{b}** |
|---|---|---|
| Week 4 | 700 < PK < 1000 | Week 6: Reduce to 2.5 mg |
| Week 6 | 700 < PK < 1000 | Week 8: Reduce to next lower dose |
| | | • 5 mg to 2.5 mg |
| | | • 2.5 mg to placebo |
| Week 8^{c} | 700 < PK < 1000 | 2 weeks later^{c}: Reduce to next lower dose |
| | | • 10 mg to 5 mg |
| | | • 5 mg to 2.5 mg |
| | | • 2.5 mg to placebo |
| Week 12 | 700 < PK < 1000 | Week 14: Reduce to next lower dose |
| | | • 10 mg to 5 mg |
| | | • 5 mg to 2.5 mg |
| | | • 2.5 mg to placebo |

| | | |
|---|---|---|
| Abbreviations: IXRS, interactive response system; LVEF, left ventricular ejection fraction; PD, pharmacodynamics; PK, pharmacokinetics. ^{a}L VEF and trough PK will be communicated directly to the IXRS from the core laboratories based on assessments so that it is blinded to the investigator, study site personnel, and the Sponsor. ^{b}Dose reduction applies if PK criterion is met. ^{c}Week 8 assessment for dose reduction will be based solely on plasma PK value, there will be no TTE performed at Week 8, and therefore, no L VEF result. | | |

**At Week 6 and Week 12: If PK/PD criteria for down-titration are not met, then potential dose up-titration may proceed as follows:**

### Dose Titration (requires LVEF ≥50%)

| **Time of Assessment: Week 6 or Week 12** | **Dose Titration^{a}** | **Week 8 Time and Dose** | **Week 14 Time and Dose** |
|---|---|---|---|
| PK <350 ng/mL AND | Increase | 5 mg to 10 mg | increase from prior dose: |
| Valsalva gradient ≥ 30 mmHg | | | |
| | | | • 5 mg to 10 mg |
| | | | • 10 mg to 15 mg |
| PK <350 ng/mL AND | No change | Continue prior dose: 2.5 mg or 5 mg | Continue prior dose: 2.5 mg, 5 mg, or 10 mg |
| Valsalva gradient < 30 mmHg | | | |
| 350 ≤ PK ≤ 700 ng/mL (regardless of Valsalva gradient) | No change | Continue prior dose: 2.5 mg or 5 mg | Continue prior dose: 2.5 mg, 5 mg, or 10 mg |

| | | | |
|---|---|---|---|
| Abbreviations: IXRS, interactive response system; LVEF, left ventricular ejection fraction; PD, pharmacodynamics; PK, pharmacokinetics. ^{a}Titration adjustments will also be communicated directly to the IXRS based on Week 6 and Week 12 including measures of peak Valsalva gradient reported by the core laboratory so that blinding is maintained. If LVEF <50%, see temporary discontinuation section. | | | |

### Dosing Period (Week 14 to Week 30)

After the second dose titration at Week 14, there are no further up-titrations; the intent is for dose to remain unchanged unless for safety or other reasons for premature discontinuation. After Week 14, participants will return to the clinical site for monitoring at scheduled 4 week intervals (Weeks 18, 22, 26, and 30 [±7 days]). At each visit, AEs, concomitant medications, and symptoms will be assessed, and ECG, plasma PK, and TTE will be performed for ongoing safety monitoring. Compliance with study drug will also be monitored by capsule count at each visit. If PK/PD criteria are met, then an unscheduled visit 2 weeks later will be required to reduce dose as shown in **Table 12.**

**Table 12 Blinded Dose Adjustment Schedule (Week 14 to Week 30/EOT) PK/PD Criteria for Dose Reduction (requires LVEF ≥50%)a**

| **Time of Assessments** | **Trough PK (ng/mL)^{a}** | **Time and Action^{b}** |
|---|---|---|
| Weeks 18, 22, 26 | 700 < PK < 1000 | 2 weeks later: Reduce to next lower dose |
| | | • 15 mg to 10 mg |
| | | • 10 mg to 5 mg |
| | | • 5 mg to 2.5 mg |
| | | • 2.5 mg to placebo |

| | | |
|---|---|---|
| Abbreviations: EOT, end of treatment; IXRS, interactive response system; LVEF, left ventricular ejection fraction; PD, pharmacodynamics; PK, pharmacokinetics. ^{a}LVEF and trough PK will be communicated directly to the IXRS from the core laboratories based on assessments, so that they are blinded to the investigator, study site personnel, and the Sponsor. If LVEF <50%, see temporary discontinuation section. ^{b}Dose reduction applies if PK criterion is met. | | |

### Blinded Dose Adjustment Leading to Temporary Discontinuation

In addition to the blinded dose adjustments described above (which are not preceded by dose discontinuation), at any time (T) during the treatment period, dosing may be temporarily discontinued in the case of exaggerated pharmacologic effect (systolic dysfunction), higher than expected plasma concentration, or excessive QTcF prolongation as described below.

If participant has a resting LVEF <50%, plasma drug concentration ≥1000 ng/mL, or QTcF ≥500 msec (as determined by the echocardiography central laboratory, PK analysis laboratory, or ECG core laboratory, respectively), it will be communicated to the investigator and Sponsor that a criterion for temporary discontinuation has been met. Upon receipt of this information, the study site/investigator will contact the participant by telephone and instruct the participant to discontinue study drug and to return for an onsite visit within 2 to 4 weeks (T+2 to 4 weeks). This could correspond to a scheduled or unscheduled visit. Note that to avoid potential bias, for each participant who has an unscheduled follow-up visit because of a safety signal, the IXRS will randomly select a participant from the placebo arm to undergo an unscheduled follow-up visit.

At the follow-up visit (T+2 to 4 weeks), ECG, plasma PK, and TTE will be repeated and another unscheduled visit will be planned for 2 weeks later (T+4 to 6 weeks). If LVEF ≥50% AND plasma drug concentration <1000 ng/mL AND QTcF <500 msec, then the study drug will be restarted at a lower dose (at T+6 weeks) for remainder of the study as follows (previous dose → restart dose):
- Placebo → placebo
- 2.5 mg → placebo
- 5 mg → 2.5 mg
- 10 mg → 5 mg
- 15 mg → 10 mg

If LVEF, plasma drug concentration and/or QTcF persist out of range at the follow-up visit, then study drug will be switched permanently to placebo.

### STUDY ASSESSMENTS

For assessments that require the participants to be in a semi-recumbent or supine position, assessments should be conducted with participants in the same position at all time points. When several assessments are to be conducted at the same time point, the preferred order of assessments is ECG, vital signs, PK, and then TTE all prior to study drug dosing. The order of assessments may vary slightly at specific time points (eg, additional 1 hour postdose PK sampling on Day 1 and Week 30/EOT) to facilitate the most contemporaneous performance of the required assessments. Unscheduled or additional safety assessments may be performed if necessary in the opinion of the investigator. Whenever possible discussion with the medical monitor is encouraged.

### Echocardiography and Cardiopulmonary Exercise Testing

All echocardiography data will be sent to a central imaging laboratory and CPET data will be sent to a central laboratory; prespecified echocardiography results from multiple visits and CPET results from Screening visit only will be transmitted to the IXRS to confirm eligibility and to maintain blinding.

### Resting Transthoracic Echocardiography

Resting TTE will be assessed prior to dosing during onsite visits. Instantaneous peak LVOT gradient at rest and provoked peak LVOT gradient (Valsalva maneuver) will be assessed. The investigator should confirm during Screening that participant can adequately perform the Valsalva maneuver. Care should be taken to select the best window and angle when obtaining Doppler signal to assess the LVOT gradient and to avoid contamination by mitral regurgitation (MR) jet if present. Left ventricular ejection fraction (2-dimensional LVEF) and left ventricular fractional shortening will also be analyzed along with a variety of other echocardiographic measures (see Study Reference Manual). All TTE results performed at each scheduled visit following randomization should be kept blinded to the investigator and local study team, except the unblinded sonographer. At Screening and EOT, resting TTE should be performed prior to post-exercise stress echocardiography and/or CPET.

### Post-exercise Stress Echocardiography

Participants will undergo a standard symptom-limited exercise test (post-exercise stress echocardiography) at Screening and between Weeks 26 and 30 prior to study drug dosing. This test may be performed on a different day than CPET; however, if both procedures are performed on the same day, participants must exercise only once for both tests, with the participant undergoing first CPET and then post-exercise stress echocardiography). Instantaneous peak LVOT gradient will be assessed immediately post-exercise by TTE. Care should be taken to select the best window and angle when obtaining Doppler signal to assess the LVOT gradient and to avoid contamination by MR jet if present. Participants on standard cardiomyopathy therapy (eg, β-blockers or calcium channel blocker) should be on the same dose at baseline and at EOT stress echocardiogram whenever possible and this medication should be administered prior to exercise testing.

### Cardiopulmonary Exercise Testing

CPET will be conducted using a standardized treadmill or bicycle ergometer at Screening and at Week 30 prior to study drug dosing. The same modality (treadmill or upright bicycle) must be used for all CPETs conducted for a given participant. Supine testing (ie, using a recumbent bicycle) is not allowed for CPET. Symptom-limited exercise tests will be performed after a 4-hour fast (water is allowed). Participants will be encouraged to perform maximally to achieve their expected peak exercise heart rate (HR) and exertion level. Cardiovascular and multiple other performance parameters will be assessed at rest and at peak exercise including, but not limited to the following: oxygen consumption (VO₂), percent age-predicted VO₂, carbon dioxide production (VCO₂), expired ventilation (VE), VE/VO₂ ratio, ventilatory efficiency (VE/VCO₂ slope), RER, circulatory power (peak VO₂/peak SBP), metabolic equivalents achieved (METS), O₂ pulse at peak exercise, and end tidal CO₂. Participants on standard cardiomyopathy therapy (eg, β-blocker or calcium channel blocker) should be on the same dose at Screening and EOT CPET whenever possible and this medication should be administered prior to exercise testing.

### New York Heart Association Class

The NYHA Functional Classification of heart failure assigns participants to 1 of 4 categories based on the participant's symptoms. Heart failure classification will be assessed by the investigator at every study visit and recorded as indicated in the clinical database.

### New York Heart Association Functional Classification of Heart

### Failure

### Class Patient Symptoms

I No limitation of physical activity. Ordinary physical activity does not cause undue fatigue, palpitation, dyspnea (shortness of breath).
II Slight limitation of physical activity. Comfortable at rest. Ordinary physical activity results in fatigue, palpitation, dyspnea (shortness of breath).
III Marked limitation of physical activity. Comfortable at rest. Less-than ordinary-activity causes fatigue, palpitation, or dyspnea.
IV Unable to carry on any physical activity without discomfort. Symptoms of heart failure at rest. If any physical activity is undertaken, discomfort increases.

Source:
**http://www.heart.org/HEARTORG/Conditions/HeartFailure/AboutHeartFailure/Cl asses-of-Heart-Failure_UCM_306328_Article.jsp#.VrtuzPkrKUl**

### Hypertrophic Cardiomyopathy Symptom Questionnaire

The HCM Symptom Questionnaire (HCMSQ) is a self-administered 11-item questionnaire that assesses the core symptoms of HCM (tiredness/fatigue, heart palpitations, chest pain, dizziness, and shortness of breath). Participants will complete the HCMSQ once per day, in the evening. The HCMSQ will be completed on a handheld electronic device. Training for using the electronic device will be given to participants at the Screening visit. During the Screening period, participants will complete the HCMSQ for a minimum of 7 consecutive days. From Day 1 through Week 6, participants will complete the HCMSQ daily. For the remainder of the treatment period (through Week 30/EOT), participants will complete the HCMSQ for 7 consecutive days every 4 weeks; participants will also complete the HCMSQ for 7 consecutive days at Week 38/EOS. Reminder alarms on the electronic device and clinic reminders from the site staff will be used to ensure completion of the HCMSQ on schedule.

### Exploratory Assessments

### Patient Global Impression of Severity and Patient Global Impression of Change

The Patient Global Impression of Severity (PGIS) is a single item questionnaire that asks participants to rate their overall symptom severity in the past week on a 5-point categorical rating scale (none, mild, moderate, severe, very severe). The Patient Global Impression of Change (PGIC) is a single item questionnaire that asks participants to rate their overall change in symptom severity over time (since started taking the study medication) on a 7-point categorical rating scale (very much better to very much worse). The PGIC and other PRO assessments completed at visits should be completed prior to any other study procedure taking place, where possible, and prior to any meaningful discussion about the study or study treatment with investigative site staff.

### Work Productivity and Activity Impairment Questionnaire

The Work Productivity and Activity Impairment (WPAI-SHP) questionnaire was created as a patient-reported quantitative assessment of ability to work and perform regular activities. Specifically, the WPAI-SHP asks about the impact of a specific health problem (in this case HCM) on absenteeism, presenteeism, and daily activity impairment. The WPAI-SHP and other PRO assessments completed at visits should be completed prior to any other study procedure taking place, where possible, and prior to any meaningful discussion about the study or study treatment with investigative site staff.

### EuroQol Five Dimensions Questionnaire (5-level)

The EuroQol five dimensions 5-level questionnaire (EQ-5D-5L) is a generic PRO instrument that measures health status and health-related quality of life. There are 2 components to the EQ-5D-5L: (1) a health utility index score derived from 5 items addressing mobility, self-care, usual activities, pain/discomfort, and anxiety/depression "today") and (2) a current ("right now") general health status score derived from a single visual analog scale (VAS) ranging from 0 to 100. The items contributing to the EQ-5D-5L health utility index score each have the same 5-point response scale (1 = no problem, 2 = slight problems, 3 = moderate problems, 4 = severe problems, 5 = very severe problems). The VAS is anchored with "best imaginable health state" and "worst imaginable health state." The EQ-5D-5L is critical for acceptance by many health technology assessment bodies for coverage decisions. The EQ-5D-5L and other PRO assessments completed at visits should be completed prior to any other study procedure taking place, where possible, and prior to any meaningful discussion about the study or study treatment with investigative site staff.

### Kansas City Cardiomyopathy Questionnaire (23-item Version)

The Kansas City Cardiomyopathy Questionnaire (23-item version) (KCCQ-23) is a patient-reported questionnaire that measures the impact of patients' cardiovascular disease or its treatment on 6 distinct domains using a 2-week recall: symptoms/signs, physical limitations, quality of life, social limitations, self-efficacy, and symptom stability. In addition to the individual domains, 2 summary scores can be calculated from the KCCQ-23: the overall summary score (includes the total symptom, physical limitation, social limitations and quality of life scores) and the clinical summary score (combines the total symptom and physical limitation scales). Scores range from 0 to 100, with higher scores reflecting better health status. Prior to any other study procedure taking place, where possible, and prior to any meaningful discussion about the study or study treatment with investigative site staff.

### Wrist-worn Accelerometer

Participants will be provided with an accelerometer (GT9X ActiGraph Link) to be worn on the wrist at 2 time points during the study to collect data on activity. The device has sufficient battery life to store activity data for up to 11 days. Following a period of data collection, the accelerometer will be returned for data upload and analysis. The analysis will describe daily step counts. These data will be used to explore the amount and type of physical activity performed before and during treatment with study drug. Accelerometers will be provided at Screening (at least 11 days before Day 1) and at Week 26.

### Pharmacokinetic, Pharmacogenetic, and Biomarker Assessments

### Pharmacokinetic Assessments

Blood samples will be collected for mavacamten plasma concentration assessments at all post-Day 1 onsite visits prior to dosing. At Week 30 (last dose), a PK blood sample will also be collected within 1 to 2 hours after dosing for PK modeling. Unscheduled or additional PK samples may be collected if appropriate in the opinion of the investigator and/or Sponsor.

At all visits from Day 1 through Week 30, study drug will be administered at the investigational site to facilitate collection of PK samples. The date and time of dosing will be documented. All trough PK samples will be sent to a core laboratory for processing and results will be transmitted to the IXRS.

### Genotype/Pharmacogenetic/Biomarker Assessment

### HCM Genotyping

For participants who provide consent, blood will be drawn prior to dosing on Day 1 for assessment of HCM genotype and potentially additional DNA sequencing. If a participant with a prior clinical genotype test that was positive for genetic mutation associated with HCM consents to provide the results, no further genotype assessment will be performed.

### Pharmacogenetic Assessments

Blood will be drawn at Screening for CYP 2C19 genotyping. Residual material from this pharmacogenetic sample will also be stored for potential future analysis of genetic biomarkers of efficacy, safety-related, PD, or PK parameters as determined by future studies using clinically meaningful endpoints, through DNA sequencing or other genetic testing. Samples from screen failures must be destroyed.

### Exploratory Biomarker Assessments

Blood samples will be collected on Day 1 and at Week 30 and stored for relevant exploratory markers of disease including but not limited to proteomic and/or RNA analysis related to disease activity, metabolic pathways, efficacy measures, or safety measures.

### NT-proBNP

Blood samples will be collected for NT-proBNP concentrations at all post-Screening onsite visits. All blood draws for NT-proBNP must be drawn at rest and prior to any exercising. Serum concentrations of NT-proBNP will be included in the data package provided for the periodic IDMC meetings. Unscheduled or additional blood samples may be collected if appropriate in the opinion of the investigator (e.g., for medical management of heart failure) and/or Sponsor. Whenever possible, discussion with the medical monitor is encouraged

### Safety Assessments

Safety will be assessed throughout the study. Safety assessments include medical history, physical examinations, ECGs, vital signs, observed and participant-reported AEs, pregnancy testing, and safety laboratory results. Any abnormal findings judged by the investigator to be clinically important will be recorded as an AE.

The following safety endpoints will be adjudicated by the CEAC: Death, stroke, acute myocardial infarction, all hospitalizations (CV and non-CV), heart failure (HF) events (includes HF hospitalizations and urgent emergency room (ER)/outpatient (OP) visits for HF), atrial fibrillation/flutter (new from screening), ICD therapy, resuscitated cardiac arrest, ventricular tachyarrhythmias (includes ventricular tachycardia (VT) and ventricular fibrillation (VF); also any Torsades de Pointe identified during CEAC review).

### Medical History

A complete medical history will be recorded at the Screening visit, which will include evaluation (past or present) of the following: general, head and neck, eyes, ears, nose, throat, chest/respiratory, heart/cardiovascular, gastrointestinal/liver, gynecological/urogenital, musculoskeletal/extremities, skin, neurological/psychiatric, endocrine/metabolic, hematologic/lymphatic, allergies/drug sensitivities, past surgeries, substance abuse, or any other diseases or disorders as well as participation in clinical studies (study medication and/or device or other therapy).

### Physical Examination

At selected visits, a complete physical examination will be conducted including a neurological examination (gross motor and deep tendon reflexes), height (Screening only) and weight, and assessment of the following: general appearance, skin, head and neck, mouth, lymph nodes, thyroid, abdomen, musculoskeletal, cardiovascular, and respiratory systems. At all other visits, an abbreviated cardiopulmonary physical examination will be conducted, with other systems assessed as directed by interval history.

Height (cm) and body weight (kg) will be measured at Screening, and body mass index (kg/m2) will be calculated. Participants will be required to remove their shoes and wear clothing as specified by the clinical site.

Body weight will be captured in clinic at Screening and Week 30.

### 12-lead ECG

12-lead ECG evaluations will be performed after 10 minutes of rest at Screening and at all onsite study visits except for the Week 8 and 14 visits. All ECG data will be sent to a central cardiac laboratory and transmitted to IXRS.

On Day 1, ECG will be performed within 2 hours predose. At all other visits, ECGs will be taken prior to dosing.

The investigator may perform 12-lead ECG safety assessments if he/she considers it is required for any other safety reason. These assessments should be recorded as an unscheduled assessment

### Cardiac Monitoring Skin Patch

At 2 time points during the study, participants will wear a small device to collect continuous HR and rhythm data for up to 14 days. The self-contained device attaches to the skin using medical adhesive and contains surface electrodes, internal electronics to capture a continuous ECG waveform, an accelerometer to capture physical activity, sufficient solid state memory to store data over multiple days, and a battery to power the device. Following a period of data collection, the device will be transported to a core laboratory where the continuous ECG waveforms and activity record stored on the device will be uploaded for analysis. The analysis will provide full disclosure capabilities for HR, heart rhythm, and physical activity over the period during which the device was properly applied and functioning. The device will be used to explore the pattern of HR and heart rhythm before and during treatment with study drug.

### Vital Signs

Vital signs are to be assessed at each onsite study visit except the Week 8 and 14 visits. At Screening, ET, Week 30/EOT, and Week 38, complete vital signs including temperature, HR, respiratory rate, and blood pressure (BP) will be obtained. At all other visits, only HR and BP are required.

Vital signs will be obtained with the participant in the same position; BP will be taken after resting for at least 5 minutes via an automated recorder.

At all visits, vital signs will be taken prior to dosing. Alert values will be flagged. Refer to the Study Laboratory Manual for additional details.

### Other Safety Assessments

Serum pregnancy testing will be performed at Screening for all females of childbearing potential. In addition, urine pregnancy testing either in clinic or at home will be conducted every 4-6 weeks throughout the study. Confirmatory serum testing will be performed if any urine test is positive.

### Cardiac Magnetic Resonance Imaging Substudy

For qualified participating sites, participants will have the option to participate in the CMR substudy. Approximately 80 participants will be enrolled (~40 per treatment group). Participants will undergo CMR at Day 1 and Week 30. Refer to the CMR Substudy Reference Manual for additional details.

### Participant Restrictions During this Study

The following restrictions apply for the specified times during the study period. If a participant does not comply with these restrictions or tests positive in any laboratory tests (eg, drug, alcohol, pregnancy), he or she may be excluded or withdrawn from the study.
- Starting 72 hours prior to the first dose until the final follow-up visit,
   participants should not engage in unaccustomed intensive exercise except during protocol-specified exercise tests
- Starting at Screening, participants will be required to abstain from blood or plasma donation until 3 months after the final study visit
- Starting on Day 1 until the final follow-up visit, participants will be asked to abstain from grapefruit or grapefruit juice, Seville oranges, and quinine (eg, tonic water).

### Example 8. Lusitropic properties of Compound 1

Compound 1 stabilizes the off-actin state of myosin, thereby reducing the number of myosin heads available to participate in contraction. This reduction causes a direct reduction in systolic function, and may improve diastolic compliance by requiring fewer heads to detach during diastole. This additional work with Compound 1 and related compounds indicates that a clinician might independently select agents that optimize systolic and diastolic effects through the selection of different agents and doses of these agents to provide greater lusitropic benefit for less negative inotropic cost. The ability to optimize these effects allows the use of Compound 1 and related compounds to treat diseases of diastolic dysfunction (including RCM) where perturbation of systolic function is to be minimal.

Pre-clinically, Compound 1 reduces contractility with a profile distinct from other negative inotropes. Acute or chronic Compound 1 administration decreased myocardial contractility and increased LV end-diastolic dimensions while preserving systemic arterial blood pressure and, LV end-diastolic pressure, suggesting improved distensibility. In contrast with the acute effects of other negative inotropes, Compound 1 preserves end-diastolic pressures as well as the estimated (linear) end-diastolic stiffness despite marked increases in preload (EDV), consistent with a salutary down-/rightward shift of the pressure-volume relationships.

Emerging data from the PIONEER study in obstructive HCM **(Table 13:** Cohort A; **Table 14:** Cohort B) further suggest that Compound 1 is improving compliance of the left ventricle in the context of impaired diastolic function, manifest by an increase in LVEDV accompanies by a reduction in in LV filling pressure (as assessed by E/e').

**Table 13. Effect of Compound 1 on echocardiographic measures of LVOT gradient and LV systolic and diastolic function - Cohort A (10-15 mg/day) N=10 (LVEF N=4); mean ± SD.**

| | Baseline | Week 12 | P value |
|---|---|---|---|
| Resting Gradient (mm Hg) | 68 ± 34 | 14 ± 25 | 0.0059 |
| Post-exercise Gradient (mm Hg) | 125 ± 60 | 19 ± 13 | 0.0020 |
| Resting LVEF (%) | 70 ± 6 | 54 ± 12 | 0.0039 |
| Post-exercise LVEF (%) | 76 ± 5 | 60 ± 22 | 0.6250 |
| Stroke Volume (mL) | Pending | Pending | |
| NT-pro BNP (pg/mL) | 939 ± 647 | 235 ± 417 | 0.0020 |
| E/e' | 12.9 ± 3.3 | 9.1 ± 2.1 | 0.0156 |
| Lateral e' (cm/s) | 6.9 ± 1.9 | 8.8 ± 1.6 | 0.0137 |
| Septal e' (cm/s) | 5.4 ± 1.6 | 6.4 ± 1.5 | 0.1875 |
| Mean e' (cm/s) | 5.7 ± 1.2 | 7.4 ± 1.6 | 0.0781 |

**Table 14. Preliminary effects of Compound 1 on echocardiographic measures of LVOT gradient and LV systolic and diastolic function - Cohort B (2 mg/day); mean ± SD (N) number of patients is 6-8 here, but 10 in Table 10.**

| | Baseline | Week 4 | P value* |
|---|---|---|---|
| Resting Gradient (mm Hg) | 84 ± 76 (6) | 59 ± 61 (5) | 0.0625 |
| Post-exercise Gradient (mm Hg) | 70 ± 45 (6) | 93 ± 56 (6) | 0.0313 |
| Resting LVEF (%) | 71 ± 4 (6) | 73 ± 6 (6) | 0.4375 |
| Post-exercise LVEF (%) | 78 ± 9 (5) | 76 ± 3 (6) | 0.6250 |
| Stroke Volume (mL) | 73.3 ± 6.5 (6) | 77.8 ± 14.3 (6) | 0.6875 |
| NT-pro BNP (pg/mL) | 2162 ± 3601 (8) | 1258 ± 1789 (7) | 0.0781 |
| E/e' | 14.0 ± 3.4 (6) | 10.7 ± 3.5 (6) | 0.0625 |
| Lateral e' (cm/s) | 5.4 ± 1.0 (6) | 7.0 ± 1.2 (6) | 0.0313 |
| Septal e' (cm/s) | 3.9 ± 1.1 (6) | 4.5 ± 1.4 (6) | 0.4063 |
| Mean e' (cm/s) | 4.7 ± 1.0 (6) | 5.7 ± 1.3 (6) | 0.0938 |

Compound 1 (15 mg/day) eliminates LVOT gradient in oHCM patients and decreases resting LV systolic function. This is accompanied by an improvement in LV filling pressure (reduced E/e') and diastolic wall stress (reduced NTpro-BNP) and increase in diastolic peak mitral annular velocity accompanied by an increase in LVEDV inconsistent with improved LV diastolic function. The effect of a decrease in LV afterload on mitral annular velocity cannot be excluded.

Preliminarily, Compound 1 (2 mg/day for 4 weeks) has a consistent effect on LVOT rest and exercise gradients in patients thus far analyzed. At that dosing level, LV systolic function at rest and immediately post-exercise is not affected by Compound 1. However, beneficial effects on LV filling pressure (↓E/e' and NTpro-BNP) and in diastolic peak mitral annular velocity (e') in the absence of a change in afterload are observed. This is consistent with a beneficial effect on diastolic function.

Top potential indications for a positive lusitropic agent would include (in increasing order of complexity):
- Symptomatic non-obstructive HCM
- Inherited restrictive non-infiltrative restrictive cardiomyopathies (e.g. Troponin I, T; Desmin related)
- Symptomatic disorders of active relaxation (present in approximately 75% of HFpEF), excluding infiltrative disease (amyloid) & fibrosis (scleroderma, radiation therapy induced cardiomyopathy, idiopathic fibrosis)
- Symptomatic post aortic valve replacement (surgical or transcatheter).

Potential indications with the highest probability of technical success will be those where there is demonstrable evidence of an active relaxation disorder. The most homogeneous population is likely to be the symptomatic non-obstructed HCM patient group. For the broader HFpEF population, eligible patients would be selected following the ESC 2016 guidelines, which stipulate normal (≥ 50%) ejection fraction accompanied by direct evidence of abnormal diastolic function: impaired LV relaxation, filling, diastolic distensibility, or stiffness as measured by echocardiography (mitral E/A ratio > 0.8; E/e'≥ 14; septal e' < 7 cm/sec; lateral e' < 10 cm/sec) or cardiac magnetic resonance (abnormal peak filling rates, time to peak filling, peak diastolic strain rates) coupled with elevated levels of BNP (> 35 pg/mL) or NT-pro BNP (>125 pg/mL)..."

### Example 9. Determination of Compound 1 Blood Plasma Concentration

Compound 1 concentrations were measured in human plasma using high pressure liquid chromatography (HPLC) coupled with tandem mass spectrometry (MS/MS). This method was used for Compound 1 bioanalysis in all clinical trials to date, and all clinical data has been collected using plasma. A brief description of the method follows.

Liquid-liquid extraction and HPLC/MS/MS was used to determine the concentration of Compound 1 in human plasma. An aliquot of the extract was injected onto an HPLC instrument that is coupled with a triple quadrupole mass spectrometer (Sciex API5500). A Lux 3u Cellulose-4 HPLC column (150 x 2.0 mm) from Supelco, was used to separate Compound 1, from interfering compounds that may be present in the sample extract. The internal standard was a multi-deuterated analog of Compound 1 to ensure that the internal standard used in the assay had similar chemical properties to the analyte but a different mass for selective detection. The peak area of the product ion of Compound 1 was measured against the peak area of the product ion of the internal standard for determination of concentration.

## Claims

1. A compound, having the formula: 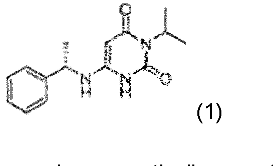 or a pharmaceutically acceptable salt thereof, for use in a method of treating hypertrophic cardiomyopathy (HCM) in a subject in need thereof, the method comprising administering a therapeutically effective amount of Compound 1 to said subject, wherein said therapeutically effective amount is a total daily dosage of 2 mg to 15 mg +/- 10%.

2. The compound for use of claim 1, wherein the subject has not received a β-blocker therapy for a period of at least two weeks prior to initiating treatment with Compound 1.

3. The compound for use of claim 1, wherein:
said subject has at least a 30 mmHg pressure gradient across the subject's left ventricular outflow tract (LVOT), prior to initiating treatment; or
the therapeutically effective amount is sufficient to lower a post-exercise peak gradient LVOT to less than or equal to 30 mmHg in said subject.

4. The compound for use of claim 1, wherein therapeutically effective amount is sufficient to increase peak VO₂ (mL/kg/min) of +3.0 or more in said subject, or +1.5 or more in said subject.

5. The compound for use of claim 1, wherein said Compound 1 is administered:
orally; or
once daily; optionally wherein said Compound 1 is administered daily for at least twelve weeks.

6. The compound for use of claim 1, wherein said subject:
has a body mass index (BMI) of 18 to 37 kg/m² +/- 10% prior to treatment; or
prior to treatment, has a post-exercise peak LVOT gradient of 50 mmHg or higher.

7. The compound for use as claimed in claim 1, in a single unit dosage tablet or capsule form comprising 2-15 mg of Compound 1, having the formula:

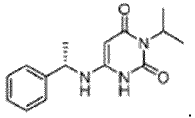

8. The compound for use as claimed in claim 7, wherein the compound is in a pharmaceutical composition.

9. A compound, having the formula: 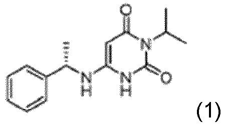 or a pharmaceutically acceptable salt thereof, for use in a method of treating myocardial diastolic dysfunction in a subject in need thereof, the method comprising administering a therapeutically effective amount of Compound 1 to said subject, wherein said therapeutically effective amount is a total daily dosage of 2 mg to 15 mg +/- 10%.

10. A compound, having the formula: 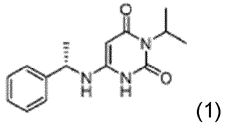
or a pharmaceutically acceptable salt thereof, for use in a method for treating hypertrophic cardiomyopathy (HCM) in an individual in need thereof comprising administering a daily dose of 5 mg of Compound 1 to the individual for at least one week, and
increasing said daily dose to 10 mg when the blood plasma concentration of Compound 1 in the individual is below 200 ng/mL; or
decreasing said daily dose to 2.5 mg when the blood plasma concentration of Compound 1 in the individual is above 750 ng/mL; or
maintaining said daily dose at 5 mg of Compound 1 when the blood plasma concentration of Compound 1 in the individual is from 200 to 750 ng/mL.

11. A compound, having the formula: 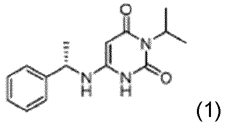 or a pharmaceutically acceptable salt thereof, for use in a method for treating hypertrophic cardiomyopathy (HCM) in an individual in need thereof by achieving and maintaining a blood plasma concentration of Compound 1 from 200 to 750 ng/mL, said method comprising
a) administering a daily dose selected from the group consisting of 2.5, 5, 10, and 15 mg of Compound 1 to an individual.

12. A compound, having the formula: 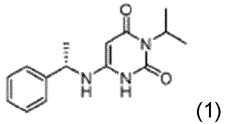 or a pharmaceutically acceptable salt thereof, for use in a method for treating hypertrophic cardiomyopathy in an individual in need thereof comprising administering to the individual a total daily dose of Compound 1 in an amount selected from the group consisting of 2.5 mg, 5 mg, 10 mg, and 15 mg.

13. The compound for use of any one of claims 1 to 8, 11 or 12, wherein said HCM is obstructive HCM (oHCM).

## Patentansprüche

1. Verbindung mit der Formel: 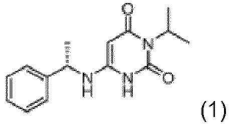 oder ein pharmazeutisch annehmbares Salz davon, zur Verwendung in einem Verfahren zur Behandlung von hypertropher Kardiomyopathie (HCM) bei einer behandlungsbedürftigen Person, wobei das Verfahren die Verabreichung einer therapeutisch wirksamen Menge von Verbindung 1 an die Person umfasst, wobei es sich bei der therapeutisch wirksamen Menge um eine tägliche Gesamtdosis von 2 mg bis 15 mg +/-10 % handelt.

2. Verbindung zur Verwendung nach Anspruch 1, wobei die Person über einen Zeitraum von mindestens zwei Wochen vor Beginn der Behandlung mit Verbindung 1 keine β-Blocker-Therapie erhalten hat.

3. Verbindung zur Verwendung nach Anspruch 1, wobei:
die Person vor Beginn der Behandlung einen Druckgradienten von mindestens 30 mmHg über dem linksventrikulären Ausflusstrakt (LVOT) der Person aufweist oder
die therapeutisch wirksame Menge ausreicht, um einen LVOT-Spitzengradienten nach der Belastung auf weniger als oder gleich 30 mmHg bei der Person zu senken.

4. Verbindung zur Verwendung nach Anspruch 1, wobei die therapeutisch wirksame Menge ausreicht, um den VO2-Spitzenwert (ml/kg/min) bei der Person um +3,0 oder mehr bzw. +1,5 oder mehr zu erhöhen.

5. Verbindung zur Verwendung nach Anspruch 1, wobei Verbindung 1 verabreicht wird:
oral oder
einmal täglich; wobei Verbindung 1 gegebenenfalls über einen Zeitraum von mindestens zwölf Wochen täglich verabreicht wird.

6. Verbindung zur Verwendung nach Anspruch 1, wobei die Person:
einen Body-Mass-Index (BMI) von 18 bis 37 kg/m² +/-10 % vor der Behandlung aufweist oder
vor der Behandlung einen LVOT-Spitzengradienten von 50 mmHg oder mehr nach Belastung aufweist.

7. Verbindung zur Verwendung nach Anspruch 1 in Form einer Tablette oder Kapsel mit einer Einzeldosis, die 2 bis 15 mg von Verbindung 1 umfasst und die Formel aufweist:

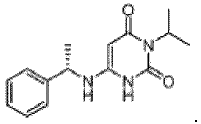

8. Verbindung zur Verwendung nach Anspruch 7, wobei die Verbindung in einer pharmazeutischen Zusammensetzung vorliegt.

9. Verbindung mit der Formel: 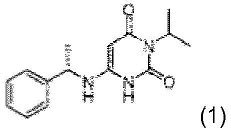 oder ein pharmazeutisch annehmbares Salz davon, zur Verwendung in einem Verfahren zur Behandlung von myokardialer diastolischer Dysfunktion bei einer behandlungsbedürftigen Person, wobei das Verfahren die Verabreichung einer therapeutisch wirksamen Menge von Verbindung 1 an die Person umfasst, wobei es sich bei der therapeutisch wirksamen Menge um eine tägliche Gesamtdosis von 2 mg bis 15 mg +/-10 % handelt.

10. Verbindung mit der Formel: 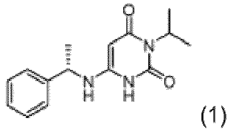
oder ein pharmazeutisch unbedenkliches Salz davon, zur Verwendung in einem Verfahren zur Behandlung von hypertropher Kardiomyopathie (HCM) bei einer behandlungsbedürftigen Person, umfassend die Verabreichung einer täglichen Dosis von 5 mg von Verbindung 1 an die Person über mindestens eine Woche, und
Erhöhung der Tagesdosis auf 10 mg, wenn die Blutplasmakonzentration von Verbindung 1 bei der Person unter 200 ng/ml beträgt, oder
Verringern der Tagesdosis auf 2,5 mg, wenn die Blutplasmakonzentration von Verbindung 1 bei der Person über 750 ng/ml beträgt, oder
Beibehaltung der Tagesdosis von 5 mg der Verbindung 1, wenn die Blutplasmakonzentration von Verbindung 1 bei der Person zwischen 200 und 750 ng/ml beträgt.

11. Verbindung mit der Formel: 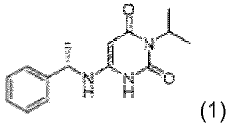 oder ein pharmazeutisch unbedenkliches Salz davon, zur Verwendung in einem Verfahren zur Behandlung von hypertropher Kardiomyopathie (HCM) bei einer behandlungsbedürftigen Person durch Erreichen und Aufrechterhalten einer Blutplasmakonzentration von Verbindung 1 in Höhe von 200 bis 750 ng/ml, wobei dieses Verfahren umfasst
a) Verabreichen einer täglichen Dosis, ausgewählt aus der Gruppe bestehend aus 2,5, 5, 10 und 15 mg von Verbindung 1 an eine Person.

12. Verbindung mit der Formel: 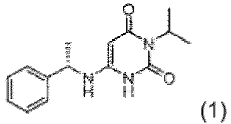 oder ein pharmazeutisch unbedenkliches Salz davon, zur Verwendung in einem Verfahren zur Behandlung von hypertropher Kardiomyopathie bei einer behandlungsbedürftigen Person, umfassend das Verabreichen einer täglichen Gesamtdosis von Verbindung 1 an die Person in einer Menge, ausgewählt aus der Gruppe bestehend aus 2,5 mg, 5 mg, 10 mg und 15 mg.

13. Verbindung zur Verwendung nach einem der Ansprüche 1 bis 8, 11 oder 12, wobei es sich bei der HCM um obstruktive HCM (oHCM) handelt.

## Revendications

1. Composé ayant la formule : 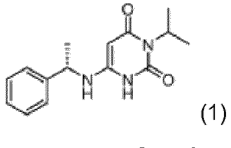 ou un sel pharmaceutiquement acceptable de celui-ci, destiné à être utilisé dans une méthode de traitement de la cardiomyopathie hypertrophique (CMH) chez un sujet en ayant besoin, la méthode comprenant l'administration d'une quantité thérapeutiquement efficace du composé 1 audit sujet, ladite quantité thérapeutiquement efficace étant une dose quotidienne totale de 2 mg à 15 mg +/- 10 %.

2. Composé selon la revendication 1, le sujet n'ayant pas reçu de traitement par des bêtabloquants pendant une période d'au moins deux semaines avant le début du traitement par le composé 1.

3. Composé selon la revendication 1, **caractérisé en ce que** :
ledit sujet présente un gradient de pression d'au moins 30 mmHg dans la chambre d'éjection du ventricule gauche (CEVG) avant le début du traitement ; ou
la quantité thérapeutiquement efficace est suffisante pour abaisser le gradient maximal de CEVG après l'effort à une valeur inférieure ou égale à 30 mmHg.

4. Composé selon la revendication 1, la quantité thérapeutiquement efficace étant suffisante pour augmenter la VO2 maximale (ml/kg/min) d'au moins +3,0, ou d'au moins +1,5 chez ledit sujet.

5. Composé selon la revendication 1, ledit composé 1 étant administré :
par voie orale ; ou
une fois par jour ; facultativement, ledit composé 1 étant administré quotidiennement pendant au moins douze semaines.

6. Composé selon la revendication 1, ledit sujet :
ayant un indice de masse corporelle (IMC) de 18 à 37 kg/m² +/- 10 % avant le traitement ; ou
avant le traitement, présentant un gradient maximal de CEVG après l'effort supérieur ou égal à 50 mmHg.

7. Composé selon la revendication 1, sous forme de comprimé ou de capsule à dose unitaire contenant 2 à 15 mg de composé 1, ayant la formule :

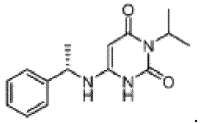

8. Composé selon la revendication 7, le composé étant présent dans une composition pharmaceutique.

9. Composé ayant la formule : 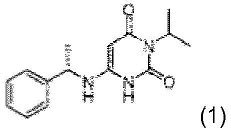 ou un sel pharmaceutiquement acceptable de celui-ci, destiné à être utilisé dans une méthode de traitement de la dysfonction diastolique myocardique chez un sujet en ayant besoin, la méthode comprenant l'administration d'une quantité thérapeutiquement efficace de composé 1 audit sujet, ladite quantité thérapeutiquement efficace étant une dose quotidienne totale de 2 mg à 15 mg +/- 10 %.

10. Composé ayant la formule : 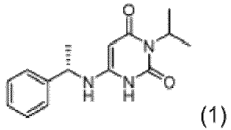
ou un sel pharmaceutiquement acceptable de celui-ci, destiné à être utilisé dans le traitement de la cardiomyopathie hypertrophique (CMH) chez un individu en ayant besoin, comprenant l'administration d'une dose quotidienne de 5 mg de composé 1 à l'individu pendant au moins une semaine, et
l'augmentation de ladite dose quotidienne à 10 mg lorsque la concentration plasmatique de composé 1 chez l'individu est inférieure à 200 ng/ml ; ou
la diminution de ladite dose quotidienne à 2,5 mg lorsque la concentration plasmatique de composé 1 chez l'individu est supérieure à 750 ng/ml ; ou
l'augmentation de ladite dose quotidienne à 5 mg de composé 1 lorsque la concentration plasmatique de composé 1 chez l'individu est de 200 ng/ml à 750 ng/ml.

11. Composé ayant la formule : 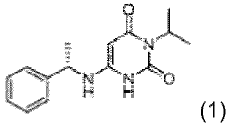 ou un sel pharmaceutiquement acceptable de celui-ci, destiné à être utilisé dans une méthode de traitement de la cardiomyopathie hypertrophique (CMH) chez un sujet en ayant besoin, par obtention et maintien d'une concentration plasmatique de composé 1 de 200 à 750 ng/ml, ladite méthode comprenant :
a) l'administration d'une dose quotidienne choisie dans le groupe constitué par 2,5, 5, 10 et 15 mg de composé 1 à un individu.

12. Composé ayant la formule : 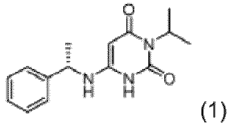 ou un sel pharmaceutiquement acceptable de celui-ci, destiné à être utilisé dans une méthode de traitement de la cardiomyopathie hypertrophique chez un individu en ayant besoin, comprenant l'administration à l'individu d'une dose quotidienne totale de composé 1 en une quantité choisie dans le groupe constitué de 2,5 mg, 5 mg, 10 mg et 15 mg.

13. Composé selon l'une quelconque des revendications 1 à 8, 11 ou 12, ladite CMH étant une CMH obstructive (CMHo).
